# EUROPEAN PATENT SPECIFICATION

(11) **EP 3 681 859 B1**
(45) Date of publication and mention of the grant of the patent: **26.10.2022**
(21) Application number: 18826077.2
(22) Date of filing: 21.12.2018
(51) Int. Cl.: C07C 225/20, C07C 237/26, C07K 14/36, C12N 9/10, C12N 9/02, C12N 9/00, C12N 9/04

(54) **GENE CLUSTER FOR THE BIOSYNTHETIC PRODUCTION OF TETRACYCLINE COMPOUNDS IN A HETEROLOGOUS HOST**
GENCLUSTER ZUR BIOSYNTHETISCHEN HERSTELLUNG VON TETRACYCLINVERBINDUNGEN IN EINEM HETEROLOGEN HOST
GROUPE DE GÈNES POUR LA BIOSYNTHÈSE DE COMPOSÉS DE TÉTRACYCLINE DANS UN HÔTE HÉTÉROLOGUE

(30) Priority: 22.12.2017 EP 17210536
(43) Date of publication of application: 22.07.2020
(73) Proprietor: Helmholtz-Zentrum für Infektionsforschung GmbH, 38124 Braunschweig (DE); Acies Bio d.o.o., 1000 Ljubljana (SI)
(72) Inventor: MÜLLER, Rolf, 38124 Braunschweig (DE); LUKEZIC, Tadeja, 1000 Ljubljana (SI); REMSKAR, Maja, 38124 Braunschweig (DE); ZABURANNYI, Nestor, 38124 Braunschweig (DE); BADER, Chantal, 38124 Braunschweig (DE); SIKANDAR, Asfandyar, 38124 Braunschweig (DE); KÖHNKE, Jesko, 38124 Braunschweig (DE)
(74) Representative: Zacco GmbH
(86) International application number: PCT/EP2018/086740
(87) International publication number: WO 2019/122400

(56) References cited:
- EP-A1- 2 154 150
- Anonymous: "UPI000BF4B0FF", , 20 December 2017 (2017-12-20), XP055470416, Retrieved from the Internet: URL:http://www.uniprot.org/uniparc/UPI000B F4B0FF [retrieved on 2018-04-25]
- Anonymous: "UPI000C01A836", , 20 December 2017 (2017-12-20), XP055470414, Retrieved from the Internet: URL:http://www.uniprot.org/uniparc/UPI000C 01A836 [retrieved on 2018-04-25]
- Anonymous: "UPI000C015918", , 20 December 2017 (2017-12-20), XP055470412, Retrieved from the Internet: URL:http://www.uniprot.org/uniparc/UPI000C 015918 [retrieved on 2018-04-25]

## Description

### Field of the invention

The present invention relates to the application of biosynthetic engineering for the heterologous expression of a gene cluster for the biosynthesis of tetracycline compounds, notably chelocardin and its analogues. More particularly, the present invention pertains to a gene cluster encoding polypeptides involved in tetracycline biosynthesis, which gene cluster is suitable for heterologous expression of the biosynthetic pathway in a host cell. The present invention further pertains to DNA constructs comprising the gene cluster, to recombinant heterologous host cells comprising the gene cluster or the DNA construct, to processes for the biosynthetic production of a tetracycline compound employing such recombinant host cells, and to tetracycline compounds thereby produced. The present invention also pertains to fusion proteins which are useful in the production of tetracycline compounds.

### Background of the invention

Chelocardin (CHD; also known as M319, cetocycline or cetotetrine) is an atypical tetracycline with broad spectrum of antibiotic activity, produced by the actinomycete *Amycolatopsis sulphurea.* Possession of a well-known tetracycline scaffold is only one the structural characteristics of chelocardin. Importantly, chelocardin is also active against tetracycline-resistant pathogens (Proctor et al. 1978). It showed promising in a small phase II clinical study on patients with urinary tract infections caused by Gram-negative pathogens in 1977 (Molnar et al. 1977). Chelocardin structure differs in quite a number of details from the one of typical tetracyclines, reflecting also in a different mode of action (Rasmussen et al. 1991; Stepanek et al. 2016).

The use of genes from a chelocardin biosynthetic gene cluster would generally enable the production of the potent broad-spectrum antibiotic chelocardin and its analogues. The chelocardin biosynthetic gene cluster from *Amycolatopsis sulphurea* and its use has been described in EP2154249 (Petkovic et al.) and Lukezic et al. (Lukezic et al. 2013). However, while chelocardin and its analogues, especially its amidated analogue 2-carboxamido-2-deacetyl-chelocardin (CDCHD), could be obtained using the wild-type producer *Amycolatopsis sulphurea* or modified variants thereof, heterologous expression of the described gene cluster has turned out to be difficult as it did not result in the production of chelocardin in the heterologous host.

Accordingly, it is an object of the present invention to provide means which enable the production of chelocardin or analogues thereof in a heterologous host.

### Summary of the invention

The present invention is based on the surprising finding that the chelocardin biosynthetic gene cluster isolated from the wild-type chelocardin producer *Amycolatopsis sulphurea* comprises a further gene (herein referred to as *chdY*) encoding a second ring cyclase which seems to be essential for the formation of the basic tetracyclic scaffold. Even more surprising, the cyclase encoded by *chdY* gene and the FAD-dependent oxygenase encoded by the *chdOII* gene are expressed in the form of a fusion protein.

Moreover, the present inventors have identified two so far undiscovered regulatory genes within the chelocardin biosynthetic gene cluster (herein referred to as *chdB* and *chdC*, respectively) which encode transcriptional activators belonging to the SARP and LuxR family, respectively. While these regulatory genes are not directly involved in the synthesis of chelocardin, they are expected to have a positive effect on the production chelocardin in a heterologous host as seen for homologous family members in oxytetracycline production.

By providing the genetic information on the gene *chdY* as well as that of genes *chdB* and *chdC*, it is now possible to produce chelocardin and its analogues in a heterologous host, which in turn allows a higher production of this atypical tetracycline compared to the chelocardin natural producer, *Amycolatopsis sulphurea.*

The present invention therefore provides in a first aspect a gene cluster encoding polypeptides involved in the biosynthesis of a tetracycline.

The present invention provides in a further aspect a DNA construct comprising the gene cluster of the present invention.

The present invention provides in a further aspect a recombinant host cell comprising the gene cluster or the DNA construct according to the present invention.

The present invention provides in a further aspect a process for the biosynthetic production of a tetracycline, said method comprises the steps of a) cultivating a recombinant host cell according to the present invention in the presence of a suitable substrate under conditions conducive to the production of said tetracycline and, optionally, b) recovering the tetracycline from the cultivation medium.

The present invention is defined by the appended claims.

### Brief description of the drawings

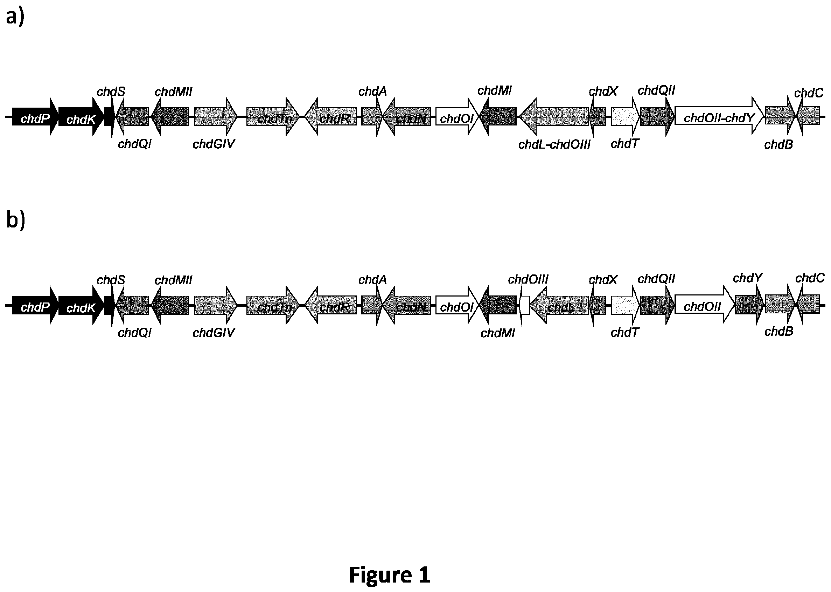

**Figure 1****:** Chelocardin biosynthetic gene cluster, and genes involved in chelocardin production. A) Chelocardin biosynthetic gene cluster as found in *A. sulphurea*; B) Alternative gene cluster with *chdL*, *chdOIII*, *chdY* and c*hdOII* present as separate genes.

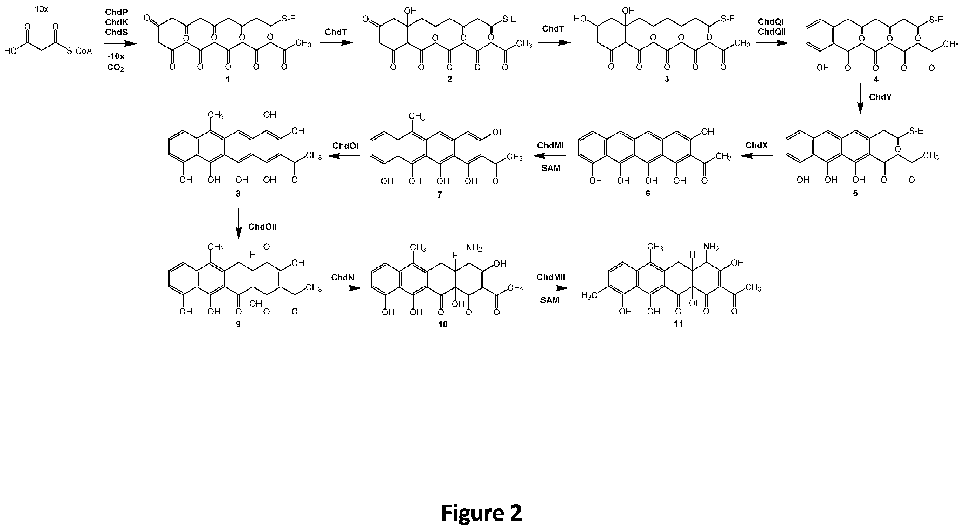

**Figure 2****:** Proposed chelocardin biosynthesis pathway, according to the invention

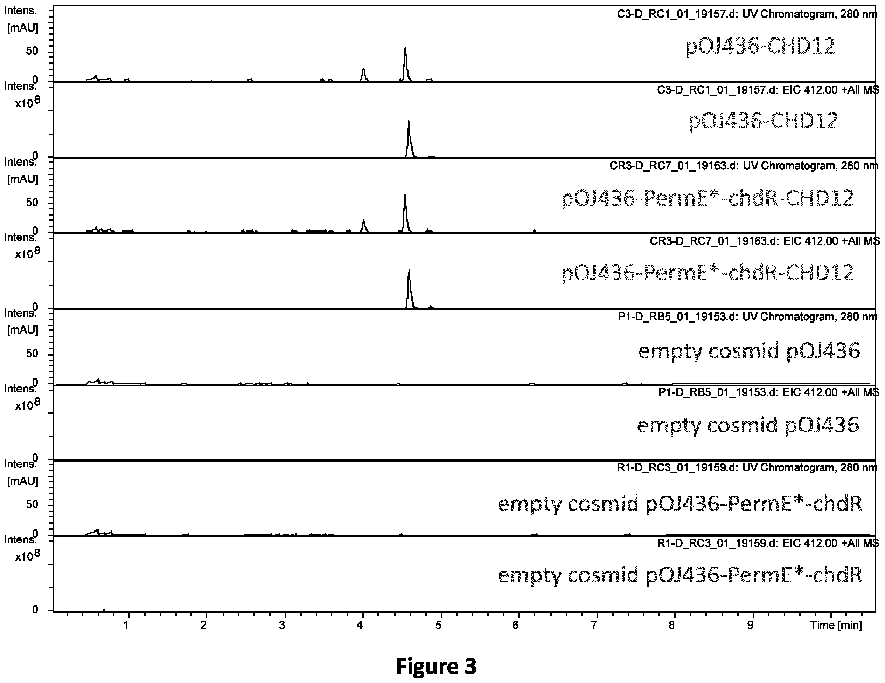

**Figure 3****:** LC-MS analysis of culture extracts of S. *albus* with integrated cosmids carrying CHD biosynthetic gene cluster (pOJ436-CHD12) or CHD biosynthetic gene cluster with additional copy of *chdR* (pOJ436-PermE*-chdR-CHD12) in comparison with culture extracts of S. *albus* with integrated empty cosmids pOJ436 or pOJ436-PermE*-chdR. UV chromatograms at detection wavelength of 280 nm and EICs for m/z 412 (± 0.5), which corresponds to CHD, are shown (chromatograms adapted from DataAnalysis (available from Bruker Daltonics)).

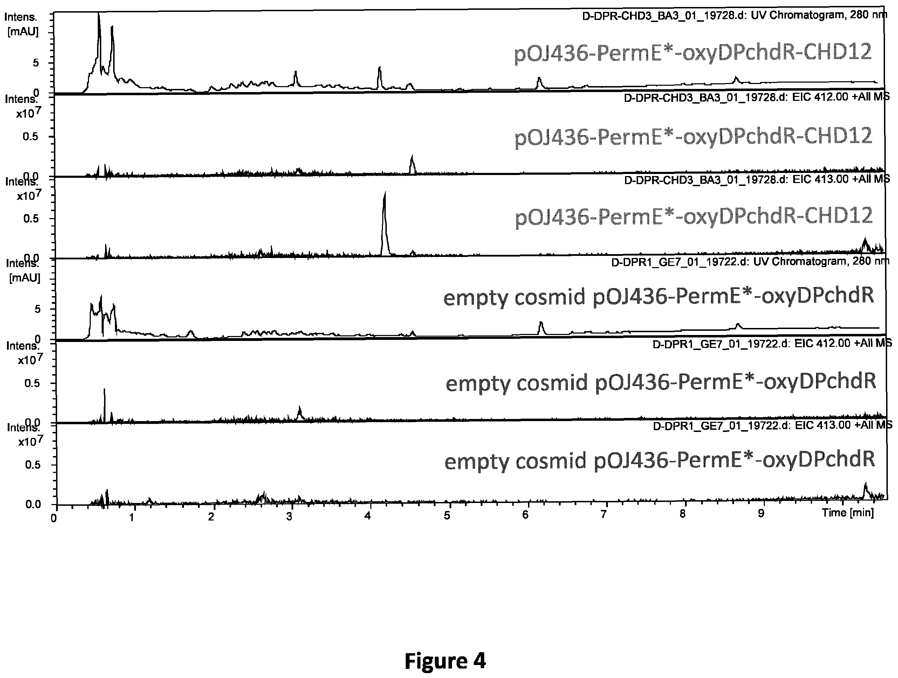

**Figure 4****:** LC-MS analysis of culture extracts of S. *albus* with integrated cosmid carrying CHD biosynthetic gene cluster together with *oxyD* and *oxyP* genes and additional copy of *chdR* (pOJ436-PermE*-oxyDPchdR-CHD12) in comparison with culture extracts of S. *albus* with integrated empty cosmid pOJ436-PermE*-oxyDPchdR. UV chromatograms at detection wavelength of 280 nm and EICs for m/z 412 (± 0.5) and 413 (± 0.5), which correspond to CHD and CDCHD, respectively, are shown (chromatograms adapted from DataAnalysis (available from Bruker Daltonics))

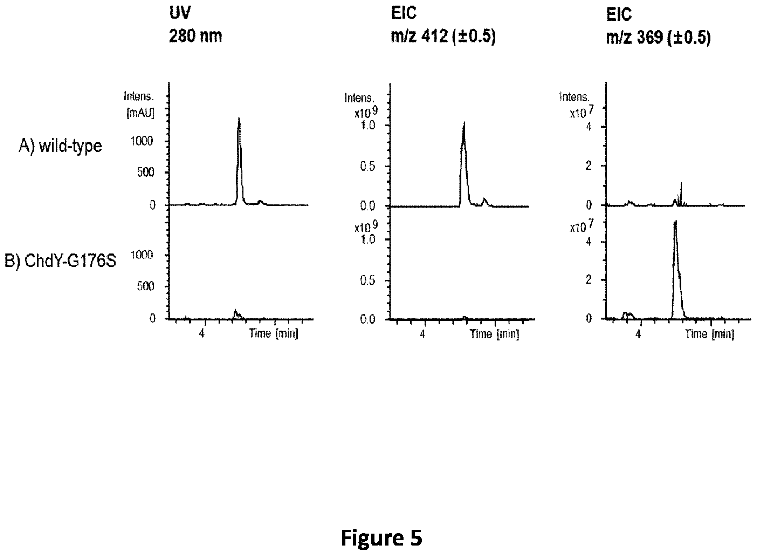

**Figure 5****:** LC-MS analysis of culture extracts of *A. sulphurea* wild-type (A) and *A. sulphurea* ChdY-G176S mutant (B). UV chromatograms at detection wavelength of 280 nm and EICs for m/z 412 (± 0.5) and 369 (± 0.5), corresponding to CHD and »compound 369«, respectively, are shown (chromatograms adapted from DataAnalysis (available from Bruker Daltonics)).

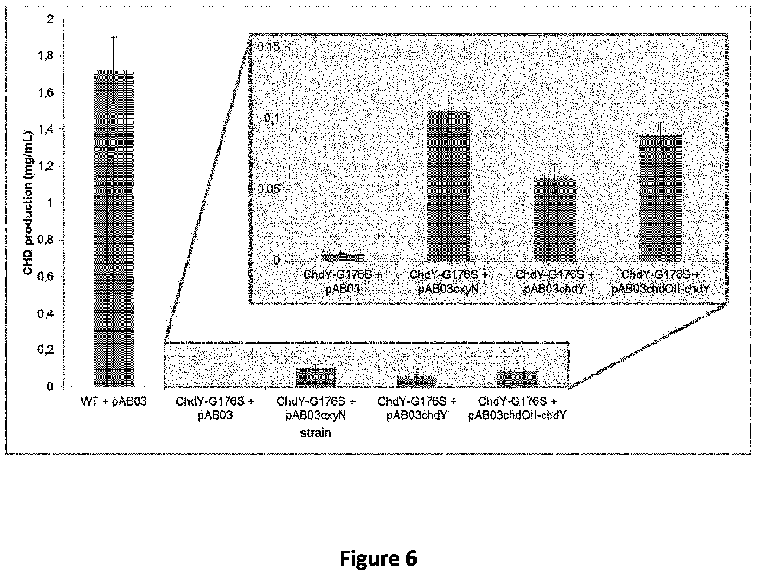

**Figure 6****:** CHD production in ChdY-G176S mutant before (ChdY-G176S + pAB03) and after complementation experiments with genes for either OxyN (ChdY-G176S + pAB03oxyN), ChdY (ChdY-G176S + pAB03chdY) or ChdOII-ChdY (ChdY-G176S + pAB03chdOII-chdY), compared to A. sulphurea WT with integrated empty plasmid pAB03 (WT + pAB03)

### Detailed description of the invention

Unless specifically defined herein, all technical and scientific terms used have the same meaning as commonly understood by a skilled artisan in the fields of biochemistry, genetics, molecular biology and microbiology.

All methods and materials similar or equivalent to those described herein can be used in the practice or testing of the present invention, with suitable methods and materials being described herein.

In case of conflict, the present specification, including definitions, will prevail. Further, the materials, methods, and examples are illustrative only and are not intended to be limiting, unless otherwise specified. The practice of the present invention will employ, unless otherwise indicated, conventional techniques of cell biology, cell culture, molecular biology, transgenic biology, microbiology, and recombinant DNA technology, which are within the skill of the art. Such techniques are explained fully in the literature. See, for example, Current Protocols in Molecular Biology (Ausubel 1987); Molecular Cloning: A Laboratory Manual, Third Edition, (Sambrook, Russell 2001); Transcription And Translation (Harnes, Higgins 1984); and the series, Methods In ENZYMOLOGY (Abelson, Simon 1998), specifically, Vols.154 and 155 (Wu et al. eds.) and Vol. 185, "Gene Expression Technology" (D. Goeddel, ed.).

As mentioned above, the present invention provides a gene cluster encoding polypeptides involved in the biosynthesis of a tetracycline, notably chelocardin or an analogue thereof. A representative overview of the gene cluster gene cluster is presented in Figure 1. Particularly, the present invention provides a (isolated) gene cluster encoding polypeptides involved in the biosynthesis of a tetracycline, wherein said gene cluster includes all of the nucleotide sequences (1) to (19):
(1) a nucleotide sequence encoding a polypeptide which comprises an amino acid sequence having at least 80%, such as at least 85%, sequence identity with the polypeptide of SEQ ID NO: 1 [ChdP];
(2) a nucleotide sequence encoding a polypeptide which comprises an amino acid sequence having at least 80%, such as at least 85%, sequence identity with the polypeptide of SEQ ID NO: 2 [ChdK];
(3) a nucleotide sequence encoding a polypeptide which comprises an amino acid sequence having at least 80%, such as at least 85%, sequence identity with the polypeptide of SEQ ID NO: 3 [ChdS];
(4) a nucleotide sequence encoding a polypeptide which comprises an amino acid sequence having at least 80%, such as at least 85%, sequence identity with the polypeptide of SEQ ID NO: 4 [ChdQI];
(5) a nucleotide sequence encoding a polypeptide which comprises an amino acid sequence having at least 80%, such as at least 85%, sequence identity with the polypeptide of SEQ ID NO: 5 [ChdQII];
(6) a nucleotide sequence encoding a polypeptide which comprises an amino acid sequence having at least 80%, such as at least 85%, sequence identity with the polypeptide of SEQ ID NO: 6 [ChdX];
(7) a nucleotide sequence encoding a polypeptide which comprises an amino acid sequence having at least 80%, such as at least 85%, sequence identity with the polypeptide of SEQ ID NO: 7 [ChdL];
(8) a nucleotide sequence encoding a polypeptide which comprises an amino acid sequence having at least 80%, such as at least 85%, sequence identity with the polypeptide of SEQ ID NO: 8 [ChdT];
(9) a nucleotide sequence encoding a polypeptide which comprises an amino acid sequence having at least 80%, such as at least 85%, sequence identity with the polypeptide of SEQ ID NO: 9 [ChdMI];
(10) a nucleotide sequence encoding a polypeptide which comprises an amino acid sequence having at least 80%, such as at least 85%, sequence identity with the polypeptide of SEQ ID NO: 10 [ChdMII];
(11) a nucleotide sequence encoding a polypeptide which comprises an amino acid sequence having at least 80%, such as at least 85%, sequence identity with the polypeptide of SEQ ID NO: 11 [ChdN];
(12) a nucleotide sequence encoding a polypeptide which comprises an amino acid sequence having at least 80%, such as at least 85%, sequence identity with the polypeptide of SEQ ID NO: 12 [ChdGIV];
(13) a nucleotide sequence encoding a polypeptide which comprises an amino acid sequence having at least 80%, such as at least 85%, sequence identity with the polypeptide of SEQ ID NO: 13 [ChdTn];
(14) a nucleotide sequence encoding a polypeptide which comprises an amino acid sequence having at least 80%, such as at least 85%, sequence identity with the polypeptide of SEQ ID NO: 14 [ChdR];
(15) a nucleotide sequence encoding a polypeptide which comprises an amino acid sequence having at least 80%, such as at least 85%, sequence identity with the polypeptide of SEQ ID NO: 15 [ChdA];
(16) a nucleotide sequence encoding a polypeptide which comprises an amino acid sequence having at least 80%, such as at least 85%, sequence identity with the polypeptide of SEQ ID NO: 16 [ChdOI];
(17) a nucleotide sequence encoding a polypeptide which comprises an amino acid sequence having at least 80%, such as at least 85%, sequence identity with the polypeptide of SEQ ID NO: 17 [ChdOIII];
(18) a nucleotide sequence encoding a polypeptide which comprises an amino acid sequence having at least 80%, such as at least 85%, sequence identity with the polypeptide of SEQ ID NO: 18 [ChdOII]; and
(19) a nucleotide sequence encoding a polypeptide which comprises an amino acid sequence having at least 80%, such as at least 85%, sequence identity with the polypeptide of SEQ ID NO: 19 [ChdY].

According to certain embodiments, the polypeptide encoded by the nucleotide sequence (1) comprises an amino acid sequence having at least 85% sequence identity with the polypeptide of SEQ ID NO: 1. According to certain embodiments, the polypeptide encoded by the nucleotide sequence (1) comprises an amino acid sequence having at least 90% sequence identity with the polypeptide of SEQ ID NO: 1. According to certain embodiments, the polypeptide encoded by the nucleotide sequence (1) comprises an amino acid sequence having at least 95% sequence identity with the polypeptide of SEQ ID NO: 1. According to certain embodiments, the polypeptide encoded by the nucleotide sequence (1) consists of the amino acid sequence of SEQ ID NO: 1.

The polypeptide encoded by the nucleotide sequence (1) has the same functional property as the polypeptide of SEQ ID NO: 1 [ChdP]. ChdP is a ketosynthase-alpha. The N terminal catalytic domain of the ChdP protein harbours a well conserved aa region around the highly conserved active site Cys173 (GPVGLVSTGCTSGVDVIGHA) responsible for catalyzing the iterative condensation of the ketoacyl:ACP intermediates. In the C terminus of the protein there is an amino-acid sequence characteristic of the acyltransferase site (VPVSSIKSMVGHSLGAIGSLEVAA) with the active Ser351 residue that binds to an acyl chain (Fernandez-Moreno et al. 1992). Specifically, ChdP catalyses a Claisen-type C-C bond formation from CoA activated acyl building blocks leading to the formation of a 20-carbon decaketide. Accordingly, the polypeptide encoded by the nucleotide sequence (1) has ketosynthase-alpha activity.

According to certain embodiments, the polypeptide encoded by the nucleotide sequence (2) comprises an amino acid sequence having at least 85% sequence identity with the polypeptide of SEQ ID NO: 2. According to certain embodiments, the polypeptide encoded by the nucleotide sequence (2) comprises an amino acid sequence having at least 90% sequence identity with the polypeptide of SEQ ID NO: 2. According to certain embodiments, the polypeptide encoded by the nucleotide sequence (2) comprises an amino acid sequence having at least 95% sequence identity with the polypeptide of SEQ ID NO: 2. According to certain embodiments, the polypeptide encoded by the nucleotide sequence (2) consists of the amino acid sequence of SEQ ID NO: 2.

The polypeptide encoded by the nucleotide sequence (2) has the same functional property as the polypeptide of SEQ ID NO: 2 [ChdK]. ChdK is a ketosynthase-beta (KSβ), also called chain-length factor. Ketosynthase domain active-site cysteine residue in ChdK is replaced by a highly conserved glutamine as in KSQ (VSEQ¹⁷⁵AGGLD) and in other chain-length factors of type II PKS synthases. Specifically, ChdK is acting together with ketosynthase-alpha in the formation of the 20-carbon decaketide. Accordingly, the polypeptide encoded by the nucleotide sequence (2) has ketosynthase-beta activity.

According to certain embodiments, the polypeptide encoded by the nucleotide sequence (3) comprises an amino acid sequence having at least 85% sequence identity with the polypeptide of SEQ ID NO: 3. According to certain embodiments, the polypeptide encoded by the nucleotide sequence (3) comprises an amino acid sequence having at least 90% sequence identity with the polypeptide of SEQ ID NO:3. According to certain embodiments, the polypeptide encoded by the nucleotide sequence (3) comprises an amino acid sequence having at least 95% sequence identity with the polypeptide of SEQ ID NO: 3. According to certain embodiments, the polypeptide encoded by the nucleotide sequence (3) consists of the amino acid sequence of SEQ ID NO: 3.

The polypeptide encoded by the nucleotide sequence (3) has the same functional property as the polypeptide of SEQ ID NO: 3 [ChdS]. ChdS is an acyl carrier protein which harbours an active Ser41 residue in the highly conserved motif (LGYDSL), to which phosphopantetheine binds in order to connect the incoming extender unit (Walsh et al. 1997). Specifically, ChdS cooperates with ketosynthase-alpha and ketosynthase-beta in the formation of the 20-carbon decaketide by serving as an anchor for the growing polyketide chain. Accordingly, the polypeptide encoded by the nucleotide sequence (3) is capable of acting as acyl carrier protein (ACP).

According to certain embodiments, the polypeptide encoded by the nucleotide sequence (4) comprises an amino acid sequence having at least 85% sequence identity with the polypeptide of SEQ ID NO: 4. According to certain embodiments, the polypeptide encoded by the nucleotide sequence (4) comprises an amino acid sequence having at least 90% sequence identity with the polypeptide of SEQ ID NO: 4. According to certain embodiments, the polypeptide encoded by the nucleotide sequence (4) comprises an amino acid sequence having at least 95% sequence identity with the polypeptide of SEQ ID NO: 4. According to certain embodiments, the polypeptide encoded by the nucleotide sequence (4) consists of the amino acid sequence of SEQ ID NO: 4.

The polypeptide encoded by the nucleotide sequence (4) has the same functional property property as the polypeptide of SEQ ID NO: 4 [ChdQI]. ChdQI is a bifunctional cyclase/ aromatase. Within ChdQI there are the highly conserved amino acids, which are, according to the homologous cyclase/aromatase BexL, responsible for the determination of the final length of the polyketide and for its proper regiospecific cyclisation and aromatization (Ames et al. 2008). These amino acids are at positions Trp-32, Trp-68, Ser-70, Arg-72, and Trp-99. Specifically, ChdQI together with ChdQII catalyses the dehydration of the C9-hydroxyl and the subsequent aromatisation of the first ring (D). Accordingly, the polypeptide encoded by the nucleotide sequence (4) has cyclase/aromatase activity.

According to certain embodiments, the polypeptide encoded by the nucleotide sequence (5) comprises an amino acid sequence having at least 85% sequence identity with the polypeptide of SEQ ID NO: 5. According to certain embodiments, the polypeptide encoded by the nucleotide sequence (5) comprises an amino acid sequence having at least 90% sequence identity with the polypeptide of SEQ ID NO: 5. According to certain embodiments, the polypeptide encoded by the nucleotide sequence (5) comprises an amino acid sequence having at least 95% sequence identity with the polypeptide of SEQ ID NO: 5. According to certain embodiments, the polypeptide encoded by the nucleotide sequence (5) consists of the amino acid sequence of SEQ ID NO: 5.

The polypeptide encoded by the nucleotide sequence (5) has the same functional property as the polypeptide of SEQ ID NO: 5 [ChdQII]. ChdQII is a bifunctional cyclase/aromatase. Similarly as in the case of ChdQI, there are the highly conserved amino acids at positions Trp-32, Phe-36, Trp-67, Ser-69, Arg-71, Met-94 and Trp-98. Specifically, ChdQII catalyses together with ChdQI the dehydration of the C-9 hydroxyl and the subsequent aromatisation of the first ring (D). Accordingly, the polypeptide encoded by the nucleotide sequence (5) has cyclase/ aromatase activity.

According to certain embodiments, the polypeptide encoded by the nucleotide sequence (6) comprises an amino acid sequence having at least 85% sequence identity with the polypeptide of SEQ ID NO: 6. According to certain embodiments, the polypeptide encoded by the nucleotide sequence (6) comprises an amino acid sequence having at least 90% sequence identity with the polypeptide of SEQ ID NO: 6. According to certain embodiments, the polypeptide encoded by the nucleotide sequence (6) comprises an amino acid sequence having at least 95% sequence identity with the polypeptide of SEQ ID NO: 6. According to certain embodiments, the polypeptide encoded by the nucleotide sequence (6) consists of the amino acid sequence of SEQ ID NO: 6.

The polypeptide encoded by the nucleotide sequence (6) has the same functional property as the polypeptide of SEQ ID NO: 6 [ChdX]. ChdX is a cyclase. Specifically, ChdX catalyses aldol condensation between C-1 and C-18, resulting in formation of the fourth ring (A). Accordingly, the polypeptide encoded by the nucleotide sequence (6) has cyclase activity.

According to certain embodiments, the polypeptide encoded by the nucleotide sequence (7) comprises an amino acid sequence having at least 85% sequence identity with the polypeptide of SEQ ID NO: 7. According to certain embodiments, the polypeptide encoded by the nucleotide sequence (7) comprises an amino acid sequence having at least 90% sequence identity with the polypeptide of SEQ ID NO: 7. According to certain embodiments, the polypeptide encoded by the nucleotide sequence (7) comprises an amino acid sequence having at least 95% sequence identity with the polypeptide of SEQ ID NO: 7. According to certain embodiments, the polypeptide encoded by the nucleotide sequence (7) consists of the amino acid sequence of SEQ ID NO: 7.

The polypeptide encoded by the nucleotide sequence (7) has the same functional property as the polypeptide of SEQ ID NO: 7 [ChdL]. ChdL is an acyl-CoA ligase. Specifically, ChdL activates carboxylic acids as CoA thioesters. Accordingly, the polypeptide encoded by the nucleotide sequence (7) has acyl-CoA ligase activity.

According to certain embodiments, the polypeptide encoded by the nucleotide sequence (8) comprises an amino acid sequence having at least 85% sequence identity with the polypeptide of SEQ ID NO: 8. According to certain embodiments, the polypeptide encoded by the nucleotide sequence (8) comprises an amino acid sequence having at least 90% sequence identity with the polypeptide of SEQ ID NO: 8. According to certain embodiments, the polypeptide encoded by the nucleotide sequence (8) comprises an amino acid sequence having at least 95% sequence identity with the polypeptide of SEQ ID NO: 8. According to certain embodiments, the polypeptide encoded by the nucleotide sequence (8) consists of the amino acid sequence of SEQ ID NO: 8.

The polypeptide encoded by the nucleotide sequence (8) has the same functional property as the polypeptide of SEQ ID NO: 8 [ChdT]. ChdT is a ketoreductase. Two conserved domains can be found within the amino acid sequence of ChdT proposed to act as a NADPH-cofactor binding sites (Hopwood, Sherman 1990; Rawlings, Cronan, J. E., Jr. 1992). Specifically, ChdT regiospecifically cyclizes the linear poly-beta-ketone from C-12 to C-7, followed by a C-9-carbonyl reduction. Accordingly, the polypeptide encoded by the nucleotide sequence (8) has ketoreductase activity.

According to certain embodiments, the polypeptide encoded by the nucleotide sequence (9) comprises an amino acid sequence having at least 85% sequence identity with the polypeptide of SEQ ID NO: 9. According to certain embodiments, the polypeptide encoded by the nucleotide sequence (9) comprises an amino acid sequence having at least 90% sequence identity with the polypeptide of SEQ ID NO: 9. According to certain embodiments, the polypeptide encoded by the nucleotide sequence (9) comprises an amino acid sequence having at least 95% sequence identity with the polypeptide of SEQ ID NO: 9. According to certain embodiments, the polypeptide encoded by the nucleotide sequence (9) consists of the amino acid sequence of SEQ ID NO: 9.

The polypeptide encoded by the nucleotide sequence (9) has the same functional property as the polypeptide of SEQ ID NO: 9 [ChdMI]. ChdMI is a S-adenosylmethionine (SAM)-dependent C-6 methyltransferase. Specifically, ChdMI catalyses the methylation of C-6. Accordingly, the polypeptide encoded by the nucleotide sequence (9) has S-adenosylmethionine (SAM)-dependent C-6 methyltransferase activity.

According to certain embodiments, the polypeptide encoded by the nucleotide sequence (10) comprises an amino acid sequence having at least 85% sequence identity with the polypeptide of SEQ ID NO: 10. According to certain embodiments, the polypeptide encoded by the nucleotide sequence (10) comprises an amino acid sequence having at least 90% sequence identity with the polypeptide of SEQ ID NO: 10. According to certain embodiments, the polypeptide encoded by the nucleotide sequence (10) comprises an amino acid sequence having at least 95% sequence identity with the polypeptide of SEQ ID NO: 10. According to certain embodiments, the polypeptide encoded by the nucleotide sequence (10) consists of the amino acid sequence of SEQ ID NO: 10.

The polypeptide encoded by the nucleotide sequence (10) has the same functional property as the polypeptide of SEQ ID NO: 10 [ChdMII]. ChdMII is S-adenosylmethionine-dependent (SAM) C-9 methyltransferase. Specifically, ChdMII catalyses the methylation of C-9. Similarly as for ChdMI, ChdMII also shows a typical glycine-rich SAM-dependent methyltransferase motif that interacts with the SAM cofactor, which is used as a source for the methyl group (Martin, McMillan 2002). Accordingly, the polypeptide encoded by the nucleotide sequence (10) has S-adenosylmethionine (SAM)-dependent methyltransferase activity.

According to certain embodiments, the polypeptide encoded by the nucleotide sequence (11) comprises an amino acid sequence having at least 85% sequence identity with the polypeptide of SEQ ID NO: 11. According to certain embodiments, the polypeptide encoded by the nucleotide sequence (11) comprises an amino acid sequence having at least 90% sequence identity with the polypeptide of SEQ ID NO: 11. According to certain embodiments, the polypeptide encoded by the nucleotide sequence (11) comprises an amino acid sequence having at least 95% sequence identity with the polypeptide of SEQ ID NO: 11. According to certain embodiments, the polypeptide encoded by the nucleotide sequence (11) consists of the amino acid sequence of SEQ ID NO: 11.

The polypeptide encoded by the nucleotide sequence (11) has the same functional property as the polypeptide of [ChdN]. ChdN is a pyridoxal 5'-phosphate-dependent aminotransferase. Specifically, ChdN catalyses the single amination at the C-4. Accordingly, the polypeptide encoded by the nucleotide sequence (11) has pyridoxal 5'-phosphate-dependent aminotransferase activity.

According to certain embodiments, the polypeptide encoded by the nucleotide sequence (12) comprises an amino acid sequence having at least 85% sequence identity with the polypeptide of SEQ ID NO: 12. According to certain embodiments, the polypeptide encoded by the nucleotide sequence (12) comprises an amino acid sequence having at least 90% sequence identity with the polypeptide of SEQ ID NO: 12. According to certain embodiments, the polypeptide encoded by the nucleotide sequence (12) comprises an amino acid sequence having at least 95% sequence identity with the polypeptide of SEQ ID NO: 12. According to certain embodiments, the polypeptide encoded by the nucleotide sequence (12) consists of the amino acid sequence of SEQ ID NO: 12.

The polypeptide encoded by the nucleotide sequence (12) has the same functional property as the polypeptide of SEQ ID NO: 12 [ChdGIV]. ChdGIV is a glycosyltransferase. Accordingly, the polypeptide encoded by the nucleotide sequence (12) has glycosyltransferase activity.

According to certain embodiments, the polypeptide encoded by the nucleotide sequence (13) comprises an amino acid sequence having at least 85% sequence identity with the polypeptide of SEQ ID NO: 13. According to certain embodiments, the polypeptide encoded by the nucleotide sequence (13) comprises an amino acid sequence having at least 90% sequence identity with the polypeptide of SEQ ID NO: 13. According to certain embodiments, the polypeptide encoded by the nucleotide sequence (13) comprises an amino acid sequence having at least 95% sequence identity with the polypeptide of SEQ ID NO: 13. According to certain embodiments, the polypeptide encoded by the nucleotide sequence (13) consists of the amino acid sequence of SEQ ID NO: 13.

The polypeptide encoded by the nucleotide sequence (13) has the same functional property as the polypeptide of SEQ ID NO: 13 [ChdTn]. ChdTn is a transposase. Accordingly, the polypeptide encoded by the nucleotide sequence (13) has transposase activity.

According to certain embodiments, the polypeptide encoded by the nucleotide sequence (14) comprises an amino acid sequence having at least 85% sequence identity with the polypeptide of SEQ ID NO: 14. According to certain embodiments, the polypeptide encoded by the nucleotide sequence (14) comprises an amino acid sequence having at least 90% sequence identity with the polypeptide of SEQ ID NO: 14. According to certain embodiments, the polypeptide encoded by the nucleotide sequence (14) comprises an amino acid sequence having at least 95% sequence identity with the polypeptide of SEQ ID NO: 14. According to certain embodiments, the polypeptide encoded by the nucleotide sequence (14) consists of the amino acid sequence of SEQ ID NO: 14.

The polypeptide encoded by the nucleotide sequence (14) has the same functional property as the polypeptide of SEQ ID NO: 14 [ChdR]. ChdR is an exporter from the EmrB/QacA subfamily. Specifically, ChdR is an integral membrane protein facilitating the efflux of chelocardin from a cell. Accordingly, the polypeptide encoded by the nucleotide sequence (14) is capable of acting as an exporter.

According to certain embodiments, the polypeptide encoded by the nucleotide sequence (15) comprises an amino acid sequence having at least 85% sequence identity with the polypeptide of SEQ ID NO: 15. According to certain embodiments, the polypeptide encoded by the nucleotide sequence (15) comprises an amino acid sequence having at least 90% sequence identity with the polypeptide of SEQ ID NO: 15. According to certain embodiments, the polypeptide encoded by the nucleotide sequence (15) comprises an amino acid sequence having at least 95% sequence identity with the polypeptide of SEQ ID NO: 15. According to certain embodiments, the polypeptide encoded by the nucleotide sequence (15) consists of the amino acid sequence of SEQ ID NO: 15.

The polypeptide encoded by the nucleotide sequence (15) has the same functional property as the polypeptide of SEQ ID NO: 15 [ChdA]. ChdA is a transcriptional regulator most similar to the tetracycline repressor from the TetR family of proteins that are involved in the transcriptional control of multidrug efflux pumps. Specifically, ChdA regulates the expression of the exporter ChdR. Accordingly, the polypeptide encoded by the nucleotide sequence (15) has transcriptional regulator activity.

According to certain embodiments, the polypeptide encoded by the nucleotide sequence (16) comprises an amino acid sequence having at least 85% sequence identity with the polypeptide of SEQ ID NO: 16. According to certain embodiments, the polypeptide encoded by the nucleotide sequence (16) comprises an amino acid sequence having at least 90% sequence identity with the polypeptide of SEQ ID NO: 16. According to certain embodiments, the polypeptide encoded by the nucleotide sequence (16) comprises an amino acid sequence having at least 95% sequence identity with the polypeptide of SEQ ID NO: 16. According to certain embodiments, the polypeptide encoded by the nucleotide sequence (16) consists of the amino acid sequence of SEQ ID NO: 16.

The polypeptide encoded by the nucleotide sequence (16) has the same functional property as the polypeptide of SEQ ID NO: 16 [ChdOI]. ChdOI is a FAD-dependent oxygenase. ChdOI possesses at the N-terminal end a typical conserved sequence G-X-G-2X-G-3X-A-6X-G (where X is any naturally occurring amino acid) which is involved in the FAD-cofactor binding (Mason, Cammack 1992). Specifically, ChdOI catalyses the hydroxylation of C-4. Accordingly, the polypeptide encoded by the nucleotide sequence (16) has a FAD-dependent oxygenase activity.

According to certain embodiments, the polypeptide encoded by the nucleotide sequence (17) comprises an amino acid sequence having at least 85% sequence identity with the polypeptide of SEQ ID NO: 17. According to certain embodiments, the polypeptide encoded by the nucleotide sequence (17) comprises an amino acid sequence having at least 90% sequence identity with the polypeptide of SEQ ID NO: 17. According to certain embodiments, the polypeptide encoded by the nucleotide sequence (17) comprises an amino acid sequence having at least 95% sequence identity with the polypeptide of SEQ ID NO: 17. According to certain embodiments, the polypeptide encoded by the nucleotide sequence (17) consists of the amino acid sequence of SEQ ID NO: 17.

The polypeptide encoded by the nucleotide sequence (17) has the same functional property as the polypeptide of SEQ ID NO: 17 [ChdOIII]. ChdOIII is an ABM (Antibiotic Biosynthesis Monooxygenase) which catalyses molecular oxygen activation. Accordingly, the polypeptide encoded by the nucleotide sequence (17) has monooxygenase activity.

According to certain embodiments, the polypeptide encoded by the nucleotide sequence (18) comprises an amino acid sequence having at least 85% sequence identity with the polypeptide of SEQ ID NO: 18. According to certain embodiments, the polypeptide encoded by the nucleotide sequence (18) comprises an amino acid sequence having at least 90% sequence identity with the polypeptide of SEQ ID NO: 18. According to certain embodiments, the polypeptide encoded by the nucleotide sequence (18) comprises an amino acid sequence having at least 95% sequence identity with the polypeptide of SEQ ID NO: 18. According to certain embodiments, the polypeptide encoded by the nucleotide sequence (18) consists of the amino acid sequence of SEQ ID NO: 18.

The polypeptide encoded by the nucleotide sequence (18) has the same functional property as the polypeptide of SEQ ID NO: 18 [ChdOII]. ChdOII is a FAD-dependent oxygenase. Specifically, ChdOII catalyses the hydroxylation of C-4 and C-12a. Like ChdOI, ChdOII possesses at the N-terminal end the typical conserved sequence G-X-G-2X-G-3X-A-6X-G (where X is any naturally occurring amino acid) which is involved in the FAD-cofactor binding (Mason, Cammack 1992). Accordingly, the polypeptide encoded by the nucleotide sequence (18) has FAD-dependent oxygenase activity.

According to certain embodiments, the polypeptide encoded by the nucleotide sequence (19) comprises an amino acid sequence having at least 85% sequence identity with the polypeptide of SEQ ID NO: 19. According to certain embodiments, the polypeptide encoded by the nucleotide sequence (19) comprises an amino acid sequence having at least 90% sequence identity with the polypeptide of SEQ ID NO: 19. According to certain embodiments, the polypeptide encoded by the nucleotide sequence (19) comprises an amino acid sequence having at least 95% sequence identity with the polypeptide of SEQ ID NO: 19. According to certain embodiments, the polypeptide encoded by the nucleotide sequence (19) consists of the amino acid sequence of SEQ ID NO: 19.

The polypeptide encoded by the nucleotide sequence (19) has the same functional property as the polypeptide of SEQ ID NO: 19 [ChdY]. ChdY is a cyclase, containing a conserved motif HXGTHXDXPXH (where X is any naturally occurring amino acid), characteristic of cyclase family PF04199 that is likely to form part of the active site. Specifically, ChdY catalyses an aldol condensation between C-5 and C-14 which results in the cyclization of the second ring (C). Accordingly, the polypeptide encoded by the nucleotide sequence (19) has cyclase activity.

As noted above, the present inventors have also identified two so far undiscovered regulatory genes within the chelocardin biosynthetic gene cluster of the wild-type chelocardin producer *A. sulphurea* which encode transcriptional activators belonging to the SARP and LuxR family, respectively. While these regulatory genes are not directly involved in the synthesis of chelocardin, they are expected to have a positive effect on the production chelocardin in a heterologous host as seen for homologous family members in oxytetracycline production.

Therefore, the gene cluster of the present invention may further comprise at least one of the nucleotide sequences (20) and (21):
(20) a nucleotide sequence encoding a polypeptide which comprises an amino acid sequence having at least 80%, such as at least 85%, sequence identity with the polypeptide of SEQ ID NO: 20 [SARP/ChdB]; and
(21) a nucleotide sequence encoding a polypeptide which comprises an amino acid sequence having at least 80%, such as at least 85%, sequence identity with the polypeptide of SEQ ID NO: 21 [LuxR/ChdC].

According to certain embodiments, the gene cluster comprises the nucleotide sequence (20).

According to certain embodiments, the gene cluster comprises the nucleotide sequence (21).

According to certain embodiments, the gene cluster comprises both nucleotide sequences (20) and (21).

According to certain embodiments, the polypeptide encoded by the nucleotide sequence (20) comprises an amino acid sequence having at least 85% sequence identity with the polypeptide of SEQ ID NO: 20. According to certain embodiments, the polypeptide encoded by the nucleotide sequence (20) comprises an amino acid sequence having at least 90% sequence identity with the polypeptide of SEQ ID NO: 20. According to certain embodiments, the polypeptide encoded by the nucleotide sequence (20) comprises an amino acid sequence having at least 95% sequence identity with the polypeptide of SEQ ID NO: 20. According to certain embodiments, the polypeptide encoded by the nucleotide sequence (20) consists of the amino acid sequence of SEQ ID NO: 20.

The polypeptide encoded by the nucleotide sequence (20) has the same functional property as the polypeptide of SEQ ID NO: 20 [ChdB]. ChdB is a transcriptional activator belonging to the family of Streptomyces antibiotic regulatory protein (SARP) family. It is homologous to OtcR, identified by Yin et al. (Yin et al. 2015), which acts as a positive pathway-specific activator of OTC biosynthesis leading to a significant increase in OTC production when overexpressed at the appropriate level. Accordingly, the polypeptide encoded by the nucleotide sequence (20) has transcriptional activator activity.

According to certain embodiments, the polypeptide encoded by the nucleotide sequence (21) comprises an amino acid sequence having at least 85% sequence identity with the polypeptide of SEQ ID NO: 21. According to certain embodiments, the polypeptide encoded by the nucleotide sequence (21) comprises an amino acid sequence having at least 90% sequence identity with the polypeptide of SEQ ID NO: 21. According to certain embodiments, the polypeptide encoded by the nucleotide sequence (21) comprises an amino acid sequence having at least 95% sequence identity with the polypeptide of SEQ ID NO: 21. According to certain embodiments, the polypeptide encoded by the nucleotide sequence (21) consists of the amino acid sequence of SEQ ID NO: 21.

The polypeptide encoded by the nucleotide sequence (21) has the same functional property as the polypeptide of SEQ ID NO: 21 [ChdC]. ChdC is a transcriptional activator with high similarity to transcriptional activators of the LuxR family, containing a conserved C-terminal helix-turn-helix (HTH) motif, characteristic of LuxR family (PROSITE PS00622). It is homologous to the regulatory protein OtcG from OTC biosynthesis, identified by Lesnik et al. (Lesnik et al. 2009)(Lesnik et al. 2015) and shown to have a conditionally positive role in OTC biosynthesis: its inactivation reduced the production of OTC by more than 40%. Accordingly, the polypeptide encoded by the nucleotide sequence (21) has transcriptional activator activity.

The various polypeptides encoded by the gene cluster of the present invention are summarized in **Table 1** below.

**Table 1: Genes of the gene cluster of the present invention**

| SEQ ID NO: | Gene Name | Functional property |
|---|---|---|
| 1 | *chdP* | Ketosynthase - alpha |
| 2 | *chdK* | Ketosynthase - beta |
| 3 | *chdS* | Acyl Carrier Protein |
| 4 | *chdQI* | Cyclase/ Aromatase |
| 5 | *chdQII* | Cyclase/ Aromatase |
| 6 | *chdX* | Cyclase |
| 7 | *chdL* | Acyl-CoA Ligase |
| 8 | *chdT* | Ketoreductase |
| 9 | *chdMI* | Methyltransferase |
| 10 | *chdMII* | Methyltransferase |
| 11 | *chdN* | Aminotransferase |
| 12 | *chdGIV* | Glycosyltransferase |
| 13 | *chdTn* | Transposase |
| 14 | *chdR* | Exporter |
| 15 | *chdA* | Transcriptional Regulator |
| 16 | *chdOI* | Oxygenase |
| 17 | *chdOIII* | Oxygenase |
| 18 | *chdOII* | Oxygenase |
| 19 | *chdY* | Cyclase |
| 20 | *chdB* | Transcriptional Activator |
| 21 | *chdC* | Transcriptional Activator |

The chelocardin biosynthetic pathway involving the above described polypeptide is shown in **Figure 2**. The polyketide skeleton of chelocardin is assembled from an acetate starter unit to which 9 malonate-derived acetate building blocks are attached by the action of the minimal PKS, namely ChdP, ChdK, ChdS. The polyketide chain is further subjected to C-9 ketoreduction and cyclisation/aromatisation, by the action of the ChdT ketoreductase, and the two cyclases/aromatases, ChdQII and ChdQI, respectively. After the cyclisation is completed by ChdY and ChdX, the nascent aromatic compound is subjected to post-PKS reactions, i.e. C-6 methylation, oxidations, C-4 amination, and C-9 methylation, catalysed by ChdMI methyltransferase, three oxygenases ChdOI/ChdOII/ChdOIII, aminotransferase ChdN, and methyltransferase ChdMII, respectively.

Further, sequence analysis of genomic DNA isolated from the wild-type chelocardin producer *A. sulphurea* has surprisingly revealed that ChdOII and ChdY, which are arranged in a successive order within the naturally occurring gene cluster, are expressed in the form of a fusion protein due to the absence of a stop codon at the end of the ChdOII encoding gene. Mutagenesis and complementation experiments have shown that the ChdOII and ChdY activities can be decoupled (Figure 6). Accordingly, each of ChdOII and ChdY can be expressed as individual polypeptides or as a fusion protein.

Therefore, according to certain embodiments, the nucleotide sequences (18) and (19) are linked in the gene cluster to form a fusion protein of the respective polypeptides encoded by them. The so formed fusion protein may thus comprise an amino acid sequence having at least 80%, such as at least 85%, sequence identity with the polypeptide of SEQ ID NO: 22 and has the same functional properties as the polypeptides of SEQ ID NO: 18 and SEQ ID NO: 19 [ChdOII+ ChdY]. Respective details are given above.

According to certain embodiments, the polypeptide encoded by the nucleotide sequence (22) comprises an amino acid sequence having at least 85% sequence identity with the polypeptide of SEQ ID NO: 22. According to certain embodiments, the polypeptide encoded by the nucleotide sequence (22) comprises an amino acid sequence having at least 90% sequence identity with the polypeptide of SEQ ID NO: 22. According to certain embodiments, the polypeptide encoded by the nucleotide sequence (22) comprises an amino acid sequence having at least 95% sequence identity with the polypeptide of SEQ ID NO: 22. According to certain embodiments, the polypeptide encoded by the nucleotide sequence (22) consists of the amino acid sequence of SEQ ID NO: 22.

In this respect, the present invention provides a (isolated) fusion protein comprises an amino acid sequence having at least 80%, such as 85%, sequence identity with the polypeptide of SEQ ID NO: 22, and a nucleic acid molecule comprising a nucleotide sequence encoding same. Said fusion protein has FAD-dependent oxygenase and cyclase activities.

According to certain embodiments, the fusion protein comprises an amino acid sequence having at least 85% sequence identity with the polypeptide of SEQ ID NO: 22. According to certain embodiments, the fusion protein comprises an amino acid sequence having at least 90% sequence identity with the polypeptide of SEQ ID NO: 22. According to certain embodiments, the fusion protein comprises an amino acid sequence having at least 95% sequence identity with the polypeptide of SEQ ID NO: 22. According to certain embodiments, the fusion protein consists of the amino acid sequence of SEQ ID NO: 22.

Sequence analysis of genomic DNA isolated from the wild-type chelocardin producer *A*. *sulphurea* has furthermore confirmed that ChdL and ChdOIII, which are arranged in a successive order within the naturally occurring gene cluster, are also expressed in the form of a fusion protein due to the absence of a stop codon at the end of the ChdL encoding gene.

Therefore, according to certain embodiments, the nucleotide sequences (7) and (17) are linked in the gene cluster to form a fusion protein of the respective polypeptides encoded by them. The so formed fusion protein may thus comprise an amino acid sequence having at least 80%, such as at least 85%, sequence identity with the polypeptide of SEQ ID NO: 23 and has the same functional properties as the polypeptides of SEQ ID NO: 7 and SEQ ID NO: 17 [ChdL+ChdOIII]. Respective details are given above.

In this respect, the present invention provides a (isolated) fusion protein comprises an amino acid sequence having at least 80%, such as 85%, sequence identity with the polypeptide of SEQ ID NO: 23, and a nucleic acid molecule comprising a nucleotide sequence encoding same. Said fusion protein has acyl-CoA ligase and oxygenase activities.

According to certain embodiments, the fusion protein comprises an amino acid sequence having at least 85% sequence identity with the polypeptide of SEQ ID NO: 23. According to certain embodiments, the fusion protein comprises an amino acid sequence having at least 90% sequence identity with the polypeptide of SEQ ID NO: 23. According to certain embodiments, the fusion protein comprises an amino acid sequence having at least 95% sequence identity with the polypeptide of SEQ ID NO: 23. According to certain embodiments, the fusion protein consists of the amino acid sequence of SEQ ID NO: 23.

The polypeptide encoding nucleotide sequences comprised by the gene cluster of the present invention may be present in any order. In other words, the ordering of the polypeptide encoding nucleotide sequences in the gene cluster of the present invention may be the same or different from the naturally occurring order of polypeptide encoding nucleotide sequences within the gene cluster found in the wild-type chelocardin producer *A*. *sulphurea.*

A representative, non-limiting, nucleotide sequence of the CHD biosynthetic cluster found in the wild-type chelocardin producer *A. sulphurea* is presented in SEQ ID NO: 26 (including additional 100 bp upstream and 100 bp downstream of the actual cluster sequence).

The present invention further relates to a DNA construct comprising the gene cluster according to the present invention.

The DNA construct may comprise at least one genetic element for facilitating expression of the polypeptide encoding nucleotide sequences comprised by the gene cluster of the present invention, such as at least one promoter. Suitably, the at least one promoter is operably linked to the gene cluster.

Promoters useful in accordance with the invention are any known promoters that are functional in a given host cell to cause the production of an mRNA molecule. Many such promoters are known to the skilled person. Such promoters include promoters normally associated with other genes, and/or promoters isolated from any bacteria. The use of promoters for protein expression is generally known to those of skilled in the art of molecular biology, for example, see Sambrook et al. (Sambrook, Russell 2001). The promoter employed may be inducible, such as a temperature inducible promoter (e.g., a pL or pR phage lambda promoters, each of which can be controlled by the temperature-sensitive lambda repressor c1857). The term "inducible" used in the context of a promoter means that the promoter only directs transcription of an operably linked nucleotide sequence if a stimulus is present, such as a change in temperature or the presence of a chemical substance ("chemical inducer"). As used herein, "chemical induction" according to the present invention refers to the physical application of an exogenous or endogenous substance (incl. macromolecules, e.g., proteins or nucleic acids) to a host cell. This has the effect of causing the target promoter present in the host cell to increase the rate of transcription. Alternatively, the promoter employed may be constitutive. The term "constitutive" used in the context of a promoter means that the promoter is capable of directing transcription of an operably linked nucleotide sequence in the absence of stimulus (such as heat shock, chemicals etc.). Examples of promoters that have been commonly used to express heterologous polypeptides, include, without limitation, P_{ermE*} promoter, Pm promoter, lac promoter, trp promoter, tac promoter, λpL promoter, T7 promoter, phoA promoter, araC promoter, xapA promoter, cad promoter and recA promoter.

Besides a promoter, the DNA construct may further comprise at least one genetic element selected from a 5' untranslated region (5'UTR) and 3' untranslated region (3' UTR). Many such 5' UTRs and 3' UTRs derived from prokaryotes are well known to the skilled person. Such genetic elements include 5' UTRs and 3' UTRs normally associated with other genes, and/or 5' UTRs and 3' UTRs isolated from any prokaryotes, notably bacteria. Usually, the 5' UTR contains a ribosome binding site (RBS), also known as the Shine Dalgarno sequence which is usually 3-10 base pairs upstream from the initiation codon. The ribosome binding site may be an RBS naturally associated with a prokaryotic gene or may be synthetic.

Further genetic elements may include, but are not limited to, an enhancer, a response element, a terminator sequence, a polyadenylation sequence, and the like.

The DNA construct may be a vector, such as an expression vector, or part of a vector, such as an expression cassette. Normally, such a vector remains extrachromosomal within the host cell which means that it is found outside of the nucleus or nucleoid region of the cell. However, it is also contemplated by the present invention that the DNA construct is stably integrated into at least one chromosome of a host cell. Means for stable integration into a chromosome of a host cell, e.g., by homologous recombination, are well known to the skilled person. For example, the DNA construct may contain one or more integration elements facilitating the integration into the chromosome of a host cell.

According to certain embodiments, the DNA construct is a vector, such as an expression vector. According to particular embodiments, the vector is a plasmid, such as a cosmid. The vector may be an integrative vector, such as an integrative plasmid.

According to certain embodiments, the DNA construct is an expression cassette.

The DNA construct may further include additional genes useful in modifying the structure of chelocardin. For example, it has been shown in EP2154249 (Petkovic et al.) and Lesnik et al. (Lesnik et al. 2015), that the chelocardin analogue 2-carboxamido-2-deacetyl-chelocardin (CDCHD) can be produced in a modified version of the wild-type producer *A*. *sulphurea* by introducing and expressing genes *oxyD* from the *S*. *rimosus* OTC gene cluster (*oxyD* alone or in combination with *oxyP*).

Therefore, according to certain embodiments, the DNA construct further comprises at least one nucleotide sequence selected from the nucleotide sequences (24) and (25):
(24) a nucleotide sequence encoding a polypeptide which comprises an amino acid sequence having at least 80%, such as at least 85%, sequence identity with the polypeptide of SEQ ID NO: 24 [OxyD]; and
(25) a nucleotide sequence encoding a polypeptide which comprises an amino acid sequence having at least 80%, such as at least 85%, sequence identity with the polypeptide of SEQ ID NO: 25 [OxyP].

According to certain embodiments, the DNA construct further comprises a nucleotide sequence encoding a polypeptide which comprises an amino acid sequence having at least 80%, such as at least 85%, sequence identity with the polypeptide of SEQ ID NO: 24 and which has the same functional property as the polypeptide of SEQ ID NO: 24 [OxyD].

According to certain embodiments, the polypeptide encoded by the nucleotide sequence (24) comprises an amino acid sequence having at least 85% sequence identity with the polypeptide of SEQ ID NO: 24. According to certain embodiments, the polypeptide encoded by the nucleotide sequence (24) comprises an amino acid sequence having at least 90% sequence identity with the polypeptide of SEQ ID NO: 24. According to certain embodiments, the polypeptide encoded by the nucleotide sequence (24) comprises an amino acid sequence having at least 95% sequence identity with the polypeptide of SEQ ID NO: 24. According to certain embodiments, the polypeptide encoded by the nucleotide sequence (24) consists of the amino acid sequence of SEQ ID NO: 24.

The polypeptide encoded by the nucleotide sequence (24) has the same functional property as the polypeptide of SEQ ID NO: 24 [OxyD]. OxyD is an amidotransferase catalysing the amidation of the acetyl group at C2 in the chelocardin structure, resulting in a carboxyamido moiety (see, e.g., Lesnik et al., 2015). Accordingly, the polypeptide encoded by the nucleotide sequence (24) has amidotransferase activity.

According to certain embodiments, the DNA construct further comprises a nucleotide sequence encoding a polypeptide which comprises an amino acid sequence having at least 80%, such as at least 85%, sequence identity with the polypeptide of SEQ ID NO: 25 and which has the same functional property as the polypeptide of SEQ ID NO: 25 [OxyP].

According to certain embodiments, the polypeptide encoded by the nucleotide sequence (25) comprises an amino acid sequence having at least 85% sequence identity with the polypeptide of SEQ ID NO: 25. According to certain embodiments, the polypeptide encoded by the nucleotide sequence (25) comprises an amino acid sequence having at least 90% sequence identity with the polypeptide of SEQ ID NO: 25. According to certain embodiments, the polypeptide encoded by the nucleotide sequence (25) comprises an amino acid sequence having at least 95% sequence identity with the polypeptide of SEQ ID NO: 25. According to certain embodiments, the polypeptide encoded by the nucleotide sequence (25) consists of the amino acid sequence of SEQ ID NO: 25.

The polypeptide encoded by the nucleotide sequence (25) has the same functional property as the polypeptide of SEQ ID NO: 25 [OxyP]. OxyP is an acyltransferase which suppresses priming by acetate by removing the competing acetyl units, leading to increase in proportion of CDCHD compared to CHD. Accordingly, the polypeptide encoded by the nucleotide sequence (25) has acyltransferase activity.

Any of the nucleotide sequences (24) and (25) may be under control of the same promoter as the nucleotide sequences of the gene cluster or under control of a different promoter.

Moreover, while the nucleotide sequences (24) and (25) are described to be comprised by the DNA construct, it is also contemplated by the present invention that any of these nucleotide sequences is/are included in the gene cluster described herein.

As further demonstrated herein, providing an additional copy of CHD efflux pump gene *chdR* improves self-resistance of a host cell during heterologous expression of CHD (Figure 3). Particularly, an additional copy *chdR* led to slightly increased production yields of CHD up to approx. 60 mg/L.

Accordingly, the DNA construct of the present invention may further comprise an additional nucleotide sequence encoding a polypeptide which comprises an amino acid sequence having at least 80%, such as at least 85%, sequence identity with the polypeptide of SEQ ID NO: 14 and which is capable of acting as an exporter.

According to certain embodiments, the polypeptide encoded by the additional nucleotide sequence comprises an amino acid sequence having at least 85% sequence identity with the polypeptide of SEQ ID NO: 14. According to certain embodiments, the polypeptide encoded by the additional nucleotide sequence comprises an amino acid sequence having at least 90% sequence identity with the polypeptide of SEQ ID NO: 14. According to certain embodiments, the polypeptide encoded by the additional nucleotide sequence (14) comprises an amino acid sequence having at least 95% sequence identity with the polypeptide of SEQ ID NO: 14. According to certain embodiments, the polypeptide encoded by the nucleotide sequence (14) consists of the amino acid sequence of SEQ ID NO: 14.

The additional nucleotide sequences may be under control of the same promoter as the nucleotide sequences of the gene cluster or under control of a different promoter.

The present invention further provides a recombinant host cell comprising the gene cluster of the present invention or the DNA construct of the present invention, wherein the gene cluster or DNA construct is heterologous to said host cell. According to particular embodiments, said recombinant host cell heterologously expresses the polypeptides encoded by the gene cluster which allows for the biosynthesis of chelocardin or an analogue thereof.

The gene cluster or DNA construct may be extrachromosomal, e.g., in the form of a extrachromosomal vector, or it may be integrated into one or more chromosomes of said host cell. According to certain embodiments, the gene cluster or DNA construct is extrachromosomal. According to certain embodiments, the gene cluster of DNA constructed is integrated into one or more chromosomes of said host cell.

The recombinant host cell may further comprise at least one nucleotide sequence selected from the nucleotide sequences (24) and (25) as described above.

According to certain embodiments, the recombinant host cell comprises a nucleotide sequence encoding a polypeptide which comprises an amino acid sequence having at least 80%, such as at least 85%, 90%, or 95%, sequence identity with the polypeptide of SEQ ID NO: 24 and has amidotransferase. According to particular embodiments, the recombinant host cell heterologously expresses a polypeptide which comprises an amino acid sequence having at least 80%, such as at least 85%, such as at least 85%, 90%, or 95%, sequence identity with the polypeptide of SEQ ID NO: 24 and which has amidotransferase activity

According to certain embodiments, the recombinant host cell comprises a nucleotide sequence encoding a polypeptide which comprises an amino acid sequence having at least 80%, such as at least 85%, such as at least 85%, 90%, or 95%, sequence identity with the polypeptide of SEQ ID NO: 25 and has acyltransferase activity. According to particular embodiments, the recombinant host cell heterologously expresses a polypeptide which comprises an amino acid sequence having at least 80%, such as at least 8%, such as at least 85%, 90%, or 95%, sequence identity with the polypeptide of SEQ ID NO: 25 and which has acyltransferase activity

The at least one nucleotide sequence selected from the nucleotide sequences (24) and (25) may be included in the gene cluster or DNA construct. However, it is also contemplated by the present invention that any of these nucleotide sequences is/are present in a further DNA construct, such as a further vector, which is different from the DNA construct comprising the gene cluster. Such further DNA construct may comprise at least one genetic element for facilitating expression of the polypeptide encoding nucleotide sequence(s) comprised thereby, such as those genetic elements detailed above, notably at least one promoter operably linked to the polypeptide encoding nucleotide sequence(s). Such further DNA construct may be extrachromosomal or integrated into one or more chromosomes of said host cell.

Therefore, according to certain embodiments, the recombinant host cell comprises a further (second) DNA construct comprising at least one nucleotide sequence selected from the nucleotide sequences (24) and (25) above.

The recombinant host cell may further comprise an additional nucleotide sequence encoding a polypeptide which comprises an amino acid sequence having at least 80%, such as at least 85%, 90% or 95%, sequence identity with the polypeptide of SEQ ID NO: 14 and whichis capable of acting as an exporter.

The additional nucleotide sequence encoding a polypeptide comprises an amino acid sequence having at least 80% sequence identity with the polypeptide of SEQ ID NO: 14, may be included in the gene cluster or DNA construct. However, it is also contemplated by the present invention that the additional nucleotide sequence encoding a polypeptide comprises an amino acid sequence having at least 80% sequence identity with the polypeptide of SEQ ID NO: 14 is present in a further (second or third) DNA construct, such as a further vector, which is different from the DNA construct comprising the gene cluster. Such further DNA construct may comprise at least one genetic element for facilitating expression of the polypeptide encoding nucleotide sequence(s) comprised thereby, such as those genetic elements detailed above, notably at least one promoter operably linked to the polypeptide encoding nucleotide sequence(s). Such further DNA construct may be extrachromosomal or integrated into one or more chromosomes of said host cell.

Therefore, according to certain embodiments, the recombinant host cell comprises a further (second or third) DNA construct comprising a nucleotide sequence encoding a polypeptide which comprises an amino acid sequence having at least 80%, such as at least 85%, 90% or 95%, sequence identity with the polypeptide of SEQ ID NO: 14 and which is capable of acting as an exporter.

The recombinant host cell in accordance with the invention can be produced from any suitable host organism, including single-celled or multicellular microorganisms such as bacteria, yeast, fungi, algae and plant.

According to certain embodiments, a recombinant host cells in accordance is a prokaryotic organism, such as a bacterium.

According to certain embodiments, a recombinant host cells in accordance is a bacterium, such as a bacterium of the order *Actinomycetales.*

A bacterial host cells may be selected from Gram-positive and Gram-negative bacteria. Non-limiting examples for Gram-negative bacterial host cells include species from the genus *Escherichia,* such as *Escherichia coli.* Non-limiting examples of Gram-positive bacterial host cells include species from the genera *Streptomyces, Amycolatopsis* and *Nocardia.*

According to certain embodiments, the recombinant host cell is a bacterium belonging to a genus selected from the group consisting of *Streptomyces*, *Amycolatopsis* and *Nocardia*, such as a bacterium selected from the group consisting of *Streptomyces lividans, Streptomyces coelicolor, Streptomyces albus, Streptomyces rimosus, Amycolatopsis mediterranei, Amycolatopsis orientalis* and *Nocardia spp..*

According to certain embodiments, the recombinant host cell is *Streptomyces lividans.* According to certain embodiments, the recombinant host cell is *Streptomyces coelicolor.* According to certain embodiments, the recombinant host cell is *Streptomyces albus.* According to certain embodiments, the recombinant host cell is *Streptomyces rimosus.* According to certain embodiments, the recombinant host cell is *Amycolatopsis mediterranei,* According to certain embodiments, the recombinant host cell is *Amycolatopsis orientalis.* According to certain embodiments, the recombinant host cell is *Nocardia spp..*

The present invention further provides a process for the biosynthetic production of a tetracycline (notably chelocardin or an analogue thereof), said process comprises the steps of a) cultivating a recombinant host cell as described herein in the presence of a suitable substrate, such as a fermentable carbon substrate, under conditions conducive to the production of said tetracycline and, optionally, b) recovering the tetracycline from the cultivation medium employed in cultivation.

The medium employed may be any conventional medium suitable for culturing the host cell in question, and may be composed according to the principles of the prior art. The medium will usually contain all nutrients necessary for the growth and survival of the respective host cell, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells, such as *B*. *subtilis*, *L. lactis* or *E. coli* cells, including minimal media and rich media such as Luria Broth (LB), M9 media, M17 media, SA media, MOPS media, Terrific Broth, YT and others. Suitable media for culturing eukaryotic cells, such as yeast cells, are RPMI 1640, MEM, DMEM, all of which may be supplemented with serum and/or growth factors as required by the particular host cell being cultured. The medium for culturing eukaryotic cells may also be any kind of minimal media such as Yeast minimal media.

Suitable conditions for culturing the respective host cell are well known to the skilled person. Typically, the recombinant host cell is cultured at a temperature ranging from about 23 to about 60°C, such as from about 25 to about 40°C, such as at about 30 to about 37°C, such as about 30°C. The pH of the medium may range from pH 1.0 to pH 14.0, such as from about pH 1 to about pH 2, from about pH 4 to about pH 11, from about pH 5 to about pH 10, from about pH 6 to about pH 10, or from about pH 7 to about pH 9.5, e.g. at pH 6.0, pH pH 7.0, pH. 7.5, pH 8.0, pH 8.5, pH 9.0, pH 9.5, pH 10.0, pH 10.5 or pH 11.0.

The process may further comprise b) recovering the tetracycline from the cultivation medium. The tetracycline may be recovered by conventional method for isolation and purification chemical compounds from a medium. Well-known purification procedures include centrifugation or filtration, precipitation, and chromatographic methods such as e.g. ion exchange chromatography, gel filtration chromatography, etc.

The present invention also pertains to a tetracycline (notably chelocardin or an analogue thereof) produced by the foregoing process. Particularly, the present invention pertains to a tetracycline having structure I: 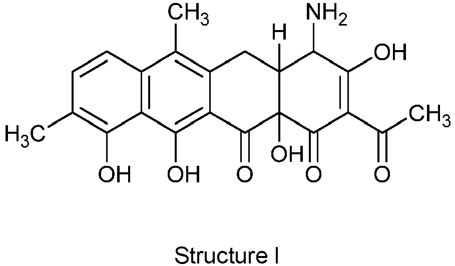 optionally as a stereoisomer, including enantiomers and diastereomers, or in form of a mixture of at least two stereoisomers, including enantiomers and/or diastereomers, in any mixing ratio, or a corresponding salt thereof, or a corresponding solvate thereof.

According to certain embodiments, the compound of structure I is in the form of a stereoisomer. According to particular embodiments, the stereoisomer has the structure II

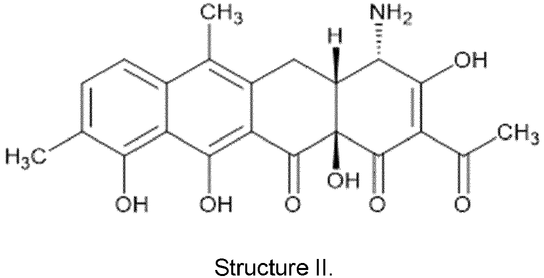

The present invention further pertains to a tetracycline having structure III: 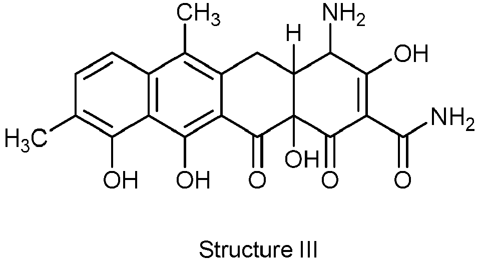 optionally as a stereoisomer, including enantiomers and diastereomers, or in form of a mixture of at least two stereoisomers, including enantiomers and/or diastereomers, in any mixing ratio, or a corresponding salt thereof, or a corresponding solvate thereof.

According to certain embodiments, the compound of structure III is in the form of a stereoisomer. According to particular embodiments, the stereoisomer has the structure IV

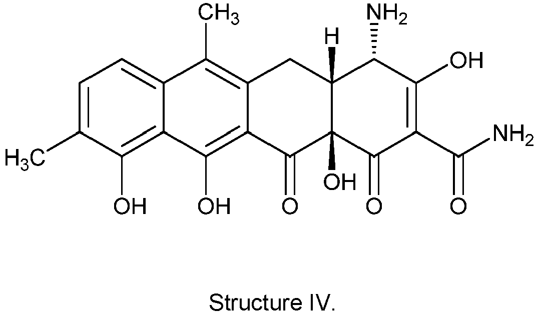

### Certain definitions

"Gene cluster", as used herein, shall be understood to be a totality of DNA coding for polypeptides required to catalyse a certain biochemical pathway. A gene cluster can be on a single DNA molecule, or can be on multiple DNA molecules, e.g. in form of a DNA library.

"Heterologous", as used herein, means that a polynucleotide or polypeptide is normally not found in or made (i.e. expressed) by the host cell, but derived from a different organism or made synthetically. Moreover, a host cell transformed with a gene cluster or DNA construct described herein which is not normally present in the host cell would be considered heterologous for the purpose of the present invention.

"Host cell" as used herein refers to a living cell or microorganism that is capable of reproducing its genetic material and along with it recombinant genetic material that has been introduced into it - e.g., via heterologous transformation.

"Recombinant", as used herein, with reference to, e.g., a host cell, polynucleotide, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant host cells expressing a gene or gene cluster that is not found within the native (non-recombinant) form of the cell or express native genes that are otherwise expressed at a different level.

"Isolated", as used herein, means that a polynucleotide (such as the gene cluster of the present invention) or polypeptide (such as a fusion protein of the present invention) is removed from its original environment (e.g., the environment in which it naturally occurs). Particularly, a polynucleotide or polypeptide which has been separated from some or all of the coexisting materials in the natural system is considered isolated.

"Expression", as used herein, includes any step involved in the production of a polypeptide (e.g., encoded enzyme) including, but not limited to, transcription, post-transcriptional modification, translation, post-translational modification, and secretion.

"Vector", as used herein, refers to a nucleic acid molecule capable of transporting another nucleic acid molecule to which it has been linked. One type of vector is a "plasmid", which refers to a circular double stranded nucleic acid loop into which additional nucleic acid segments can be ligated. Certain vectors are capable of directing the expression of genes to which they are operatively linked. Such vectors are referred to herein as "expression vectors". Certain other vectors are capable of facilitating the insertion of an exogenous nucleic acid molecule into a chromosome of a host cell, such as a bacterium. Such vectors are referred to herein as "transformation vectors". In general, vectors of utility in recombinant nucleic acid techniques are often in the form of plasmids. In the present specification, "plasmid" and "vector" can be used interchangeably as the plasmid is the most commonly used form of a vector. Large numbers of suitable vectors are known to those of skill in the art and commercially available.

"Promoter", as used herein, refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A suitable "promoter" is generally one which is capable of supporting the initiation of transcription in a bacterium of the invention, causing the production of an mRNA molecule.

"Operably linked", as used herein, refers to a juxtaposition wherein the components described are in a relationship permitting them to function in their intended manner. A control sequence "operably linked" to a coding sequence is ligated in such a way that expression of the coding sequence is achieved under conditions compatible with the control sequence. A promoter sequence is "operably-linked" to a gene when it is in sufficient proximity to the transcription start site of a gene to regulate transcription of the gene.

The terms "cassette", "expression cassette" and "gene expression cassette" refer to a segment of DNA that can be inserted into a target nucleic acid molecule, such as a vector or genomic DNA, at specific restriction sites or by homologous recombination. The segment of DNA comprises a nucleotide sequence that encodes a polypeptide of interest, and the cassette and restriction sites are designed to ensure insertion of the cassette in the proper reading frame for transcription and translation. An expression cassette of the invention may also comprise one or more elements that allow for expression of a nucleotide sequence encoding a polypeptide of interest in a host cell. These elements may include, but are not limited to: a promoter, an enhancer, a response element, a terminator sequence, a polyadenylation sequence, and the like.

"Extrachromosomal", as used herein, refers to a DNA that is found outside of a chromosome of a cell in question.

"Fusion protein", as used herein, refers to a protein created through the joining of two or more nucleotide sequences that otherwise would code for separate proteins. This typically occurs through the absence of a stop codon from a DNA sequence coding for the first protein, thereby appending the DNA sequence of the second protein in frame. The DNA sequence will then be expressed by a cell as a single protein with functional properties derived from each of the original proteins. A fusion protein contains all functional domains of the parent proteins.

"% sequence identity" or "% identity", as used herein, refers to identity between two nucleotide or amino acid sequences. Identity can be determined by comparing a position in each sequence which may be aligned for purposes of comparison. When a position in the compared sequence is occupied by the same base or amino acid, then the molecules are identical at that position. A degree of identity between nucleic acid or amino acid sequences is a function of the number of identical or matching nucleotides or amino acids at positions shared by the nucleotide or amino acid sequences, respectively. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting.

More particularly, "% sequence identity" of an amino acid sequence to a reference amino acid sequence, as used herein, defines the % sequence identity calculated from the two amino acid sequences as follows: The sequences are aligned using Version 9 of the Genetic Computing Group's GAP (global alignment program), using the default BLOSUM62 matrix with a gap open penalty of -12 (for the first null of a gap) and a gap extension penalty of -4 (for each additional null in the gap). After alignment, percentage identity is calculated by expressing the number of matches as a percentage of the number of amino acids in the reference amino acid sequence.

Where a numerical limit or range is stated herein, the endpoints are included. Also, all values and sub ranges within a numerical limit or range are specifically included as if explicitly written out.

Having generally described this invention, a further understanding can be obtained by reference to certain specific examples, which are provided herein for purposes of illustration only, and are not intended to be limiting unless otherwise specified.

### EXAMPLES

### Materials and methods

### Bacterial strains and culture conditions

*Amycolatopsis sulphurea* NRRL2822 was used for production of CHD and as a source of DNA and for microbiological manipulations. *Streptomyces rimosus* M4018 was used as a source of DNA (Rhodes et al. 1984). S. *albus* del14 (Myronovskyi et al., 2018) was used for heterologous expression of CHD biosynthetic gene cluster. *Escherichia coli* DH10β was used for standard cloning procedures (Sambrook, Russell 2001), *E. coli* ET12567 (MacNeil et al. 1992) and SCS110 (Stratagene) strains for isolation of non-methylated plasmid DNA, suitable for transformation of *A. sulphurea,* and *E. coli* GB2006 (Gene Bridges) for preparation of *A. sulphurea* cosmid library. *Escherichia coli* ET12567 carrying PUZ8002 plasmid (Paget et al. 1999) was used as a donor strain for intergeneric conjugation with S. *albus* del14. Soya mannitol (MS) agar and tryptone soy broth (TSB) (Kieser et al. 2000) with incubation at 30 °C were used for sporulation and cultivation of actinomycetes in liquid medium, respectively. For CHD and CHD analogues production, *A. sulphurea* was cultivated in CH-V seed medium (1.5 % soy flour, 0.1 % yeast extract, 1.5 % glucose, 0.5 % NaCl, 0.1 % CaCO₃, pH 7.0) and CH-F2 production medium (2 % soy flour, 0.5 % yeast extract, 0.2 % CaCO₃, 0.05 % citric acid, 5 % glucose, pH 7.0) (adapted from (Oliver et al. 1962; Oliver, Sinclair) and (Mitscher et al. 1983)). Cultivations were performed in Falcon tubes at 30 °C on a rotary shaker at 220 rpm for 36h in seed medium with 15 % (v/v) used to inoculate CH-F2 production medium and cultivated for further 7 days under the same conditions. For heterologous production of CHD and CHD analogues, S. *albus* was cultivated in TSB seed medium and four different production media: CH-F2, DNPM (4 % dextrin, 0.75 % soytone, 0.5 % baking yeasts, 2.1 % MOPS, pH 6.8 (Bilyk et al. 2016)), NL5Y (0.1 % NaCl, 0.1 % KH₂PO₄, 0.05 % MgSO₄ × 7H₂O, 2.5 % glycerol, 0.584 % L-glutamin, 0.2 % trace elements solution, 1 % yeast extract, pH 7.3 (Bilyk et al. 2017), and SG1 (2 % glucose, 0.5 % yeast extract, 1 % soytone, 0.2 % CaCO₃, pH 7.2 (Koshla et al. 2017)). Cultivations were performed in Falcon tubes at 30 °C on a rotary shaker at 220 rpm for 36h in seed medium with 5 % (v/v) used to inoculate production media and cultivated for further 7 days under the same conditions. For transformation of *A. sulphurea,* S27M and R2L media were used (Madon, Hutter 1991). Apramycin (Apr; 200 µg mL⁻¹), erythromycin (Erm; 20 µg mL⁻¹) or kanamycin (Kan; 300 µg mL⁻¹) was used for selection of *A. sulphurea* transformants on S27M. For further subcultivation of *A. sulphurea* transformants, MS was supplemented with Apr (400 µg mL⁻¹), Erm (20 µg mL⁻¹) or kanamycin (400 µg mL⁻¹). For intergeneric conjugation between S. *albus* and *E. coli* MS medium, supplemented with 10 mM MgCl₂ was used. Apramycin (50 µg mL⁻¹) together with nalidixic acid (25 µg mL⁻¹) was used for selection of *S*. *albus* exconjugants on MS. For selection of *E. coli* transformants, ampicillin (Amp; 100 µg mL⁻¹), Apr (50 µg mL⁻¹), Kan (25 µg mL⁻¹) or chloramphenicol (Cm; 10 µg mL⁻¹) were added into LB medium.

### DNA isolation and manipulation

Isolation and manipulation of DNA in *E. coli* (Table 2) were carried out according to standard protocols (Sambrook, Russell 2001; Kieser et al. 2000). Transformation of *A. sulphurea* NRRL2822 was carried out by the protocol for transformation of *A. mediterranei* (Madon, Hutter 1991), using vectors pAB03 and pNV18 already described previously (Lukezic et al. 2013). Cosmids were introduced into *S*. *albus* del14 via conjugation (Kieser et al. 2000).

### Sequencing of genomic DNA

Salting out procedure (Kieser et al. 2000) was used to isolate genomic DNA from *A*. *sulphurea* which was sequenced by Illumina sequencing.

### Preparation of A. sulphurea cosmid library

Genomic DNA was partially digested with *Sau3A*I and the DNA fragments of approximate size 35-40 kb were ligated into the *BamH*I site of replicative conjugative cosmid vector pOJ456, a modified version of the pOJ436 vector (Bierman et al. 1992), where 2,5 kb ΦC31 integrase cassette was excised with *Hind*III (overhangs were filled in with Klenow polymerase) and replaced with 2,5 kb pSG5 replication cassette excised with *Eco81*I and *Sph*I (overhangs were filled in with Klenow polymerase) from medium copy number vector pKC1139 (Bierman et al. 1992). The ligated DNA was packaged into phage particles (Gigapack III Gold Packaging kit, Agilent Technologies) and introduced into *E. coli* GB2006.

### Identification and sequencing of cosmid carrying CHD biosynthetic gene cluster

The cosmid library was screened by combining all 3400 colonies and streaking the mixture onto LB agar plates supplemented with 3 µg mL⁻¹ of CHD to select for CHD-resistant single colonies expressing ChdR efflux pump encoded in the CHD biosynthetic gene cluster. 18 positive clones were selected to isolate cosmid DNA and additional PCR screening was carried out using the primer pairs CobU1/CobU2 and glu1/glu2 (Table 3), designed to anneal to the flanking regions of CHD biosynthetic gene cluster. Based on the PCR screen, two cosmids were selected for complete sequencing by Illumina sequencing, resulting in confirmation of cosmid pOJ456CHD12, carrying the complete CHD biosynthetic gene cluster, whose correct and complete sequence was also identified from genomic DNA sequence of *A. sulphurea.*

### Variations of cosmids carrying CHD biosynthetic gene cluster

34kbp CHD biosynthetic gene cluster from pOJ456CHD12 was cloned via Spel and *Xba*I into integrative conjugative cosmids pOJ436, pOJ436e*chdR, pOJ436e*oxyDP, or pOJ436e*oxyDPchdR, resulting in pOJ436CHD12, pOJ436e*chdRCHD12, pOJ436e*oxyDPCHD12, and pOJ436e*oxyDPchdRCHD12, respectively. pOJ436e*chdR, pOJ436e*oxyDP, or pOJ436e*oxyDPchdR were constructed from pOJ436 by introducing 1,8 kb, 3,2 kb and 4,7 kb fragments, carrying *chdR, oxyDP* and *oxyDPchdR* genes, respectively, all under the control of P_{ermE*} promoter. Fragments were excised with *Ecl136*II (overhang was filled in with Klenow polymerase) and *Xba*I from plasmids pAB03e*chdR, pAB03e*oxyDP and pAB03e*oxyDPchdR, respectively, and used to replace the 1,9 kb fragment in pOJ436, excised with *Nru*I (overhang was filled in with Klenow polymerase) and *Xba*I*.* pAB03e*chdR was constructed by cloning 1,5 kb *chdR* gene, amplified by PCR using primers chdRF and chdRR, digested with *Nde*I and *Xba*I and ligated into pAB03e* (pAB03 vector with P_{ermE*} promoter instead of actII-ORF4/P_{actI} activator/promoter system). pAB03e*oxyD was constructed by cloning *oxyD* gene, excised from pAB03oxyD (Lesnik et al., 2015) with *Nde*I and *Xba*I and ligated into pAB03e*. pAB03e*oxyDP was constructed by cloning *oxyP* gene, excised from pAB03oxyDP (Lesnik et al., 2015) with *Xba*I*,* into *Xba*I site of pAB03e*oxyD downstream of *oxyD* gene. pAB03e*oxyDPchdR was constructed by cloning *chdR* gene, excised from pAB03e*chdR with *Cla*I and *Hind*III (overhangs were filled in with Klenow polymerase), into *Xba*I (overhangs were filled in with Klenow polymerase) site of pAB03e*oxyDP downstream of *oxyP* gene.

### Heterologous expression of CHD biosynthetic gene cluster

Cosmids carrying the CHD biosynthetic gene cluster, pOJ456CHD12, pOJ436CHD12, pOJ436e*chdRCHD12, pOJ436e*oxyDPCHD12 and pOJ436e*oxyDPchdRCHD12, and empty control cosmids, pOJ456, pOJ436, pOJ436e*chdR, pOJ436e*oxyDP and pOJ436e*oxyDPchdR, were transformed into *E. coli* ET12567 (MacNeil et al. 1992) carrying PUZ8002 which was then used as donor strain for intergeneric conjugation with S. *albus* del14. MS plates supplemented with 10 mM MgCl₂ were overlaid with Apr and nalidixic acid after overnight incubation. Each exconjugant was further repatched onto MS agar containing Apr (50 µg mL⁻¹) and nalidixic acid (25 µg mL⁻¹), followed by inoculation into TSB medium as seed culture for production media CH-F2 (2 % soy flour, 0.5 % yeast extract, 0.2 % CaCO₃, 0.05 % citric acid, 5 % glucose, pH 7.0) (adapted from (Oliver et al. 1962; Oliver, Sinclair) and (Mitscher et al. 1983)), DNPM (4 % dextrin, 0.75 % soytone, 0.5 % baking yeasts, 2.1 % MOPS, pH 6.8 (Bilyk et al. 2016)), NL5Y (0.1 % NaCl, 0.1 % KH₂PO₄, 0.05 % MgSO₄ × 7H₂O, 2.5 % glycerol, 0.584 % L-glutamin, 0.2 % trace elements solution, 1 % yeast extract, pH 7.3 (Bilyk et al. 2017)), and SG1 (2 % glucose, 0.5 % yeast extract, 1 % soytone, 0.2 % CaCO₃, pH 7.2 (Koshla et al. 2017)). Cultivations were performed in Falcon tubes at 30 °C on a rotary shaker at 220 rpm for 36h in seed medium with 5 % (v/v) used to inoculate production media and cultivated for further 7 days under the same conditions. Culture broths were extracted and analysed by LC-MS to check for production of CHD or CHD analogues.

### Site-directed mutagenesis of chdY in A. sulphurea

The mutation was introduced using the double cross-over approach to replace the target gene with the mutated gene. Catalytic residue Gly176 of ChdY was replaced by Ser. First, vector for homologous recombination was constructed: *ermE* gene was amplified by PCR using primers FSB01C and FSB02C (Table 3) and later digested with *EcoR*I and *Xba*I to obtain the 1,6 kb fragment, which was ligated into pNV18 to obtain pNV18Erm. To mutate residue Gly176 via site-directed mutagenesis, 0,7 kb upstream and 0,7 kb downstream fragments were amplified, by using primer pairs chdYserLF/chdYserLR and chdYserRF/chdYserRR. Primers labeled with LR (left reverse) and RF (right forward) were designed to anneal to the region containing the catalytic residue Gly176 and introduce the desired mutation (Table 3). Third PCR was performed with outer set of primers, chdYserLF and chdYserRR, using previous two PCR products as template, which were overlapping in the region where the mutation was introduced, yielding 1,4 kb fragment. Resulting fragment was digested with *Sph*I and Spel and ligated into pNV18Erm to obtain pNV18ErmchdYser, which was transformed into E. coli SCS110 (Stratagene) to obtain the non-methylated plasmid, which was then introduced into *A. sulphurea* via direct transformation of mycelium (Madon, Hutter 1991). S27M plates were overlaid with Erm after overnight incubation. Each transformant colony was further re-patched onto MS agar containing Erm and subcultivated. After three or more subcultivations in TSB without antibiotic, Erm-sensitive (Erm^{s}) colonies (secondary recombinants) were isolated. To confirm that secondary recombinants contain the introduced mutation and are not revertants to wild-type, colony PCR using the outer pair of primers labeled with LF (left forward) and RR (right reverse), chdYserLF and chdYserRR, respectively, followed by DNA sequencing, was performed.

### Homologous expression of wild-type chdY and chdOII genes in mutant strain of A. sulphurea

*A. sulphurea* mutant obtained through previously described site-directed mutagenesis approach was complemented with wild-type genes *chdY* and *chdOII-chdY* from A. *sulphurea.* Genes for ChdY (cyclase) and ChdOII-ChdY (oxygenase-cyclase fusion) were amplified by PCR using chdYF/chdYR or chdOIIF/chdYR sets of primers, respectively, and genomic DNA of *A. sulphurea* as a template. PCR products were digested with *Nde*I and *Xba*I and separately cloned into pAB03, resulting in pAB03chdY and pAB03chdOII-chdY (Table 2), respectively. Constructs were confirmed by sequencing, transformed into *E. coli* SCS110 and introduced into *A. sulphurea* mutant via direct transformation of mycelium (Madon, Hutter 1991). Plasmids pAB03chdY and pAB03chdOII-chdY were separately integrated into *A. sulphurea* ChdY-G176S.

### Heterologous expression of oxyN in mutant strain of A. sulphurea

*A. sulphurea* mutant was complemented also with heterologous gene *oxyN.* OxyN (cyclase) (Zhang et al. 2006) from S. *rimosus* M4018 (Rhodes et al. 1984) was amplified by PCR using primers oxyN Fw and oxyN Rv (Table 3), digested with *Nde*I and *Xba*I and cloned into pAB03 vector, resulting in pAB03oxyN(Table 2). Construct was confirmed by sequencing, transformed into *E. coli* SCS110 and introduced into mutated strain of *A. sulphurea* via direct transformation of mycelium (Madon, Hutter 1991). Plasmid pAB03oxyN was integrated into *A. suphurea* ChdY-G176S.

**Table 2. Bacterial strains and plasmids used herein^{[a]}**

| Strain or plasmid | Relevant characteristics | Reference or source |
|---|---|---|
| ***Escherichia coli*** | | |
| DH10β | F- *endA*1 *recA*1 *galE*15 *galK*16 *upG rpsL Δ*/*acX*74 Φ80/*acZ*ΔM15 araD139 Δ(*ara-leu*)7697 *mcrA* Δ(*mrr-hsdRMS-mcrBC)* λ- | Invitrogen |
| ET12567 | F- dam13::Tn9, *dcm6, hsdM, hsdR, rec*F143::Tn*1I*, *galK*2*, galT*22*,* ara14, /*acY*1*, xyl*5*, leuB6, thi1, ton*A31, *rpsL*136*, hisG4, tsx78, mtl*1 *glnV*44 | (MacNeil et al. 1992) |
| SCS110 | *rpsL* (Str^{r}) *thr leu endA thi-1 lacY galK galT ara tonA tsx dam dcm supE44* Δ(*lac-proAB*) [F' *traD36 proAB lacI*^{q}*Z*Δ*M15*] | Stratagene |
| GB2006 | δM109 *rpsL-* Δ*rfuA* | Gene Bridges |

| ***Amycolatopsis sulphurea*** | | |
|---|---|---|
| NRRL 2822 | Wild-type producer of chelocardin | ARS Culture Collection |

| ***Streptomyces rimosus*** | | |
|---|---|---|
| M4018 | Producer of oxytetracycline | (Rhodes et al. 1984) |

| ***Streptomyces albus*** | | |
|---|---|---|
| S. ***albus*** del14 | Host strain for heterologous expression | (Myronovsk yi et al. 2018) |

| **Plasmids** | | |
|---|---|---|
| pNV18 | Kan^{r}, *lacZα* | (Chiba et al. 2007) |
| **pNV18Erm** | Kan^{r}, Erm^{r}, *lacZα* | This study |
| pAB03 | pSET152-derived, containing ΦBT, Apr^{r} | (Lukezic et al. 2013) |
| pAB03oxyD | oxyD cloned into pAB03 | (Lesnik et al. 2015) |
| pAB03oxyDP | *oxyD* and *oxyP* cloned into pAB03 | (Lesnik et al. 2015) |
| pOJ436 | pSET152-derived cosmid, containing ΦC31, Apr^{r} | (Bierman et al. 1992) |
| pOJ436CHD12 | pOJ436 cosmid carrying CHD biosynthetic cluster | This study |
| pKC1139 | bifunctional *oriT* RK2 vector, pSG5 ori, Apr^{r} | (Bierman et al. 1992) |
| pOJ456 | pOJ436-derived cosmid, ΦC31 integrase cassette replaced with pSG5 replication cassette, Apr^{r} | This study |
| pOJ456CHD12 | pOJ456 cosmid carrying CHD biosynthetic cluster | This study |
| pAB03e* | pAB03 vector with P_{ermE*} promoter instead of actll-ORF4/Pactl activator/promoter system | Acies Bio d.o.o. |
| pAB03e*chdR | pAB03e* carrying *chdR* gene | This study |
| pAB03e*oxyD | pAB03e* carrying *oxyD* gene | This study |
| pAB03e*oxyDP | pAB03e* carrying *oxyD* and *oxyP* genes | This study |
| pAB03e*oxyDPchdR | pAB03e* carrying *oxyD, oxyP* and *chdR* genes | This study |
| pOJ436e*chdR | pOJ436 carrying a 1,8 kb fragment from pAB03e*chdR containing *chdR* gene under the control of P_{ermE*} promoter | This study |
| pOJ436e*oxyDP | pOJ436 carrying a 3,2 kb fragment from pAB03e*oxyDP containing *oxyD* and *oxyP* genes under the control of P_{ermE*} promoter | This study |
| pOJ436e*oxyDPchdR | pOJ436 carrying a 4,7 kb fragment from pAB03e*oxyDPchdR containing *oxyD, oxyP* and *chdR* genes under the control of P_{ermE*} promoter | This study |
| pOJ436e*chdRCHD12 | pOJ436e*chdR carrying also CHD biosynthetic cluster | This study |
| pOJ436e*oxyDPCHD12 | pOJ436e*oxyDP carrying also CHD biosynthetic cluster | This study |
| pOJ436e*oxyDPchdRCH D12 | pOJ436e*oxyDPchdR carrying also CHD biosynthetic cluster | This study |
| pNV18ErmchdYser | Fragment containing cyclase gene *chdY* with mutation Gly176Ser cloned into pNV18Erm | This study |
| pAB03chdY | Cyclase gene *chdY* cloned into pAB03 | This study |
| pAB03chdOII-chdY | Gene *chdOII-chdY* with oxygenase and cyclase domain cloned into pAB03 | This study |
| pAB03oxyN | Cyclase gene *oxyN* cloned into pAB03 | This study |

| | | |
|---|---|---|
| ^{[a]}Apr^{r}, apramycin resistant; Erm^{r}, erythromycin resistant; Kan^{r}, kanamycin resistant | | |

**Table 3. Sequences of oligonucleotide primers for PCR experiments used in this study^{[a]}**

| Primers | Sequence |
|---|---|
| CobU1 | 5'-TCCTCACTGCAGGTCGAGTACC-3' |
| CobU2 | 5'-CGGGAAGTCGCGGTATGC-3' |
| glu1 | 5'-CGCGCTGGTCAAAGTCTACG -3' |
| glu2 | 5'-CTGGACGCCTCGCCGTAC-3' |
| chdRF | 5'- TATATACATATGAAGGACAATCTCGCGAGA-3' |
| chdRR | 5'- TATATATCTAGAGGACCTCCGCATCAGGC-3' |
| FSB01C | 5'-AGTCGAATTCGCACCATATGAGACCAAGCGCGTCCGGGTG-3' |
| FSB02C | 5'-CGACTCTAGAGGATCACTAGTTACCAGCCCGACCCGAGCACGC-3' |
| chdYserLF | 5'-TATATAGCATGCGCATCATCGACC-3' |
| chdYserLR | 5'-GCGTCGGT**G**C**T**GATGACCC-3' |
| chdYserRF | 5'-GGTCATCAGC**A**C**C**GACGCG-3' |
| chdYserRR | 5'-TATATAACTAGTCGTCCAGCTGCAGCAGATAAC-3' |
| chdYF | 5'-A TATACATATGCGCATCATCGACCTGTC-3' |
| chdYR | 5'- TATATATCTAGACTAGTCCAGCAGGGCAACGG-3' |
| chdOIIF | 5'-ATATACATATGCCTGAGGACTCCGGC-3' |
| oxyNFw | 5'-TATATACATATGCGCATCATCGATCTGTCGA-3' |
| oxyNRv | 5'-ATATATCTAGACTACTCCTCCACCACCGCC-3' |

| | |
|---|---|
| ^{[a]} Restriction sites are underlined, introduced point-mutations are in bold | |

### LC-MS analysis

To check for production of CHD and CHD analogues, *A. sulphurea* or S. *albus* culture broths were acidified to pH 1-2 with 50% TFA, followed by extraction with 2V of MeOH. The extract was centrifuged and analyzed by LC-MS. All measurements were performed on a Dionex Ultimate 3000 LC system using a Luna C-18 (2) HST, 100 × 2,0 mm, 2,5 µm column (Phenomenex). Separation of 1 µl sample was achieved by a linear gradient from (A) H₂O + 0,1 % FA to (B) ACN + 0,1 % FA at a flow rate of 500 µl/min and 45°C. The gradient was initiated by a 0,5 min isocratic step at 5 % B, followed by an increase to 95 % B in 9 min to end up with a 1,5 min step at 95 % B before reequilibration with initial conditions. UV spectra were recorded by a DAD in the range from 200 to 600 nm. The MS measurement was carried on an amaZon speed mass spectrometer (BrukerDaltonics, Bremen, Germany) using the standard ESI source. Mass spectra were acquired in centroid mode ranging from 200 - 2000 m/z in positive ionization mode.

### Results and discussion

### CHD biosynthetic gene cluster

After sequencing the genomic DNA of CHD producer, *A. sulphurea,* one additional gene in the CHD biosynthetic gene cluster, essential for the biosynthesis of CHD, and two more regulatory genes were discovered lying downstream of already identified CHD biosynthetic genes. The newly discovered biosynthetic gene is *chdY,* encoding a putative second ring cyclase, homologous to OxyN from oxytetracycline biosynthesis (Pickens, Tang 2010). Interestingly, ChdOII and ChdY are encoded as fusion proteins (opposite to separately encoded homologs found in OTC biosynthetic gene cluster, OxyL and OxyN) and similar is observed for *chdL* and *chdOIII* nucleotide sequences which are also operably linked to form a fusion protein of the respective polypeptides encoded by them. The same is true for homologs from OTC biosynthetic gene cluster, *oxyH* and oxyG, respectively. Bioinformatic analysis of the sequence downstream of biosynthetic genes revealed two regulatory genes, encoding SARP and LuxR, which are also found to regulate OTC and CTC biosynthesis (Lesnik et al. 2009; Yin et al. 2015).

### CHD biosynthesis

Biosynthesis of CHD can be directly compared to OTC, as all oxy genes (Pickens, Tang 2010) responsible for the generation of basic TC scaffold have homologs in CHD biosynthetic gene cluster and also one of the intermediates in OTC biosynthesis, 4-keto-ATC strongly resembles putative CHD precursor, 4-keto-9-desmethyl-CHD, differing only in the moiety at C2 position, resulting from incorporation of a different starter unit. However, intermediate in OTC biosynthesis, leading to an impurity, ADOTC, which is primed by acetate (as CHD), should then be the same as in CHD biosynthesis, 4-keto-9-desmethyl-CHD.

Polyketide skeleton of CHD is supposedly synthesized, as previously described (Lukezic et al. 2013), by type II minimal polyketide synthase (minimal PKS) genes, consisting of ketosynthase a, ketosynthase β and acyl carrier protein (ACP), designated as ChdP, ChdK and ChdS, respectively (Figures 1 and 2), condensating 10 malonate-derived building blocks into acetate-primed decaketide. The malonyl-CoA:ACP acyltransferase, needed for the transfer of the extender unit malonyl-CoA to ACP, was proposed to be shared with fatty acid biosynthesis (Revill et al. 1995). As in OTC biosynthesis by OxyJ (Pickens, Tang 2010), initial folding of the growing polyketide chain is most probably directed by a ketoreductase ChdT, reducing the keto group at C9 (Figure 2). Closure of rings leading to the formation of CHD backbone is most likely directed by aromatases/cyclases ChdQI, ChdQII, ChdY and ChdX, first two being similar to OxyK and the last two homologous to OxyN and Oxyl, respectively (Pickens, Tang 2010). Based on homologies to aromatases, encoded in other aromatic polyketide biosynthetic gene clusters, we believe that didomain aromatases ChdQI and ChdQII are responsible for first ring (D) formation (4 in Figure 2), while monodomain cyclase ChdY is needed for second ring (C) closure. As in biosynthesis of other aromatic polyketides, formation of third ring (B) could be spontaneous (5 in Figure 2). Candidate for the last ring (A) cyclization is cyclase ChdX, deducing from comparison with chromomycin and mithramycin biosynthesis (Menendez et al. 2004), while the function of its homologue in OTC biosynthesis, Oxyl, on the other hand, has not been elucidated yet (Pickens, Tang 2010). Such generated tetracyclic scaffold is then further processed towards CHD through different post-PKS tailoring reactions, the last two also leading CHD biosynthesis away from that of typical tetracyclines. ChdMI, OxyF homologue (Zhang et al. 2007), could methylate C6 position in CHD biosynthetic intermediate, while oxygenase pair ChdOII and ChdOI, homologs of OxyL and OxyE (Wang et al. 2009), respectively, could be responsible for a double hydroxylation of ring A at C4/C12a. Hydroxylation at C4 is a followed by transamination by ChdN, a PLP-dependent aminotransferase only distantly related to OxyQ, which is responsible for incorporation of an amino group at C4 in OTC biosynthesis (Pickens, Tang 2010). The activity of such different aminotransferases represents a diverging point between CHD and typical TCs biosynthesis and results in different products: amino group incorporated into CHD is in R-configuration, while the one in OTC biosynthesis stands in S-confguration. In contrast to more decorated backbone of typical TCs, there is only one more tailoring reaction leading to CHD, C9-methylation, which is believed to be catalysed by ChdMII, homolog of C9-methyltransferases from chromomycin and mithramycin biosynthesis (Menendez et al. 2004).

### Regulation of CHD biosynthesis and self-resistance

One of the putative regulatory proteins found in CHD biosynthetic cluster belongs to the *Streptomyces* antibiotic regulatory protein (SARP) transcription activators. It is homologous to OtcR, identified by Yin et al. (Yin et al. 2015), which acts as a positive pathway-specific activator of OTC biosynthesis leading to a significant increase in OTC production when overexpressed at the appropriate level. The second putative regulatory protein, found in CHD biosynthetic gene cluster, belongs to the LuxR family and is homologous to regulatory protein OtcG from OTC biosynthesis, identified by Lesnik et al. (Lesnik et al. 2009). OtcG has a conditionally positive role in OTC biosynthesis: its inactivation reduced the production of OTC by more than 40 %, while its overexpression under the strong constitutive promoter P_{ermE*} did not yield any statistically significant change in the production of OTC (Lesnik et al. 2009). *chdR* encodes a putative integral membrane protein that is most probably responsible for the efflux of CHD from the cell and is probably regulated by another regulatory protein, the putative TetR family repressor protein ChdA (Lukezic et al. 2013).

### Heterologous expression of CHD biosynthetic gene cluster

Replicative cosmid pOJ456-CHD12, carrying CHD biosynthetic gene cluster, was fished out from *A. sulphurea* cosmid library by selection on CHD containing agar plates. After confirming its correct sequence, the cosmid was introduced into S. *albus* by conjugation in attempt to heterologously express CHD biosynthetic gene cluster. The CHD biosynthetic cluster was transferred into an integrative cosmid (pOJ436) to allow a stable integration of CHD biosynthetic cluster into the genome of heterologous host. Indeed, heterologous expression of CHD biosynthetic cluster from integrated cosmid pOJ436-CHD12 was successful and resulted in production of CHD, reaching up to approx. 50 mg/L.

Furthermore, we constructed another integrative cosmid carrying CHD biosynthetic cluster with additional copy of CHD efflux pump gene *chdR* under strong promoter P_{erm*} (construct pOJ436-PermE*-chdR-CHD12) to overcome possible self-resistance issues during heterologous expression of CHD. Additional copy of efflux pump gene chdR led to slightly increased production yields of CHD up to approx. 60 mg/L (Figure 3).

Additionally, with the aim to produce CDCHD (Lesnik et al. 2015), we constructed integrative cosmid carrying the CHD biosynthetic cluster and *oxyDPchdR* genes under strong promoter Pₑᵣₘ* (construct pOJ436-PermE*-oxyDPchdR-CHD12), whose expression resulted in production of CDCHD (less than 5 mg/L; Figure 4).

### Inactivation and complementation experiments

We mutated putative second ring (C) cyclase ChdY residue G176, which was chosen based on comparison with DpsY, a cyclase from daunomycin biosynthesis (Hautala et al. 2003). Mutation of conserved Gly to Ser (G191S), even though most probably not being a part of active site (Díaz-Sáez et al. 2014), resulted in inactivation of DpsY (Hautala et al. 2003). Also in our ChdY inactivation experiment production of CHD was not observed anymore or only in traces (Figure 5).

Mutation of *chdY* led to such increase in production of a shunt product (»compound 369«) (Figure 5), which allowed its isolation and structure elucidation by HRMS and NMR analysis. The HRMS mass for "compound 369" was found to be 369.09 [*M*⁺+H], which corresponds to the expected mass of 369.0969 for C₂₀H₁₇O₇⁺. This shunt product is most probably the result of spontaneous cyclization following first ring (D) closure and aromatization mediated by intact ketoreductase ChdT and aromatase pair ChdQI/ChQII.

The mutant was then complemented with non-mutated wild-type genes *chdY* or *chdOII-chdY* (encoding a fusion protein as found in CHD cluster), which partly restored the production of CHD. Such complemented mutants were necessary as they represent, contrary to wild-type CHD producer strain, directly comparable controls for later complementation experiment with homologous enzyme from OTC biosynthesis. In all complementation experiments the genes were introduced by integration of pAB03 plasmid, carrying the selected genes, into a genome location distant from the wild-type location in CHD biosynthetic gene cluster, allowing the mutant to produce both, the mutated and wild-type protein. For complementation experiment we chose a homologous enzyme from OTC biosynthesis, whose function was demonstrated by heterologus expression in *Streptomyces* host and isolation of shunt products.

6% restored production of CHD after complementation of *chdY* mutant with OxyN (second ring cyclase in OTC biosynthesis) compared to 3% restored CHD production with wild-type ChdY or similarly 5% restored CHD production with whole fusion protein ChdOII-ChdY (Figure 6), led us to the conclusion that ChdY is responsible for second (C) ring cyclization. CHD production in negative control with integrated empty plasmid was less than 0,3% of production level in wild-type strain.

In the inactivation mutant traces of CHD production was still observed, which could possibly be due to some remaining catalytic activity of mutated cyclase or spontaneous cyclization leading to synthesis of small amounts of CHD. The reason for low production of CHD after complementation could be because complemented mutant generated through site-directed mutagenesis is still expressing both, the mutated and introduced wild-type protein, which are both taking part in PKS complex structure. Incorporation of structurally similar but functionally impaired mutated protein might thus prevent the PKS complex to reach its full biosynthetic potential.

### List of certain references cited in the description

Abelson, John N.; Simon, Melvin I. (1998): Methods in enzymology. Cumulative subject index. Vols. 263, 264, 266-289 / editors-in-chief, John N. Abelson and Melvin I. Simon. London: Academic Press. Available online at http://www.elsevier.com/journals BLDSS.

Ames, B. D.; Korman, T. P.; Zhang, W.; Smith, P.; Vu, T.; Tang, Y.; Tsai, S. C. (2008): Crystal structure and functional analysis of tetracenomycin ARO/CYC. implications for cyclization specificity of aromatic polyketides. In Proc Natl Acad Sci U S A 105 (14), pp. 5349-5354. DOI: 10.1073/pnas.07092231050709223105.

Ausubel, Frederick M. (1987-): Current protocols in molecular biology. Brooklyn, N. Y.: Greene Publishing Associates; Media. Available online at http://onlinelibrary.wiley.com/ BLDSS.

Bierman, M.; Logan, R.; O'Brien, K.; Seno, E. T.; Rao, R. N.; Schoner, B. E. (1992): Plasmid cloning vectors for the conjugal transfer of DNA from Escherichia coli to Streptomyces spp. In Gene 116 (1), pp. 43-49.

Bilyk, Bohdan; Horbal, Liliya; Luzhetskyy, Andriy (2017): Chromosomal position effect influences the heterologous expression of genes and biosynthetic gene clusters in Streptomyces albus J1074. In Microb Cell Fact 16 (1), p. 5. DOI: 10.1186/s12934-016-0619-z.

Bilyk, Oksana; Sekurova, Olga N.; Zotchev, Sergey B.; Luzhetskyy, Andriy (2016): Cloning and Heterologous Expression of the Grecocycline Biosynthetic Gene Cluster. In PloS one 11 (7), e0158682. DOI: 10.1371/journal.pone.0158682.

Chiba, K.; Hoshino, Y.; Ishino, K.; Kogure, T.; Mikami, Y.; Uehara, Y.; Ishikawa, J. (2007): Construction of a pair of practical Nocardia-Escherichia coli shuttle vectors. In Jpn. J. Infect. Dis. 60 (1), pp. 45-47.

Díaz-Sáez, Laura; Srikannathasan, Velupillai; Zoltner, Martin; Hunter, William N. (2014): Structures of bacterial kynurenine formamidase reveal a crowded binuclear zinc catalytic site primed to generate a potent nucleophile. In Biochem J 462 (3), pp. 581-589. DOI: 10.1042/BJ20140511.

Fernandez-Moreno, M. A.; Martinez, E.; Boto, L.; Hopwood, D. A.; Malpartida, F. (1992): Nucleotide sequence and deduced functions of a set of cotranscribed genes of Streptomyces coelicolor A3(2) including the polyketide synthase for the antibiotic actinorhodin. In J. Biol. Chem. 267 (27), pp. 19278-19290.

Harnes, B. D.; Higgins, S. J. (1984): Transcription and translation. A practical approach / edited by B.D. Hames, S.J. Higgins. Oxford: IRL (Practical approach series).

Hautala, Anne; Torkkell, Sirke; Räty, Kaj; Kunnari, Tero; Kantola, Jaana; Mantsälä, Pekka et al. (2003): Studies on a second and third ring cyclization in anthracycline biosynthesis. In J. Antibiot. 56 (2), pp. 143-153.

Hopwood, D. A.; Sherman, D. H. (1990): Molecular genetics of polyketides and its comparison to fatty acid biosynthesis. In Annu Rev Genet 24, pp. 37-66. DOI: 10.1146/annurev.ge.24.120190.000345.

Kieser, T.; Bibb, M. J.; Buttner, M. J.; Chater, K. F.; Hopwood, D. A. (2000): Practical Streptomyces Genetics. Norwich: John Innes Foundation.

Koshla, Oksana; Lopatniuk, Maria; Rokytskyy, Ihor; Yushchuk, Oleksandr; Dacyuk, Yuriy; Fedorenko, Victor et al. (2017): Properties of Streptomyces albus J1074 mutant deficient in tRNALeuUAA gene bldA. In Arch Microbiol 199 (8), pp. 1175-1183. DOI: 10.1007/s00203-017-1389-7.

Lesnik, U.; Gormand, A.; Magdevska, V.; Fujs, S.; Raspor, P.; Hunter, I.; Petkovic, H. (2009): Regulatory elements in tetracycline-encoding gene clusters. the otcG gene positively regulates the production of oxytetracycline in Streptomyces rimosus. In Food Technology & Biotechnology 47 (3), pp. 323-330.

Lesnik, Urska; Lukezic, Tadeja; Podgorsek, Ajda; Horvat, Jaka; Polak, Tomaz; Sala, Martin et al. (2015): Construction of a new class of tetracycline lead structures with potent antibacterial activity through biosynthetic engineering. In Angew. Chem., Int. Ed. 54 (13), pp. 3937-3940. DOI: 10.1002/anie.201411028.

Lukezic, T.; Lesnik, U.; Podgorsek, A.; Horvat, J.; Polak, T.; Sala, M. et al. (2013): Identification of the chelocardin biosynthetic gene cluster from Amycolatopsis sulphurea. a platform for producing novel tetracycline antibiotics. In Microbiology 159 (Pt 12), pp. 2524-2532. DOI: 10.1099/mic.0.070995-0mic.0.070995-0.

MacNeil, D. J.; Gewain, K. M.; Ruby, C. L.; Dezeny, G.; Gibbons, P. H.; MacNeil, T. (1992): Analysis of Streptomyces avermitilis genes required for avermectin biosynthesis utilizing a novel integration vector. In Gene 111 (1), pp. 61-68.

Madon, J.; Hutter, R. (1991): Transformation system for Amycolatopsis (Nocardia) mediterranei. direct transformation of mycelium with plasmid DNA. In J. Bacteriol. 173 (20), pp. 6325-6331.

Martin, J. L.; McMillan, F. M. (2002): SAM (dependent) I AM. the S-adenosylmethionine-dependent methyltransferase fold. In Curr Opin Struct Biol 12 (6), pp. 783-793.

Mason, J. R.; Cammack, R. (1992): The electron-transport proteins of hydroxylating bacterial dioxygenases. In Annu Rev Microbiol 46, pp. 277-305. DOI: 10.1146/annurev.mi.46.100192.001425.

Menendez, N.; Nur-e-Alam, M.; Brana, A. F.; Rohr, J.; Salas, J. A.; Mendez, C. (2004): Biosynthesis of the antitumor chromomycin A3 in Streptomyces griseus. analysis of the gene cluster and rational design of novel chromomycin analogs. In Chem Biol 11 (1), pp. 21-32. DOI: 10.1016/j.chembiol.2003.12.011S1074552103002837.

Mitscher, L. A.; Swayze, J. K.; Hogberg, T.; Khanna, I.; Rao, G. S.; Theriault, R. J. et al. (1983): Biosynthesis of cetocycline. In J. Antibiot. 36 (10), pp. 1405-1407.

Molnar, V.; Matkovic, Z.; Tambic, T.; Kozma, C. (1977): Klinicko-farmakolosko ispitivanje kelokardina u bolesnika s infekcijom mokracnih puta. In Lij. vjes. 99, pp. 560-562.

Myronovskyi, Maksym; Rosenkränzer, Birgit; Nadmid, Suvd; Pujic, Petar; Normand, Philippe; Luzhetskyy, Andriy (2018): Generation of a cluster-free Streptomyces albus chassis strains for improved heterologous expression of secondary metabolite clusters. In Metab Eng. DOI: 10.1016/j.ymben.2018.09.004.

Oliver, T. J.; Prokop, J. F.; Bower, R. R.; Otto, R. H. (1962): Chelocardin, a new broad-spectrum antibiotic. I. Discovery and biological properties. In Antimicrob. Agents Chemother. 1962, pp. 583-591.

Oliver, T. J.; Sinclair, A. C.: Antibiotic M-319. Patent no. 3155582. 3155582.

Paget, M. S.; Chamberlin, L.; Atrih, A.; Foster, S. J.; Buttner, M. J. (1999): Evidence that the extracytoplasmic function sigma factor sigmaE is required for normal cell wall structure in Streptomyces coelicolor A3(2). In J. Bacteriol. 181 (1), pp. 204-211.

Petkovic, H.; Raspor, P.; Lesnik, U.: Genes for biosynthesis of tetracycline compounds and uses thereof. EP2154249.

Pickens, L. B.; Tang, Y. (2010): Oxytetracycline biosynthesis. In J. Biol. Chem. 285 (36), pp. 27509-27515. DOI: 10.1074/jbc.R110.130419R110.130419.

Proctor, R.; Craig, W.; Kunin, C. (1978): Cetocycline, tetracycline analog. in vitro studies of antimicrobial activity, serum binding, lipid solubility, and uptake by bacteria. In Antimicrob. Agents Chemother. 13 (4), pp. 598-604.

Rasmussen, B.; Noller, H. F.; Daubresse, G.; Oliva, B.; Misulovin, Z.; Rothstein, D. M. et al. (1991): Molecular basis of tetracycline action. identification of analogs whose primary target is not the bacterial ribosome. In Antimicrob. Agents Chemother. 35 (11), pp. 2306-2311.

Rawlings, M.; Cronan, J. E., Jr. (1992): The gene encoding Escherichia coli acyl carrier protein lies within a cluster of fatty acid biosynthetic genes. In J. Biol. Chem. 267 (9), pp. 5751-5754.

Revill, W. P.; Bibb, M. J.; Hopwood, D. A. (1995): Purification of a malonyltransferase from Streptomyces coelicolor A3(2) and analysis of its genetic determinant. In J. Bacteriol. 177 (14), pp. 3946-3952.

Rhodes, P. M.; Hunter, I. S.; Friend, E. J.; Warren, M. (1984): Recombinant DNA methods for the oxytetracycline producer Streptomyces rimosus. In Biochem. Soc. Trans. 12 (4), pp. 586-587.

Sambrook, Joseph; Russell, David W. (2001): Molecular Cloning. a Laboratory Manual. 3rd. Cold Spring Harbor, N.Y.: Cold Spring Harbor Laboratory Press.

Stepanek, Jennifer J.; Lukezic, Tadeja; Teichert, Ines; Petković, Hrvoje; Bandow, Julia E. (2016): Dual mechanism of action of the atypical tetracycline chelocardin. In Biochim. Biophys. Acta 1864 (6), pp. 645-654. DOI: 10.1016/j.bbapap.2016.03.004.

Walsh, C. T.; Gehring, A. M.; Weinreb, P. H.; Quadri, L. E.; Flugel, R. S. (1997): Post-translational modification of polyketide and nonribosomal peptide synthases. In Curr Opin Chem Biol 1 (3), pp. 309-315.

Wang, P.; Zhang, W.; Zhan, J.; Tang, Y. (2009): Identification of OxyE as an ancillary oxygenase during tetracycline biosynthesis. In Chembiochem 10 (9), pp. 1544-1550. DOI: 10.1002/cbic.200900122.

Yin, Shouliang; Wang, Weishan; Wang, Xuefeng; Zhu, Yaxin; Jia, Xiaole; Li, Shanshan et al. (2015): Identification of a cluster-situated activator of oxytetracycline biosynthesis and manipulation of its expression for improved oxytetracycline production in Streptomyces rimosus. In Microb Cell Fact 14, p. 46. DOI: 10.1186/s12934-015-0231-7.

Zhang, W.; Watanabe, K.; Wang, C. C.; Tang, Y. (2007): Investigation of early tailoring reactions in the oxytetracycline biosynthetic pathway. In J. Biol. Chem. 282 (35), pp. 25717-25725. DOI: 10.1074/jbc.M703437200.

Zhang, Wenjun; Ames, Brian D.; Tsai, Shiou-Chuan; Tang, Yi (2006): Engineered biosynthesis of a novel amidated polyketide, using the malonamyl-specific initiation module from the oxytetracycline polyketide synthase. In Applied and environmental microbiology 72 (4), pp. 2573-2580. DOI: 10.1128/AEM.72.4.2573-2580.2006.

## Claims

1. A gene cluster encoding polypeptides involved in the biosynthesis of a tetracycline, wherein said gene cluster includes all of the nucleotide sequences (1) to (19):
(1) a nucleotide sequence encoding a polypeptide which comprises an amino acid sequence having at least 80%, such as at least 85%, sequence identity with the polypeptide of SEQ ID NO: 1 and which has ketosynthase-alpha activity;
(2) a nucleotide sequence encoding a polypeptide which comprises an amino acid sequence having at least 80%, such as at least 85%, sequence identity with the polypeptide of SEQ ID NO: 2 and which has ketosynthase-beta activity;
(3) a nucleotide sequence encoding a polypeptide which comprises an amino acid sequence having at least 80%, such as at least 85%, sequence identity with the polypeptide of SEQ ID NO: 3 and which is capable of acting as acyl carrier protein (ACP);
(4) a nucleotide sequence encoding a polypeptide which comprises an amino acid sequence having at least 80%, such as at least 85%, sequence identity with the polypeptide of SEQ ID NO: 4 and which has cyclase/aromatase activity;
(5) a nucleotide sequence encoding a polypeptide which comprises an amino acid sequence having at least 80%, such as at least 85%, sequence identity with the polypeptide of SEQ ID NO: 5 and which has cyclase/ aromatase activity;
(6) a nucleotide sequence encoding a polypeptide which comprises an amino acid sequence having at least 80%, such as at least 85%, sequence identity with the polypeptide of SEQ ID NO: 6 and which has cyclase activity;
(7) a nucleotide sequence encoding a polypeptide which comprises an amino acid sequence having at least 80%, such as at least 85%, sequence identity with the polypeptide of SEQ ID NO: 7 and which has acyl-CoA ligase activity;
(8) a nucleotide sequence encoding a polypeptide which comprises an amino acid sequence having at least 80%, such as at least 85%, sequence identity with the polypeptide of SEQ ID NO: 8 and which has ketoreductase activity;
(9) a nucleotide sequence encoding a polypeptide which comprises an amino acid sequence having at least 80%, such as at least 85%, sequence identity with the polypeptide of SEQ ID NO: 9 and which has S-adenosylmethionine (SAM)-dependent C-6 methyltransferase activity;
(10) a nucleotide sequence encoding a polypeptide which comprises an amino acid sequence having at least 80%, such as at least 85%, sequence identity with the polypeptide of SEQ ID NO: 10 and which has S-adenosylmethionine (SAM)-dependent methyltransferase activity;
(11) a nucleotide sequence encoding a polypeptide which comprises an amino acid sequence having at least 80%, such as at least 85%, sequence identity with the polypeptide of SEQ ID NO: 11 and which has pyridoxal 5'-phosphate-dependent aminotransferase activity
(12) a nucleotide sequence encoding a polypeptide which comprises an amino acid sequence having at least 80%, such as at least 85%, sequence identity with the polypeptide of SEQ ID NO: 12 and which has glycosyltransferase activity;
(13) a nucleotide sequence encoding a polypeptide which comprises an amino acid sequence having at least 80%, such as at least 85%, sequence identity with the polypeptide of SEQ ID NO: 13 and which has transposase activity;
(14) a nucleotide sequence encoding a polypeptide which comprises an amino acid sequence having at least 80%, such as at least 85%, sequence identity with the polypeptide of SEQ ID NO: 14 and which is capable of acting as an exporter;
(15) a nucleotide sequence encoding a polypeptide which comprises an amino acid sequence having at least 80%, such as at least 85%, sequence identity with the polypeptide of SEQ ID NO: 15 and which has transcriptional regulator activity;
(16) a nucleotide sequence encoding a polypeptide which comprises an amino acid sequence having at least 80%, such as at least 85%, sequence identity with the polypeptide of SEQ ID NO: 16 and which has FAD-dependent oxygenase activity;
(17) a nucleotide sequence encoding a polypeptide which comprises an amino acid sequence having at least 80%, such as at least 85%, sequence identity with the polypeptide of SEQ ID NO: 17 and which has monooxygenase activity;
(18) a nucleotide sequence encoding a polypeptide which comprises an amino acid sequence having at least 80%, such as at least 85%, sequence identity with the polypeptide of SEQ ID NO: 18 and which has FAD-dependent oxygenase activity; and
(19) a nucleotide sequence encoding a polypeptide which comprises an amino acid sequence having at least 80%, such as at least 85%, sequence identity with the polypeptide of SEQ ID NO: 19 and which has cyclase activity.

2. The gene cluster according to claim 1, wherein said gene cluster further comprises at least one of the nucleotide sequences (20) and (21):
(20) a nucleotide sequence encoding a polypeptide which comprises an amino acid sequence having at least 80%, such as at least 85%, sequence identity with the polypeptide of SEQ ID NO: 20 and which has transcriptional activator activity; and
(21) a nucleotide sequence encoding a polypeptide which comprises an amino acid sequence having at least 80%, such as at least 85%, sequence identity with the polypeptide of SEQ ID NO: 21 and which has transcriptional activator activity .

3. The gene cluster according to claim 2, wherein said gene cluster comprises both nucleotide sequences (20) and (21).

4. The gene cluster according to any one of claims 1 to 3, wherein the nucleotide sequences (18) and (19) are linked to form a fusion protein of the respective polypeptides encoded by them.

5. The gene cluster according to claim 4, wherein the fusion protein comprises an amino acid sequence having at least 80%, such as at least 85%, sequence identity with the polypeptide of SEQ ID NO: 22 and has FAD-dependent oxygenase and cyclase activities.

6. The gene cluster according to any one of claims 1 to 5, wherein the nucleotide sequences (7) and (17) are linked to form a fusion protein of the respective polypeptides encoded by them.

7. The gene cluster according to claim 6, wherein the fusion protein comprises an amino acid sequence having at least 80%, such as at least 85%, sequence identity with the polypeptide of SEQ ID NO: 23 and has acyl-CoA ligase and oxygenase activities.

8. A DNA construct comprising the gene cluster according to any one of claims 1 to 7.

9. The DNA construct according to claim 8, wherein said DNA construct further comprises a nucleotide sequence encoding a polypeptide which comprises an amino acid sequence having at least 80%, such as at least 85%, sequence identity with the polypeptide of SEQ ID NO: 24 and which has amidotransferase activity.

10. A recombinant host cell comprising the gene cluster according to any one of claims 1 to 7 or the DNA construct according to claim 8 or 9, wherein the gene cluster or DNA construct is heterologous to said host cell.

11. The recombinant host cell according to claim 10, which heterologously expresses the polypeptides encoded by the gene cluster.

12. The recombinant host cell according to claim 10 or 11, which heterologously expresses a polypeptide which comprises an amino acid sequence having at least 80%, such as at least 85%, sequence identity with the polypeptide of SEQ ID NO: 24 and which has amidotransferase activity.

13. The recombinant host cell according to any one of claims 10 to 12, which is a bacterium.

14. A process for the biosynthetic production of a tetracycline, said process comprises the steps of a) cultivating a recombinant host cell according to any one of claims 10 to 13 in the presence of a suitable substrate under conditions conducive to the production of said tetracycline and, optionally, b) recovering the tetracycline from the medium employed in cultivation.

15. The process according to claim 14, wherein the tetracycline is chelocardin or an analogue thereof, wherein the chelocardin is a compound having structure I 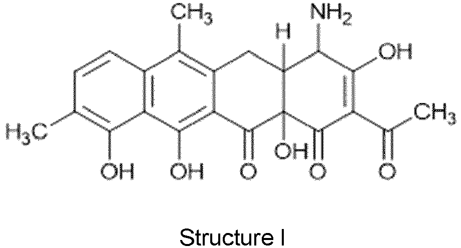 optionally as a stereoisomer, including enantiomers and diastereomers, or in form of a mixture of at least two stereoisomers, including enantiomers and/or diastereomers, in any mixing ratio, or a corresponding salt thereof, or a corresponding solvate thereof;
and wherein the chelocardin analogue is a compound having structure III 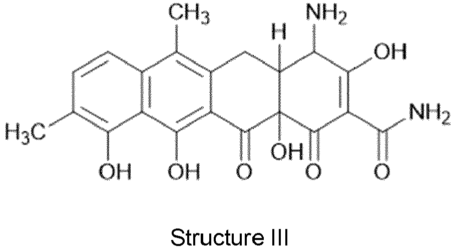 optionally as a stereoisomer, including enantiomers and diastereomers, or in form of a mixture of at least two stereoisomers, including enantiomers and/or diastereomers, in any mixing ratio, or a corresponding salt thereof, or a corresponding solvate thereof.

## Patentansprüche

1. Gencluster, das Polypeptide kodiert, die an der Biosynthese eines Tetracyclins beteiligt sind, wobei das Gencluster alle die Nukleotidsequenzen (1) bis (19) einschließt:
(1) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 1 umfasst und welches Ketosynthase-Alpha-Aktivität aufweist;
(2) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 2 umfasst und welches Ketosynthase-Beta-Aktivität aufweist;
(3) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 3 umfasst und welches dazu im Stande ist, als Acylträgerprotein (ACP) zu wirken;
(4) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 4 umfasst und welches Cyclase/Aromatase-Aktivität aufweist;
(5) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 5 umfasst und welches Cyclase/Aromatase-Aktivität aufweist;
(6) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 6 umfasst und welches Cyclase-Aktivität aufweist;
(7) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 7 umfasst und welches Acyl-CoA-Ligase-Aktivität aufweist;
(8) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 8 umfasst und welches Ketoreduktase-Aktivität aufweist;
(9) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 9 umfasst und welches S-Adenosylmethionin-(SAM)-abhängige C-6-Methyltransferase-Aktivität aufweist;
(10) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 10 umfasst und welches S-Adenosylmethionin-(SAM)-abhängige Methyltransferase-Aktivität aufweist;
(11) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 11 umfasst und welches pyridoxale 5'-Phosphat-abhängige Aminotransferase-Aktivität aufweist;
(12) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 12 umfasst und welches Glycosyltransferase-Aktivität aufweist;
(13) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 13 umfasst und welches Transposase-Aktivität aufweist;
(14) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 14 umfasst und welches dazu im Stande ist, als Exporter zu wirken;
(15) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 15 umfasst und welches Transkriptionsregulatoraktivität aufweist;
(16) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 16 umfasst und welches FAD-abhängige Oxygenase-Aktivität aufweist;
(17) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 17 umfasst und welches Monooxygenase-Aktivität aufweist;
(18) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 18 umfasst und welches FAD-abhängige Oxygenase-Aktivität aufweist; und
(19) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 19 umfasst und welches Cyclase-Aktivität aufweist.

2. Gencluster nach Anspruch 1, wobei das Gencluster weiter zumindest eine der Nukleotidsequenzen (20) und (21) umfasst:
(20) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 20 umfasst und welches Transkriptionsaktivatoraktivität aufweist; und
(21) eine Nukleotidsequenz, die ein Polypeptid kodiert, welches eine Aminosäuresequenz mit zumindest 80 %, wie beispielsweise zumindest 85 %, Sequenzidentität mit dem Polypeptid mit SEQ ID NO: 21 umfasst und welches Transkriptionsaktivatoraktivität aufweist.

3. Gencluster nach Anspruch 2, wobei das Gencluster weiter beide Nukleotidsequenzen (20) und (21) umfasst.

4. Gencluster nach einem der Ansprüche 1 bis 3, wobei die Nukleotidsequenzen (18) und (19) verbunden sind, um ein Fusionsprotein der jeweiligen durch diese kodierten Polypeptide zu bilden.

5. Gencluster nach Anspruch 4, wobei das Fusionsprotein eine Aminosäuresequenz mit zumindest 80 %, wie beispielsweise zumindest 85 %, Sequenzidentität mit dem Polypeptid mit SEQ ID NO: 22 umfasst und FAD-abhängige Oxygenase- und Cyclaseaktivitäten aufweist.

6. Gencluster nach einem der Ansprüche 1 bis 5, wobei die Nukleotidsequenzen (7) und (17) verbunden sind, um ein Fusionsprotein der jeweiligen durch diese kodierten Polypeptide zu bilden.

7. Gencluster nach Anspruch 6, wobei das Fusionsprotein eine Aminosäuresequenz mit zumindest 80 %, wie beispielsweise zumindest 85 %, Sequenzidentität mit dem Polypeptid mit SEQ ID NO: 23 umfasst und Acyl-CoA-Ligase- und Oxygenaseaktivitäten aufweist.

8. DNA-Konstrukt umfassend das Gencluster nach einem der Ansprüche 1 bis 7.

9. DNA-Konstrukt nach Anspruch 8, wobei das DNA-Konstrukt weiter eine Nukleotidsequenz umfasst, die ein Polypeptid kodiert, welches eine Aminosäuresequenz mit zumindest 80 %, wie beispielsweise zumindest 85 %, Sequenzidentität mit dem Polypeptid mit SEQ ID NO: 24 umfasst und welches Amidotransferase-Aktivität aufweist.

10. Rekombinante Wirtszelle umfassend das Gencluster nach einem der Ansprüche 1 bis 7 oder das DNA-Konstrukt nach Anspruch 8 oder 9, wobei das Gencluster oder das DNA-Konstrukt gegenüber der Wirtszelle heterolog ist.

11. Rekombinante Wirtszelle nach Anspruch 10, welche die durch das Gencluster kodierten Polypeptide heterolog exprimiert.

12. Rekombinante Wirtszelle nach Anspruch 10 oder 11, welche ein Polypeptid heterolog exprimiert, welches eine Aminosäuresequenz mit zumindest 80 %, wie beispielsweise zumindest 85 %, Sequenzidentität mit dem Polypeptid mit SEQ ID NO: 24 umfasst und welches Amidotransferase-Aktivität aufweist.

13. Rekombinante Wirtszelle nach einem der Ansprüche 10 bis 12, welche ein Bakterium ist.

14. Verfahren für die biosynthetische Herstellung eines Tetracyclins, wobei das Verfahren die folgenden Schritte umfasst a) Kultivieren einer rekombinanten Wirtszelle nach einem der Ansprüche 10 bis 13 in Gegenwart eines geeigneten Substrats unter Bedingungen, die für die Herstellung des Tetracyclins förderlich sind, und, wahlweise, b) Gewinnen des Tetracyclins aus dem beim Kultivieren verwendeten Mediums.

15. Verfahren nach Anspruch 14, wobei das Tetracyclin Chelocardin oder ein Analogon davon ist, wobei das Chelocardin eine Verbindung mit der Struktur I ist 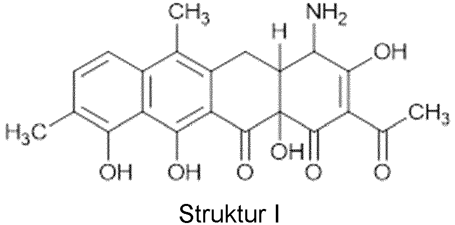 eventuell als Stereoisomer, umfassend Enantiomere und Diastereomere, oder in Form einer Mischung von mindestens zwei Stereoisomeren, umfassend Enantiomere und/oder Diastereomere, in irgendeinem Mischverhältnis, oder ein entsprechendes Salz davon oder ein entsprechendes Solvat davon;
und wobei das Chelocardin-Analogon eine Verbindung mit der Struktur III ist 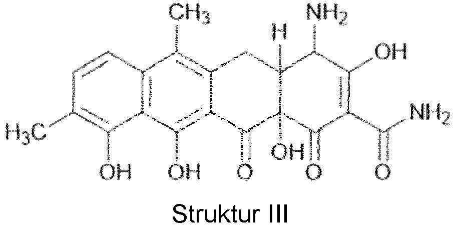 eventuell als Stereoisomer, umfassend Enantiomere und Diastereomere, oder in Form einer Mischung von mindestens zwei Stereoisomeren, umfassend Enantiomere und/oder Diastereomere, in irgendeinem Mischverhältnis, oder ein entsprechendes Salz davon oder ein entsprechendes Solvat davon.

## Revendications

1. Groupe de gènes codant pour des polypeptides impliqués dans la biosynthèse d'une tétracycline, ledit groupe de gènes comprenant toutes les séquences nucléotidiques (1) à (19) :
(1) une séquence nucléotidique codant pour un polypeptide qui comprend une séquence d'acides aminés ayant au moins 80%, tel qu'au moins 85%, d'identité de séquence avec le polypeptide de SEQ ID NO : 1 et qui a une activité cétosynthase-alpha ;
(2) une séquence nucléotidique codant pour un polypeptide qui comprend une séquence d'acides aminés ayant au moins 80%, tel qu'au moins 85%, d'identité de séquence avec le polypeptide de SEQ ID NO : 2 et qui a une activité cétosynthase-beta ;
(3) une séquence nucléotidique codant pour un polypeptide qui comprend une séquence d'acides aminés ayant au moins 80%, tel qu'au moins 85%, d'identité de séquence avec le polypeptide de SEQ ID NO : 3 et qui est capable d'agir en tant que protéine porteuse d'acyle (ACP) ;
(4) une séquence nucléotidique codant pour un polypeptide qui comprend une séquence d'acides aminés ayant au moins 80%, tel qu'au moins 85%, d'identité de séquence avec le polypeptide de SEQ ID NO : 4 et qui a une activité cyclase / aromatase ;
(5) une séquence nucléotidique codant pour un polypeptide qui comprend une séquence d'acides aminés ayant au moins 80%, tel qu'au moins 85%, d'identité de séquence avec le polypeptide de SEQ ID NO : 5 et qui a une activité cyclase / aromatase ;
(6) une séquence nucléotidique codant pour un polypeptide qui comprend une séquence d'acides aminés ayant au moins 80%, tel qu'au moins 85%, d'identité de séquence avec le polypeptide de SEQ ID NO : 6 et qui a une activité cyclase ;
(7) une séquence nucléotidique codant pour un polypeptide qui comprend une séquence d'acides aminés ayant au moins 80%, tel qu'au moins 85%, d'identité de séquence avec le polypeptide de SEQ ID NO : 7 et qui a une activité acyl-CoA ligase ;
(8) une séquence nucléotidique codant pour un polypeptide qui comprend une séquence d'acides aminés ayant au moins 80%, tel qu'au moins 85%, d'identité de séquence avec le polypeptide de SEQ ID NO : 8 et qui a une activité cétoréductase ;
(9) une séquence nucléotidique codant pour un polypeptide qui comprend une séquence d'acides aminés ayant au moins 80%, tel qu'au moins 85%, d'identité de séquence avec le polypeptide de SEQ ID NO : 9 et qui a une activité de méthyltransférase C-6 dépendante de S-adénosylméthionine (SAM) ;
(10) une séquence nucléotidique codant pour un polypeptide qui comprend une séquence d'acides aminés ayant au moins 80%, tel qu'au moins 85%, d'identité de séquence avec le polypeptide de SEQ ID NO : 10 et qui a une activité de méthyltransférase dépendante de S-adénosylméthionine (SAM) ;
(11) une séquence nucléotidique codant pour un polypeptide qui comprend une séquence d'acides aminés ayant au moins 80%, tel qu'au moins 85%, d'identité de séquence avec le polypeptide de SEQ ID NO : 11 et qui a une activité aminotransférase dépendante du phosphate de pyridoxal 5'
(12) une séquence nucléotidique codant pour un polypeptide qui comprend une séquence d'acides aminés ayant au moins 80%, tel qu'au moins 85%, d'identité de séquence avec le polypeptide de SEQ ID NO : 12 et qui a une activité glycosyltransférase ;
(13) une séquence nucléotidique codant pour un polypeptide qui comprend une séquence d'acides aminés ayant au moins 80%, tel qu'au moins 85%, d'identité de séquence avec le polypeptide de SEQ ID NO : 13 et qui a une activité de transposase ;
(14) une séquence nucléotidique codant pour un polypeptide qui comprend une séquence d'acides aminés ayant au moins 80%, tel qu'au moins 85%, d'identité de séquence avec le polypeptide de SEQ ID NO : 14 et qui est capable d'agir en tant qu'exportateur ;
(15) une séquence nucléotidique codant pour un polypeptide qui comprend une séquence d'acides aminés ayant au moins 80%, tel qu'au moins 85%, d'identité de séquence avec le polypeptide de SEQ ID NO : 15 et qui a une activité de régulateur transcriptionnel ;
(16) une séquence nucléotidique codant pour un polypeptide qui comprend une séquence d'acides aminés ayant au moins 80%, tel qu'au moins 85%, d'identité de séquence avec le polypeptide de SEQ ID NO : 16 et qui a une activité oxygénase dépendante de la FAD ;
(17) une séquence nucléotidique codant pour un polypeptide qui comprend une séquence d'acides aminés ayant au moins 80%, tel qu'au moins 85%, d'identité de séquence avec le polypeptide de SEQ ID NO : 17 et qui a une activité de monooxygénase ;
(18) une séquence nucléotidique codant pour un polypeptide qui comprend une séquence d'acides aminés ayant au moins 80%, tel qu'au moins 85%, d'identité de séquence avec le polypeptide de SEQ ID NO : 18 et qui a une activité oxygénase dépendante de la FAD ; et
(19) une séquence nucléotidique codant pour un polypeptide qui comprend une séquence d'acides aminés ayant au moins 80%, tel qu'au moins 85%, d'identité de séquence avec le polypeptide de SEQ ID NO : 19 et qui a une activité cyclase.

2. Groupe de gènes selon la revendication 1, dans lequel ledit groupe de gènes comprend au moins une des séquences nucléotidiques (20) et (21) :
(20) une séquence nucléotidique codant pour un polypeptide qui comprend une séquence d'acides aminés ayant au moins 80%, tel qu'au moins 85%, d'identité de séquence avec le polypeptide de SEQ ID NO : 20 et qui a une activité d'activateur transcriptionnel ; et
(21) une séquence nucléotidique codant pour un polypeptide qui comprend une séquence d'acides aminés ayant au moins 80%, tel qu'au moins 85%, d'identité de séquence avec le polypeptide de SEQ ID NO : 21 et qui a une activité d'activateur transcriptionnel.

3. Groupe de gènes selon la revendication 2, dans lequel ledit groupe de gènes comprend les deux séquences nucléotidiques (20) et (21).

4. Groupe de gènes selon l'une quelconque des revendications 1 à 3, dans lequel les séquences nucléotidiques (18) et (19) sont liées pour former une protéine de fusion des polypeptides respectifs codés par elles.

5. Groupe de gènes selon la revendication 4, dans lequel la protéine de fusion comprend une séquence d'acides aminés ayant au moins 80%, tel qu'au moins 85 %, d'identité de séquence avec le polypeptide de SEQ ID NO: 22 et a une activité cyclase et oxygénase dépendante de la FAD.

6. Groupe de gènes selon l'une quelconque des revendications 1 à 5, dans lequel les séquences nucléotidiques (7) et (17) sont liées pour former une protéine de fusion des polypeptides respectifs codés par elles.

7. Groupe de gènes selon la revendication 6, dans lequel la protéine de fusion comprend une séquence d'acides aminés ayant au moins 80%, tel qu'au moins 85 %, d'identité de séquence avec le polypeptide de SEQ ID NO: 23 et présente des activités acyl-CoA ligase et oxygénase.

8. Construction d'ADN comprenant le groupe de gènes selon l'une quelconque des revendications 1 à 7.

9. Construction d'ADN selon la revendication 8, dans laquelle ladite construction d'ADN comprend en outre une séquence nucléotidique codant pour un polypeptide qui comprend une séquence d'acides aminés ayant au moins 80%, tel qu'au moins 85%, d'identité de séquence avec le polypeptide de SEQ ID NO: 24 et qui a une activité amidotransférase.

10. Cellule hôte recombinante comprenant le groupe de gènes selon l'une quelconque des revendications 1 à 7 ou la construction d'ADN selon la revendication 8 ou 9, dans laquelle le groupe de gènes ou la construction d'ADN est hétérologue à ladite cellule hôte.

11. Cellule hôte recombinante selon la revendication 10, qui exprime de manière hétérologue les polypeptides codés par le groupe de gènes.

12. Cellule hôte recombinante selon la revendication 10 ou 11, qui exprime de manière hétérologue un polypeptide qui comprend une séquence d'acides aminés ayant au moins 80%, tel qu'au moins 85%, d'identité de séquence avec le polypeptide de SEQ ID NO: 24 et qui a une activité amidotransférase.

13. Cellule hôte recombinante selon l'une quelconque des revendications 10 à 12, qui est une bactérie.

14. Procédé de production biosynthétique d'une tétracycline, ledit procédé comprenant les étapes consistant à a) cultiver une cellule hôte recombinante selon l'une quelconque des revendications 10 à 13 en présence d'un substrat approprié dans des conditions propices à la production de ladite tétracycline et, éventuellement, b) récupérer la tétracycline du milieu utilisé en culture.

15. Procédé selon la revendication 14, dans lequel la tétracycline est la chélocardine ou un analogue de celle-ci, dans lequel la chélocardine est un composé ayant la structure I 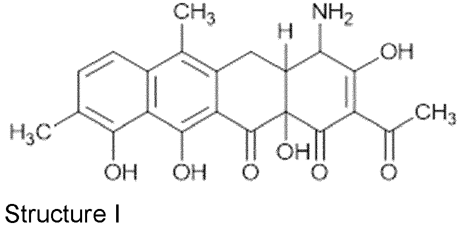 éventuellement en tant que stéréo-isomère, comprenant des énantiomères ou des diastéréoisomères, ou sous forme d'un mélange d'au moins deux stéréoisomères, comprenant des énantiomères et / ou des diastéréoisomères, dans un rapport de mélange quelconque, ou un de leurs sels correspondants, ou son solvate correspondant ;
et l'analogue de la chélocardine étant un composé ayant la structure III 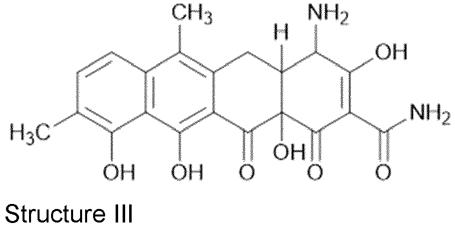 éventuellement en tant que stéréo-isomère, comprenant des énantiomères ou des diastéréoisomères, ou sous forme d'un mélange d'au moins deux stéréoisomères, comprenant des énantiomères et / ou des diastéréoisomères, dans un rapport de mélange quelconque, ou un de leurs sels correspondants, ou son solvate correspondant.


### SEQUENCE LISTING

<110> Helmholtz-Zentrum für Infektionsforschung GmbH Acies Bio d.o.o.
<120> Gene cluster for the biosynthetic production of tetracycline compounds in a heterologous host
<130> HZ01P001WO
<150> EP17210536
   <151> 2017-12-22
<160> 49
<170> PatentIn version 3.5
<210> 1
   <211> 432
   <212> PRT
   <213> Amycolatopsis sulphurea
<400> 1
   Met Thr Gly Pro Ser Asp Ala His Arg Val Val Ile Thr Gly Ile Gly
   1               5                   10                  15
   Val Val Ala Pro Gly Asp Arg Gly Thr Lys Gln Phe Trp Glu Leu Ile
               20                  25                  30
   Thr Ala Gly Arg Thr Ala Thr Arg Pro Ile Ser Leu Phe Asp Ala Ser
           35                  40                  45
   Ser Phe Arg Ser Arg Val Ala Ala Glu Cys Asn Phe Asp Pro Ile Ala
       50                  55                  60
   Ala Gly Leu Ser Gln Arg Gln Ile Arg Lys Trp Asp Arg Thr Thr Gln
   65                  70                  75                  80
   Phe Cys Val Val Ala Ala Arg Glu Ala Val Ala Asp Ser Gly Met Leu
                   85                  90                  95
   Gly Glu Gln Asp Pro Leu Arg Thr Gly Val Ala Ile Gly Thr Ala Cys
               100                 105                 110
   Gly Met Thr Gln Ser Leu Asp Arg Glu Tyr Ala Val Val Ser Asp Glu
           115                 120                 125
   Gly Ser Ser Trp Leu Val Asp Pro Asp Tyr Gly Val Pro Gln Leu Tyr
       130                 135                 140
   Asp Tyr Phe Leu Pro Ser Ser Met Ala Thr Glu Ile Ala Trp Leu Val
   145                 150                 155                 160
   Glu Ala Glu Gly Pro Val Gly Leu Val Ser Thr Gly Cys Thr Ser Gly
                   165                 170                 175
   Val Asp Val Ile Gly His Ala Ala Asp Leu Ile Arg Asp Gly Glu Ala
               180                 185                 190
   Asp Ile Met Val Ala Gly Ala Ser Glu Ala Pro Ile Ser Pro Ile Thr
           195                 200                 205
   Val Ala Cys Phe Asp Ala Ile Lys Ala Thr Thr Ala Arg Asn His Glu
       210                 215                 220
   Pro Glu Ser Ala Ser Arg Pro Phe Asp Gln Thr Arg Ser Gly Phe Val
   225                 230                 235                 240
   Leu Gly Glu Gly Ala Ala Val Phe Val Leu Glu Glu Leu Arg His Ala
                   245                 250                 255
   Lys Arg Arg Gly Ala His Ile Tyr Ala Glu Ile Val Gly Tyr Ala Ser
               260                 265                 270
   Arg Cys Asn Ala Tyr Ser Met Thr Gly Leu Arg Pro Asp Gly Arg Glu
           275                 280                 285
   Met Ala Asp Ala Ile Asp Gly Ala Leu Asn Gln Ala Arg Ile Asp Pro
       290                 295                 300
   Ser Arg Ile Gly Tyr Val Asn Ala His Gly Ser Ser Thr Arg Gln Asn
   305                 310                 315                 320
   Asp Arg His Glu Thr Ala Ala Ile Lys Thr Ser Leu Gly Ala His Ala
                   325                 330                 335
   Tyr Gln Val Pro Val Ser Ser Ile Lys Ser Met Val Gly His Ser Leu
               340                 345                 350
   Gly Ala Ile Gly Ser Leu Glu Val Ala Ala Cys Ala Leu Thr Ile Glu
           355                 360                 365
   His Ser Val Ile Pro Pro Thr Ala Asn Leu His Val Pro Asp Pro Glu
       370                 375                 380
   Cys Asp Leu Asp Tyr Val Pro Leu Val Ala Arg Glu Gln Glu Val Asp
   385                 390                 395                 400
   Val Val Leu Ser Val Ala Ser Gly Phe Gly Gly Phe Gln Ser Ala Ile
                   405                 410                 415
   Leu Leu Thr Gly Pro Asp Gly Arg Thr Gly Lys Arg Val Thr Gln Arg
   420 425 430
<210> 2
   <211> 418
   <212> PRT
   <213> Amycolatopsis sulphurea
<400> 2
   Met Ser Val Glu Thr Glu Pro Ala Pro Val Pro Gly Arg Ser Thr Val
   1 5 10 15
   Arg Pro Val Val Thr Gly Leu Gly Val Ile Ala Pro Asn Gly Met Gly
   20 25 30
   Thr Glu Ala Tyr Trp Ala Ala Thr Leu Arg Gly Asp Ser Gly Leu Arg
   35 40 45
   Arg Ile Thr Arg Phe Asp Pro Asp Gly Tyr Pro Ala Arg Ile Ala Gly
   50 55 60
   Glu Val Ser Phe Asp Pro Ala Gly Arg Leu Pro Asp Arg Leu Leu Pro
   65 70 75 80
   Gln Thr Asp His Met Thr Arg Leu Ala Leu Ile Ala Ala Glu Glu Ala
   85 90 95
   Leu Ala Asp Ala Gly Ala Asp Pro Arg Asn Leu Pro Asp Tyr Ala Thr
   100 105 110
   Gly Val Met Thr Ala Ala Ser Gly Gly Gly Phe Glu Phe Gly Gln Arg
   115 120 125
   Glu Leu Gln Glu Leu Trp Ser Lys Gly Gly Ser Tyr Val Ser Ala Tyr
   130 135 140
   Gln Ser Phe Ala Trp Phe Tyr Pro Val Asn Thr Gly Gln Ile Ser Ile
   145 150 155 160
   Arg His Gly Met Arg Gly Ser Ser Gly Thr Leu Val Ser Glu Gln Ala
   165 170 175
   Gly Gly Leu Asp Ala Val Ala Lys Ala Arg Arg His Val Arg Asp Gly
   180 185 190
   Thr Pro Leu Met Val Thr Gly Gly Ile Asp Gly Ser Leu Cys Pro Trp
   195 200 205
   Ser Trp Leu Cys Met Leu Arg Ser Gly Arg Leu Ser Thr Ala Ser Asp
       210                 215                 220
   Pro Gln Arg Ala Tyr Leu Pro Phe Asp Thr Glu Ala Ser Gly Met Val
   225                 230                 235                 240
   Pro Gly Glu Gly Gly Ala Leu Leu Val Ile Glu Asp Pro Ala Ala Ala
                   245                 250                 255
   Gln Arg Arg Gly Val Asp Arg Ile Tyr Gly Gln Ile Ala Gly Tyr Cys
               260                 265                 270
   Ala Thr Phe Asp Pro Gly Pro Gly Ser Arg Arg Pro Pro Gly Leu Arg
           275                 280                 285
   Arg Ala Val Glu Gln Ala Leu Ala Glu Ala Arg Leu His Pro Ser Glu
       290                 295                 300
   Val Asp Val Val Phe Ala Asp Ala Ala Gly Leu Pro Asp Leu Asp Arg
   305                 310                 315                 320
   Ala Glu Ile Glu Val Leu Val Arg Ile Phe Gly Ala Arg Ala Val Pro
                   325                 330                 335
   Val Thr Ala Pro Lys Thr Met Thr Gly Arg Leu Leu Ala Gly Gly Ser
               340                 345                 350
   Ser Leu Asp Leu Ala Thr Ala Leu Leu Ser Leu Arg Asp Lys Val Ile
           355                 360                 365
   Pro Pro Thr Val His Ile Gly Lys Phe Gly Tyr Arg Asp Glu Ile Asp
       370                 375                 380
   Leu Val Arg Asp Ser Pro Arg Gln Ala Pro Leu Ser Thr Ala Leu Val
   385                 390                 395                 400
   Leu Ala Arg Gly Tyr Gly Gly Phe Asn Ser Ala Met Val Leu Arg Gly
                   405                 410                 415
   Ala Thr
<210> 3
   <211> 88
   <212> PRT
   <213> Amycolatopsis sulphurea
<400> 3
   Met Ala Glu Phe Thr Ile Ala Glu Leu Val Arg Leu Leu Arg Glu Cys
   1 5 10 15
   Ala Gly Glu Glu Glu Gly Val Asp Leu Asp Gly Glu Val Gly Asp Leu
   20 25 30
   Pro Phe Asp Glu Leu Gly Tyr Asp Ser Leu Ala Leu Phe Asn Thr Ile
   35 40 45
   Gly Arg Ile Glu Arg Glu Tyr Thr Val Asp Leu Pro Glu Asp Val Val
   50 55 60
   Trp Gln Ala Thr Thr Pro Gly Ala Leu Val Asp Leu Val Asn Ser Ser
   65 70 75 80
   Arg Thr Ser Pro Ala Ala Ala Asp
   85
<210> 4
   <211> 302
   <212> PRT
   <213> Amycolatopsis sulphurea
<400> 4
   Met Ser His Pro Glu Ala Glu Gln Thr Gln Ala Ser Ile Val Val Asp
   1 5 10 15
   Ala Pro Ala Glu Ile Thr Tyr Ala Met Leu Val Asp Val Ala Asn Trp
   20 25 30
   Pro Leu Leu Tyr Pro Trp Ile Ala His Thr Glu Phe Val Glu Arg Ala
   35 40 45
   Pro Thr Glu Asp Leu Val Gln Phe Trp Ala Val Asn Pro Leu Gly Arg
   50 55 60
   Ile Arg Ile Trp Thr Ser Arg Arg Tyr Leu Asp Ala Ser Ala Leu Arg
   65 70 75 80
   Met Asp Ile Glu Gln Gln Gly Ser Val Gly Pro Ile Thr Gly Leu Thr
   85 90 95
   Gly Ser Trp Thr Phe Lys Pro Leu Pro Gly Asp Arg Cys Leu Val Glu
   100 105 110
   Ser Arg His Ala Phe His Ala Ala Thr Pro Glu Asp Arg Ala Ala Gly
   115 120 125
   Val Thr Glu Leu Asn Arg His Gly Lys Leu Gln Met Glu Thr Leu Lys
       130                 135                 140
   Ser Arg Val Glu Asn Arg Thr Arg Leu Ala Glu Leu Thr Trp Ser Phe
   145                 150                 155                 160
   Glu Asp Ser Leu Val Ile Glu Ser Glu Leu Gly Gln Val Tyr Arg Ala
                   165                 170                 175
   Leu Arg Asp Val Gly Ser Trp Pro Ala His Leu Pro Cys Leu Thr Ala
               180                 185                 190
   Leu Glu Val Thr Glu Asp Glu Asn Asp Val Gln Phe Tyr Asp Val Arg
           195                 200                 205
   Thr Gln Asp Ala Asp Glu Pro Ser Arg Phe Val Arg Ile Cys Leu Pro
       210                 215                 220
   Asp Lys Gly Ile Ala Tyr Lys Gln Leu Thr Val Thr Ala Pro Val Asp
   225                 230                 235                 240
   Leu His Leu Gly Arg Trp Thr Leu Thr Glu Thr Pro Ala Gly Val Ala
                   245                 250                 255
   Val Thr Ser Ala His Thr Val Leu Val Asn Pro Ser Ala Ala Glu Gln
               260                 265                 270
   Leu Pro Glu Leu Arg Asp Arg Leu His Lys Thr Ser Ser Ala Asp Ser
           275                 280                 285
   Leu Ala Glu Leu Gln Leu Val Lys Arg Leu Ala Glu Thr Arg
       290                 295                 300
<210> 5
   <211> 315
   <212> PRT
   <213> Amycolatopsis sulphurea
<400> 5
   Met Pro Ala Ala Ala Gln Gln His Thr Glu His Arg Ile Asp Ile Asp
   1               5                   10                  15
   Ala Pro Ala Gly Leu Val Tyr Arg Ile Ile Ala Asp Ala Thr Glu Trp
               20                  25                  30
   Pro Arg His Phe Thr Pro Thr Val His Val Asp Gln Ser Glu Leu Asp
           35                  40                  45
   Gly His Thr Glu Arg Leu His Ile Trp Ala Asn Ala Asn Gly Gln Leu
       50                  55                  60
   Lys Ser Trp Thr Ser Leu Arg Glu Leu Asp Glu Arg Ala Gly Arg Ile
   65                  70                  75                  80
   Arg Phe Arg Gln Glu Val Ser Ala Pro Pro Val Ala Ser Met Ser Gly
                   85                  90                  95
   Glu Trp Ile Val Ser Glu Arg Val Ala Glu Arg Thr Thr Leu Val Leu
               100                 105                 110
   Thr His Asp Phe Ala Ala Val Asp Asp Asp Pro Ala Gly Val Glu Trp
           115                 120                 125
   Ile Thr Lys Ala Thr Asn Gly Asn Ser Asp Thr Glu Leu Ala Asn Ile
       130                 135                 140
   Lys Ala Leu Ala Glu Arg Trp Glu Arg Met Asp Arg Leu Ala Phe Asp
   145                 150                 155                 160
   Phe Glu Asp Ser Val Leu Val Arg Ala Pro Lys Glu Arg Ala Tyr His
                   165                 170                 175
   Phe Leu Asp Arg Val Asp Leu Trp Pro Asp Arg Leu Pro His Val Ala
               180                 185                 190
   Arg Leu Glu Leu Arg Glu Asp Val Pro Gly Val Gln His Met Ser Met
           195                 200                 205
   Asp Thr Lys Ala Lys Asp Gly Ser Thr His Thr Thr Val Ser Val Arg
       210                 215                 220
   Val Cys Phe Pro Glu Ala Arg Ile Val Tyr Lys Gln Leu Val Pro Pro
   225                 230                 235                 240
   Ala Leu Leu Thr Thr His Thr Gly Val Trp Thr Phe Glu Asp Thr Ala
                   245                 250                 255
   Asp Gly Val Leu Val Thr Ser Ala His Thr Val Val Leu Asn Glu Ala
               260                 265                 270
   Asn Ile Gly Thr Val Pro Gly Pro Ala Ala Thr Val Glu Ser Thr Arg
           275                 280                 285
   Asp Phe Val Arg Asn Ala Ile Ser Gly Asn Ser Gln Ala Thr Leu Arg
   290 295 300
   His Ala Lys Ala Phe Ala Glu Ala Thr Asp Ala
   305 310 315
<210> 6
   <211> 150
   <212> PRT
   <213> Amycolatopsis sulphurea
<400> 6
   Met Ser Gln Ala Val Gln Ala Val Gly Ser Thr Glu Asp Ile Ala Leu
   1 5 10 15
   Tyr Val Glu Val Gln Gln Phe Tyr Gly Arg Gln Met Arg Tyr Leu Asp
   20 25 30
   Glu Gly Arg Val Gln Glu Trp Ala Lys Thr Phe Thr Glu Asp Gly Met
   35 40 45
   Phe Ala Ala Asn Ala His Pro Glu Pro Ala Arg Gly Arg Thr Ala Ile
   50 55 60
   Glu Ala Gly Ala Leu Glu Ala Ala Thr Arg Leu Ala Glu Gln Gly Ile
   65 70 75 80
   Gln Arg Arg His Trp Leu Gly Met Val Gln Val Asp Pro Gln Pro Asp
   85 90 95
   Gly Ser Ile Val Ala Lys Ser Tyr Ala Val Ile Ile Gly Thr Pro Leu
   100 105 110
   Gly Gly Lys Ala Ala Val Asp Leu Ser Cys Asp Cys Val Asp Val Leu
   115 120 125
   Val Arg Glu Gly Gly Ala Leu Leu Val Arg Glu Arg Gln Val Tyr Arg
   130 135 140
   Asp Asp Leu Pro Arg Asn
   145 150
<210> 7
   <211> 547
   <212> PRT
   <213> Amycolatopsis sulphurea
<400> 7
   Met Ala Thr Pro Glu Thr Thr Val Ala Gln Asp Ser Thr Val Ser Gly
   1               5                   10                  15
   Asp Pro Leu Thr Gly Ile Ala Gly Phe Ile Gly Gly Pro Arg Val Asp
               20                  25                  30
   Asp Leu Leu Arg Ser Ala Ala Glu Arg Ala Pro Glu His Val Ala Ile
           35                  40                  45
   Arg Ala Ala Ala Cys Glu Leu Ser Tyr Ala Ala Leu Asp Glu Arg Ala
       50                  55                  60
   Gly Arg Leu Ala Arg Ala Val His Ala Gln Leu Ala Glu Pro Gly Gln
   65                  70                  75                  80
   Val Val Ala Leu Ala Gly Val Leu Asp Pro Ala Phe Ala Val Ser Phe
                   85                  90                  95
   Phe Gly Ile Ala Arg Ser Gly Ala Val Pro Ala Leu Ile Asn Pro Leu
               100                 105                 110
   Leu Leu Ala Asp Gly Leu Val His Val Leu Gly Thr Ser Gly Ala Val
           115                 120                 125
   Thr Ala Ile Val Pro Pro Glu Val His Arg Arg Leu Val Pro Val Leu
       130                 135                 140
   Asp Arg Leu Pro Glu Leu Arg His Leu Val Leu Thr His Arg Asp Asp
   145                 150                 155                 160
   Gln Thr Glu Pro Ser Gly Pro Pro Thr Leu Asp Glu Val Leu Ala Ser
                   165                 170                 175
   Ala Pro Asp Gly Val Leu Pro Ala Thr Thr Cys Asp Glu Asn Ser Val
               180                 185                 190
   Ala Cys Leu Gln Phe Thr Ser Gly Thr Thr Gly Ala Ala Lys Ala Val
           195                 200                 205
   Arg Leu Ser His Arg Asn Ile Thr Val Asn Ala Ala Gln Ser Gly His
       210                 215                 220
   Ala His Gly Ile Thr Pro Ser Ser Val Leu Phe Asn Tyr Leu Pro Thr
   225                 230                 235                 240
   Phe His Leu Met His Leu Thr Met Ala Val Thr Phe Ala Ala Thr Leu
                   245                 250                 255
   Val Leu His Val Gly Asp Asp Val Ala Gln Ala Val Asp Ala Ala Asp
   260 265 270
   Asn Glu Lys Ala Thr His Phe Tyr Ser Leu Pro Met Arg Leu Ser Arg
   275 280 285
   Leu Ala Val His Pro Arg Leu Ser Thr Leu Ala Ala Asp Ala Leu Gln
   290 295 300
   Val Ile Leu Cys Gly Gly Ser Ala Leu Pro Leu Pro Ser Thr Arg Ala
   305 310 315 320
   Leu Thr Gly Cys Phe Gly Val Pro Val Val Gln Gly Tyr Gly Leu Gln
   325 330 335
   Glu Thr Ser Pro Ser Thr His Phe Asp Ser Leu Ser Cys Pro Lys Thr
   340 345 350
   Gly Ser Ser Gly Arg Pro Val Ala Gly Thr Gly Cys Arg Ile Val Asp
   355 360 365
   Val Asp Ser Arg Ala Val Leu Pro Val Gly Glu Lys Gly Glu Ile Gln
   370 375 380
   Val Arg Gly Pro Gln Leu Met Leu Gly Tyr Leu Gly Arg Glu Pro Gly
   385 390 395 400
   Gln Asp Val Asp Pro Asp Gly Trp Phe Ser Thr Gly Asp Val Gly Tyr
   405 410 415
   Val Asp Ala Glu Gly Val Leu Phe Val Val Asp Arg Ile Lys Asp Val
   420 425 430
   Phe Lys Cys Asp Asn Trp Leu Val Ser Pro Thr Glu Ile Glu Arg Val
   435 440 445
   Val Leu Ser His Pro Glu Val Ala Asp Cys Val Val Leu Asp Tyr Pro
   450 455 460
   Asp Asp Phe Ser Gly Ser Val Ala Tyr Gly Leu Val Val Pro Lys Gly
   465 470 475 480
   Ala Gly Leu Asn Pro Ala Gln Leu Ala Glu Phe Val Ala Glu Arg Leu
   485 490 495
   Pro Tyr Tyr Ala His Leu Arg His Val Glu Leu Thr Asp Arg Ile Pro
   500 505 510
   Arg Ser Pro Asn Gly Lys Leu Gln Arg Arg Ala Leu Arg Glu Gln Ile
   515 520 525
   His Ala Arg Asn Ala Asp Gly Ala Ser Glu Ile Ala Arg Gln Asn Arg
   530 535 540
   Ser Lys Thr
   545
<210> 8
   <211> 262
   <212> PRT
   <213> Amycolatopsis sulphurea
<400> 8
   Met Arg Ser Thr Glu Glu Pro Arg Val Ala Leu Val Thr Gly Ala Thr
   1 5 10 15
   Ser Gly Ile Gly Leu Ala Val Thr Lys Ala Leu Ala Ala Arg Gly Leu
   20 25 30
   Arg Val Phe Ile Cys Ala Arg Asn Arg Glu Asn Val Val Ser Thr Val
   35 40 45
   Lys Glu Leu Arg Ala Gln Gly Leu Asp Val Asp Gly Gln Ala Gly Asp
   50 55 60
   Val Arg Ser Val Ala Ala Val Arg Glu Val Val Glu Ser Ala Val Asn
   65 70 75 80
   Arg Phe Gly Thr Ile Ser Val Leu Val Asn Asn Ala Gly Arg Ser Gly
   85 90 95
   Gly Gly Ile Thr Ala Lys Ile Thr Asp Glu Leu Trp Gln Asp Val Ile
   100 105 110
   Asp Thr Asn Leu Asn Ser Val Phe Thr Val Thr Arg Glu Val Leu Thr
   115 120 125
   Thr Gly Gly Leu Asp Gly Ala Asp Gly Gly Arg Ile Ile Asn Ile Ala
   130 135 140
   Ser Thr Gly Gly Lys Gln Gly Val Pro Leu Gly Ala Pro Tyr Ser Ala
   145 150 155 160
   Ala Lys Ser Gly Val Ile Gly Phe Thr Lys Ala Leu Gly Lys Glu Leu
   165 170 175
   Ala Lys Thr Gly Val Thr Val Asn Ala Val Cys Pro Gly Tyr Val Glu
   180 185 190
   Thr Pro Met Ala Val Arg Val Arg Gln Ala Tyr Ala Ser Thr Trp Asp
   195 200 205
   Thr Thr Asp Glu Asn Val Leu Ala Arg Phe Asn Asp Lys Ile Pro Leu
   210 215 220
   Gly Arg Tyr Cys Thr Pro Glu Glu Val Ala Gly Met Val Asp Tyr Leu
   225 230 235 240
   Val Ala Asp Thr Ala Ala Ser Val Thr Ala Gln Ala Ile Asn Val Cys
   245 250 255
   Gly Gly Leu Gly Asn Tyr
   260
<210> 9
   <211> 341
   <212> PRT
   <213> Amycolatopsis sulphurea
<400> 9
   Met Thr Gly Thr Val Leu Pro Ala Ala Val Met Arg Val Arg Glu Leu
   1 5 10 15
   Ala Leu Ser Ala Ala Cys Ala Ala Ser Val Arg Ala Ala Ala Lys Leu
   20 25 30
   Gly Leu Ala Asp Val Leu Asp Asp Gln Pro Ala Thr Val Asp Glu Leu
   35 40 45
   Ala Lys Ala Val His Ala Asp Pro Gly Ala Leu Arg Arg Leu Met Arg
   50 55 60
   Ser Leu Thr Cys Phe Glu Val Phe Ala Glu Pro Glu Pro Asp Lys Phe
   65 70 75 80
   Val His Thr Asp Ala Ser Arg Leu Leu Arg Glu Asp Ala Pro Arg Ser
   85 90 95
   Leu Lys His Ile Leu Leu Trp Gly Thr Glu Pro Trp Thr Trp Glu Leu
   100 105 110
   Trp Pro His Leu Asp Gln Ala Val Arg Thr Gly Lys Asn Val Phe Asp
   115 120 125
   Asp Leu His Gly Lys Asp Phe Phe Glu Tyr Leu His Glu Gln Trp Pro
   130 135 140
   Glu Ser Ala Glu Val Phe Asp Lys Ala Met Thr Gln Ser Ser Lys Leu
   145 150 155 160
   Ser Ala Leu Ala Ile Ala Asp Arg Leu Asp Leu Thr Gly Ala Glu Arg
   165 170 175
   Leu Ala Asp Ile Ala Gly Gly Gln Gly Asn Val Leu Ala Thr Leu Leu
   180 185 190
   Ser Arg Asn Glu Lys Leu Asn Gly Val Leu Phe Asp Leu Pro Ala Val
   195 200 205
   Val Ala Gly Ala Asp Glu Arg Leu Arg Val Gly Gly Ala Leu Ala Asp
   210 215 220
   Arg Ala Glu Leu Val Ala Gly Asp Cys Arg Arg Glu Ile Pro Val Gln
   225 230 235 240
   Ala Asp Val Tyr Leu Phe Lys Asn Ile Leu Glu Trp Asp Asp Glu Ser
   245 250 255
   Thr Val Leu Ala Leu Arg Asn Ala Val Ala Ala Gly Arg Pro Gly Ala
   260 265 270
   Arg Val Val Ile Ile Glu Asn Leu Val Asp Gly Thr Pro Glu Met Lys
   275 280 285
   Phe Ala Thr Ala Met Asp Leu Leu Leu Leu Leu Asn Val Gly Gly Lys
   290 295 300
   Lys His Thr Lys Asp Gly Leu Leu Gly Leu Ile Gly Gln Ala Gly Leu
   305 310 315 320
   Gln Val Asp Arg Val Ser Ala Val Asn Ser Tyr Leu His Met Val Glu
   325 330 335
   Thr Thr Ile Pro Gly
   340
<210> 10
   <211> 343
   <212> PRT
   <213> Amycolatopsis sulphurea
<400> 10
   Met Thr Glu Thr Glu Pro Thr Thr Val Thr Ala Lys Lys Leu His Glu
   1               5                   10                  15
   Ile Met Arg Gly Tyr Val Lys Thr Ala Leu Leu Arg Thr Ala Ile Glu
               20                  25                  30
   Leu Asn Ile Phe Asp Gly Ile Gly Asp Arg Thr Val Asp Ala Asp Gly
           35                  40                  45
   Leu Ala Arg Ala Leu Gly Val Asp Ala Arg Gly Leu Arg Ile Thr Leu
       50                  55                  60
   Asp Ser Leu Ala Ala Ile Gly Leu Leu Arg Thr Val Asp Gly Lys Tyr
   65                  70                  75                  80
   Ala Leu Pro Val Asp Gly Asp Lys Phe Leu Leu Ser Ser Ser Pro Thr
                   85                  90                  95
   Phe Phe Gly Pro Ser Leu Lys Leu Gly Ala Ser Asp Trp Glu Trp Asp
               100                 105                 110
   Ala Gln Lys Arg Leu Thr Glu Ala Val Arg Lys Gly Gly Ala Val Met
           115                 120                 125
   Asp Ser His Ala Leu Thr Pro Glu Phe Asp Tyr Trp Glu Asp Phe Ala
       130                 135                 140
   Glu Asn Thr Thr Trp Phe Asn Asn Gly Ala Ala Glu Leu Met Ala Glu
   145                 150                 155                 160
   Gln Leu Leu Pro Trp Ala Lys Asp Arg Asp Ser Val Asp Val Leu Asp
                   165                 170                 175
   Val Ala Cys Ser His Gly Tyr Tyr Gly Val Asn Leu Ala Lys Ala Glu
               180                 185                 190
   Pro Lys Ala Arg Val Trp Gly Val Asp Trp Pro Asn Val Leu Pro Ile
           195                 200                 205
   Thr Ala Lys Asn Tyr Glu Arg Asn Gly Ile Ser Asp Arg Phe Glu Gly
       210                 215                 220
   Ile Pro Gly Asp Met Phe Ser Val Pro Leu Gly Gly Pro Tyr Asp Val
   225                 230                 235                 240
   Val Met Ile Thr Asn Val Leu His His Phe Ser Ala Asp Thr Ser Thr
                   245                 250                 255
   Asn Leu Leu Arg Arg Leu Phe Asp Val Leu Lys Pro Gly Gly Arg Ile
   260 265 270
   Ala Val Thr Gly His Thr Phe Val Glu Gly Glu Arg Pro Glu Asp Lys
   275 280 285
   Pro Leu Pro Tyr Leu Phe Ser Gln Ile Met Leu Val Met Thr Asp Glu
   290 295 300
   Gly Glu Thr His Ser Thr Lys Thr Tyr Glu Arg Met Phe Thr Asp Ala
   305 310 315 320
   Gly Phe Val Asn Pro Gln Ile Phe Thr Ala Glu Lys Ala Met His Thr
   325 330 335
   Val Phe Thr Ala Asp Lys Ala
   340
<210> 11
   <211> 448
   <212> PRT
   <213> Amycolatopsis sulphurea
<400> 11
   Met Thr Met Thr Lys Ala Arg Glu Asp Leu Thr Thr Gly Ser Ala Val
   1 5 10 15
   Thr Leu Glu Leu Ala Gly Leu His Thr Gly Val Ser Asp Pro Arg Met
   20 25 30
   Asp Ser Met Arg Leu Leu Ser Glu Thr Ala Phe Arg Tyr Pro Gln Ala
   35 40 45
   Val Ser Phe Ala Ser Gly Arg Pro Tyr Glu Gly Phe Phe Asp Ile Ala
   50 55 60
   Lys Leu His His Tyr Leu Asp Arg Phe Val Glu His Leu Arg Glu Arg
   65 70 75 80
   Gly Met Pro Glu Glu Arg Ile Lys Lys Ala Leu Phe Gln Tyr Gly Pro
   85 90 95
   Ile Asn Gly Leu Ile Arg Asp Met Ile Ala Arg Thr Leu Glu Val Asp
   100 105 110
   Glu Asp Ile His Val Ala Pro Glu Ala Val Met Val Thr His Gly Cys
   115 120 125
   Gln Glu Ala Met Met Ile Ala Leu Arg Gly Leu Phe Ala Ser Pro Ser
   130 135 140
   Asp Val Leu Leu Thr Val Ser Pro Cys Tyr Val Gly Ile Ala Gly Ala
   145 150 155 160
   Ala Lys Met Leu Asp Ile Pro Met Ala Ala Val Pro Glu Gly Ala Glu
   165 170 175
   Gly Ile Asp Pro Glu Gln Val Ala Ala Val Ala Arg Glu Val Arg Ala
   180 185 190
   Ser Gly Leu Arg Pro Val Ala Cys Tyr Val Thr Pro Asp Phe Ser Asn
   195 200 205
   Pro Ser Gly His Ser Leu Pro Ile Ala Thr Arg His Arg Leu Leu Glu
   210 215 220
   Val Ala Ala Glu Gln Asp Leu Leu Leu Leu Glu Asp Asn Pro Tyr Gly
   225 230 235 240
   Leu Phe Gly Arg Asp Gly Ala Gln Val Pro Thr Leu Lys Ala Leu Asp
   245 250 255
   Thr Gln Arg Arg Val Ile Tyr Leu Gly Ser Phe Ala Lys Thr Val Phe
   260 265 270
   Pro Gly Ala Arg Val Gly Tyr Leu Val Ala Asp Gln Glu Val Thr Gly
   275 280 285
   Gly Pro Gly Ala Ala Lys Pro Leu Ala Glu Glu Leu Gly Lys Val Lys
   290 295 300
   Ser Met Phe Thr Val Gly Thr Ser Gly Ile Ser Gln Ala Leu Val Gly
   305 310 315 320
   Gly Val Leu Leu Asp Ala Asp Phe Ser Leu Arg Thr Ala Asn Arg Glu
   325 330 335
   Leu Ala Asp Leu Tyr Val Arg His Leu Glu Val Thr Leu Ser Ser Leu
   340 345 350
   Ala Glu His Phe Pro Pro Glu Arg Phe Ala Glu His Gly Val Arg Trp
   355 360 365
   Asn Val Pro Gly Gly Gly Phe Phe Leu Ser Val Glu Val Pro Phe Val
   370 375 380
   Ala Gly Leu Glu Ala Leu Asp Arg Ser Ala Arg Asp His Gly Val Gly
   385 390 395 400
   Trp Ala Pro Met Ser Met Phe Tyr Val Gly Asp Gly Gly Glu His Ile
   405 410 415
   Val Arg Leu Gly Phe Ser Pro Leu Thr Ala Glu Glu Ile Arg Glu Gly
   420 425 430
   Val Arg Arg Leu Ala Glu Phe Val Lys Ala Thr Pro Arg Thr Asp Arg
   435 440 445
<210> 12
   <211> 399
   <212> PRT
   <213> Amycolatopsis sulphurea
<400> 12
   Met Arg Leu Leu Leu Val Thr Trp Asn Ala Pro Ala His Leu Phe Ala
   1 5 10 15
   Met Val Pro Leu Gly Trp Ala Ala Gln Val Ala Gly His Glu Val Arg
   20 25 30
   Val Ala Ala Pro Pro Ser Cys Thr Glu Ala Ile Gly Arg Thr Gly Leu
   35 40 45
   Thr Ala Val Pro Val Gly Thr Gln Arg Pro Ala Ala Pro Ser Gly Pro
   50 55 60
   Pro Pro Gly Ala Pro Ser Gly Arg Trp Pro Val Asp Trp Ala Val His
   65 70 75 80
   Pro Glu Leu Leu Asp Asp Ser Arg His Glu Leu Leu Arg Ser Leu Ala
   85 90 95
   Ala Arg Gln Phe Ala Ala Ala Glu Pro Met Leu Asp Asp Leu Ile Glu
   100 105 110
   Phe Ala Arg Trp Trp Ser Pro Asp Val Val Val Tyr Asp Pro Thr Ser
   115 120 125
   Leu Ala Gly Glu Val Ala Ala Thr Val Leu Gly Val Pro Ala Phe Ala
   130 135 140
   Cys Ser Trp Gly Arg Ala Ala Ala Val Arg Ile Glu Arg Gly Leu Gly
   145 150 155 160
   Ser Glu Pro Leu Leu Gly Tyr Ala Arg Leu Phe Glu Arg Phe Gly Cys
   165 170 175
   Gln Ala Pro Gln Gly Pro Ala Ser Trp Phe Asp Pro Phe Pro Ala Gly
   180 185 190
   Leu Trp Leu Ala Glu Pro Asp Leu Pro Arg Gln Ala Met Arg Phe Val
   195 200 205
   Pro Gly Thr Gly Gly Asp Ala Gly Ser Leu Pro Gly Trp Leu Arg Glu
   210 215 220
   His Ser Ala Arg Pro Arg Ile Cys Val Thr Ser Ala Glu Pro Gly Gly
   225 230 235 240
   Leu Leu Arg Pro Glu Ala Val Arg Ala Phe Tyr Arg His Ala Leu Thr
   245 250 255
   Val Leu Ser Asp Val Asp Ala Glu Val Val Val Pro Ala Gly Pro Ala
   260 265 270
   Ala Arg Thr Leu Leu Ala Glu Ile Pro His Thr Ala Arg Ile Val Asp
   275 280 285
   Pro Val Ala Ala His Leu Leu Val Pro Ala Cys Arg Leu Thr Val His
   290 295 300
   Gln Gly Asp Gly Leu Ser Thr Leu Ala Gly Leu Asn Ser Gly Val Pro
   305 310 315 320
   Gln Tyr Val Leu Ala Pro Arg Pro Glu Gln Glu Leu Val Gly His Gln
   325 330 335
   Leu His Arg Ala Gly Ala Gly Gly Tyr Arg Ser Leu Ser Glu Pro Val
   340 345 350
   Asp Val Pro Ala Glu Arg Ala Val Leu Asp Ala Leu Leu Ala Pro Glu
   355 360 365
   Ser Gly Gly Ala Ala Ala Arg Lys Leu Gln Glu Glu Thr Leu Ala Leu
   370 375 380
   Pro Leu Pro Ser Ala Val Leu Gly Arg Ile Glu Ser Ala Thr Arg
   385 390 395
<210> 13
   <211> 511
   <212> PRT
   <213> Amycolatopsis sulphurea
<400> 13
   Met Ser Val His Gly Val Met Pro Ser Gly Trp Ser Ser Pro Val Gly
   1               5                   10                  15
   Arg Leu Leu Ser Gln Ala Gly Leu Gly Leu Leu Ser Trp Val Val Pro
               20                  25                  30
   Pro Ala Leu Val Asp Glu Ala Leu Ala Val Ala Gly Arg Asp Glu Arg
           35                  40                  45
   Arg Phe Arg Ala Leu Pro Ser Arg Leu Gly Val Tyr Phe Val Leu Ala
       50                  55                  60
   Leu Cys Leu Leu Arg Thr Lys Ser Gly Asn Ala Thr Ile Arg Ala Met
   65                  70                  75                  80
   Phe Ser Gln Glu Ser Leu Pro Arg Leu Ser Val Leu Gly Trp Trp Pro
                   85                  90                  95
   Pro Ala Ser Thr Ala Leu Thr Lys Leu Arg Asp Arg Ile Gly Val Val
               100                 105                 110
   Pro Phe Gln Leu Leu Phe Gly Ala Leu Ala Arg Ala Ala Pro Thr Arg
           115                 120                 125
   Asn Arg Pro Trp Ser His Ala Phe Gly Leu Glu Val Cys Ala Trp Asp
       130                 135                 140
   Gly Thr Glu Val Glu Pro Ala Asp Thr Ala Ala Asn Arg Glu His Phe
   145                 150                 155                 160
   Pro Pro His His Arg Thr Gly Val Ala Arg Gly Pro Ser Lys Ile Arg
                   165                 170                 175
   Val Leu Val Leu Leu Ser Cys Gly Ser Arg Arg Leu Leu Gly Ala Val
               180                 185                 190
   Thr Gly Pro Leu Ser Gln Gly Glu Pro Thr Leu Ala Tyr Gln Leu Leu
           195                 200                 205
   Pro Arg Leu His Asp Arg Met Leu Leu Leu Ala Asp Arg Cys Phe Leu
       210                 215                 220
   Gly Tyr Pro Leu Trp Thr Ala Ala Arg Glu Arg Gly Ala His Leu Leu
   225                 230                 235                 240
   Trp Arg Ala Lys Gln Asn Thr Pro Lys Leu Pro Val Gln His Ala Leu
                   245                 250                 255
   Pro Asp Glu Ser Trp Leu Ser Thr Leu His Ala Pro Ala Asp Ala Arg
               260                 265                 270
   Arg Trp Ala Arg Asn Val Arg Arg Asn Lys Gln Arg Gly His Arg Pro
           275                 280                 285
   Pro Thr Pro Arg Pro Ile Asn Gly Ile Val Val Arg Val Val Glu Ala
       290                 295                 300
   Leu Ile Thr Val Thr Val Asp Gly Val Thr Arg Thr Glu Lys Tyr Arg
   305                 310                 315                 320
   Leu Val Thr Ser Leu Leu Asp Pro Ala His Ala Pro Ala Gly Gln Leu
                   325                 330                 335
   Val Ala Leu Tyr Ala Arg Arg Trp Thr Ala Glu Thr Gly Ile Lys Glu
               340                 345                 350
   Ile Lys Thr Thr Leu Leu Ala Lys Arg Pro Leu Arg Gly His Thr Pro
           355                 360                 365
   Ile Arg Ala Gln Gln Glu Leu Trp Ala Thr Leu Ile Val Tyr Gln Ala
       370                 375                 380
   Ile Arg Leu Leu Ile Ser His Ala Ala Leu Thr Gln Asn Leu Asp Pro
   385                 390                 395                 400
   Ser Arg Ile Ser Phe Thr Ser Ala Arg Asp Ala Ala Glu His Ala Ile
                   405                 410                 415
   Thr Thr Thr Pro Ala Asp Thr Ser Arg His Leu Gln Trp Val Ala Gln
               420                 425                 430
   Asp Leu Cys Arg Gln Leu Ile Thr Val His Thr His His Arg Val Tyr
           435                 440                 445
   Pro Arg Ala Leu Lys Arg Thr Thr Thr Arg Tyr Pro His Arg Ser Lys
       450                 455                 460
   Thr Pro Gln Pro Thr Ser Thr Lys Ala Ser Tyr Gln Val His Ile Leu
   465                 470                 475                 480
   Pro Thr Ala Glu Thr Thr Pro Pro Thr Thr Thr Lys Pro Thr Pro His
   485 490 495
   Gln Pro Arg Thr Asp Leu Ser Ser Trp His Trp Thr Gln Ser Pro
   500 505 510
<210> 14
   <211> 481
   <212> PRT
   <213> Amycolatopsis sulphurea
<400> 14
   Met Lys Asp Asn Leu Ala Arg Pro Asp Thr Val Gly Ala Asp Glu Asn
   1 5 10 15
   Arg Ile Ser Pro Ala Leu Trp Gly Leu Ala Ser Ile Leu Ile Leu Gly
   20 25 30
   Gly Phe Thr Ser Met Phe Thr Ser Thr Ile Val Asn Val Ala Leu Asp
   35 40 45
   Thr Leu Ser Gln Lys Leu Ser Ala Pro Leu Gly Thr Val Gln Trp Thr
   50 55 60
   Ala Thr Gly Tyr Leu Met Ala Leu Ala Thr Ala Val Pro Val Ser Gly
   65 70 75 80
   Trp Ala Ser Lys Arg Tyr Gly Ala Thr Arg Leu Trp Leu Gly Ser Val
   85 90 95
   Ala Leu Phe Thr Leu Phe Ser Ala Leu Cys Ala Leu Ser Thr Ser Val
   100 105 110
   Glu Met Leu Ile Thr Phe Arg Val Leu Gln Gly Ile Ala Gly Gly Leu
   115 120 125
   Leu Val Pro Ala Gly Gln Ile Leu Leu Val Thr Ala Ala Gly Pro Lys
   130 135 140
   Arg Ile Gly Arg Met Leu Thr Ala Val Ser Val Pro Ile Tyr Leu Ala
   145 150 155 160
   Pro Ala Val Gly Thr Thr Leu Gly Ser Val Leu Thr Gln Gly Leu Gly
   165 170 175
   Trp Pro Trp Leu Phe Trp Ile Thr Val Pro Leu Gly Ala Leu Gly Phe
   180 185 190
   Phe Ala Gly Leu Arg Trp Leu Pro Lys Ala Pro Pro Lys Gly Ala Pro
   195 200 205
   Ala Leu Asp Val Arg Gly Leu Ile Ile Leu Val Ala Gly Leu Pro Leu
   210 215 220
   Leu Thr Tyr Gly Val Ala Gly Ile Gly Glu Asn Gly Gly Arg Thr Glu
   225 230 235 240
   Thr Ile Ala Val Ile Ala Ala Val Ala Gly Ala Leu Leu Leu Ala Leu
   245 250 255
   Phe Thr Leu His Ala Val Arg Ser Arg Asn Pro Leu Leu Asn Leu Arg
   260 265 270
   Leu Phe Lys Asp Arg Ala Phe Ser Ser Ala Ala Val Val Ile Phe Cys
   275 280 285
   Met Gly Ile Ala Leu Phe Gly Ala Met Ile Val Leu Pro Ile Tyr Phe
   290 295 300
   Leu Gln Val Arg His Glu Asp Leu Val Thr Ala Gly Leu Leu Thr Ala
   305 310 315 320
   Pro Ser Ala Ile Gly Thr Val Leu Ala Leu Pro Leu Ala Gly Lys Met
   325 330 335
   Thr Asp Lys Ile Gly Gly Ala Arg Val Ile Phe Ala Gly Leu Val Val
   340 345 350
   Thr Ile Ile Gly Thr Ile Pro Leu Ala Leu Val Thr Pro His Asp Ser
   355 360 365
   Tyr Val Trp Leu Ser Leu Val Gln Ile Val Arg Gly Ile Gly Ile Gly
   370 375 380
   Met Thr Thr Thr Pro Ala Met Ala Ala Gly Leu Ala Met Ile Gly Lys
   385 390 395 400
   Glu Asp Val Pro His Ala Thr Pro Ile Phe Asn Val Leu Gln Arg Val
   405 410 415
   Gly Gly Ser Phe Gly Thr Ala Leu Thr Thr Val Leu Val Ala Phe Gln
   420 425 430
   Leu Ala Ser Gly Pro Gln Thr Asp Glu Gly Ala Ala Asp Ala Ile Gly
   435 440 445
   Tyr Thr His Trp Trp Ile Val Ala Cys Thr Ala Ile Val Leu Ile Pro
       450                 455                 460
   Ser Met Leu Leu Val Gln Val Glu Ser Arg Arg Arg Gln Ala Ala Ala
   465                 470                 475                 480
   Ala
<210> 15
   <211> 190
   <212> PRT
   <213> Amycolatopsis sulphurea
<400> 15
   Met Arg Leu Ser Pro Glu Thr Phe Ala Arg Ala Ala Leu Lys Leu Leu
   1               5                   10                  15
   Asn Lys Ser Gly Leu Glu Gly Val Ser Leu Arg Lys Leu Gly Asp Glu
               20                  25                  30
   Leu Gly Val Gln Gly Pro Ala Leu Tyr Ala His Phe Lys Asn Lys Gln
           35                  40                  45
   Glu Leu Leu Asp Leu Met Ala Glu Ile Met Leu Asp Glu Ala Leu Ala
       50                  55                  60
   Pro Leu Asp Ala Met Thr Glu Val Ala Asp Trp His Trp Trp Leu Ala
   65                  70                  75                  80
   Glu Arg Ala Arg Thr Ile Arg Arg Thr Leu Leu Ser Tyr Arg Asp Gly
                   85                  90                  95
   Ala Leu Leu His Ala Gly Ser Arg Pro Thr Ala Asp Gly Ala Glu Ala
               100                 105                 110
   Ile Pro Ala Leu Leu Arg Pro Leu Arg Glu Ala Gly Phe Ser Asp Lys
           115                 120                 125
   Glu Ala Leu Thr Val Ile Ile Thr Ile Gly Arg Tyr Thr Leu Gly Cys
       130                 135                 140
   Val Ile Asp Glu Gln Arg Pro Gly Glu Pro Ala Pro Gln Pro Gly Pro
   145                 150                 155                 160
   Gly Ala Asp Asp Thr Phe Glu Phe Gly Leu Gln Ala Leu Leu Ala Gly
                   165                 170                 175
   Leu Arg Ala Arg Leu Pro Glu Arg Val Pro Asp Ser Ala Gly
   180 185 190
<210> 16
   <211> 404
   <212> PRT
   <213> Amycolatopsis sulphurea
<400> 16
   Met Thr Asp Ile Arg Thr Asp Phe Cys Val Val Gly Gly Gly Pro Ala
   1 5 10 15
   Gly Leu Thr Leu Ala Leu Leu Leu Ala Arg Ser Gly Val Arg Val Val
   20 25 30
   Val Val Glu Arg Ser Arg Ser Phe Asp Arg Glu Tyr Arg Gly Glu Ile
   35 40 45
   Leu Gln Pro Gly Gly Gln Ala Leu Leu Ala Glu Leu Gly Val Leu Thr
   50 55 60
   Pro Ala Arg Glu His Gly Ala His Glu His His Arg Phe Leu Leu Glu
   65 70 75 80
   Glu His Gly Lys Val Leu Ile Asn Gly Asp Tyr Arg Arg Leu Pro Gly
   85 90 95
   Pro Phe Asn Cys Leu Leu Ser Ile Pro Gln Arg His Leu Leu Arg Glu
   100 105 110
   Leu Leu Ala Gln Cys His Glu His Ala Gly Phe Gln Tyr Leu Ser Gly
   115 120 125
   Thr Lys Val Thr Gly Leu Val Glu Asp Gly Gly Arg Val Arg Gly Val
   130 135 140
   Val Cys Gly Asp Asp Gln Val Val Leu Ala His Cys Val Ile Gly Ala
   145 150 155 160
   Asp Gly Arg Tyr Ser Lys Val Arg Gln Leu Ala Gly Ile Pro Ala Asp
   165 170 175
   Arg Val Glu Gly Phe Arg Gln Asp Val Leu Trp Phe Lys Leu Ser Ala
   180 185 190
   Asp Gly Glu Leu Pro Ser Glu Val Arg Val Phe Arg Ala Gly Gly Asn
   195 200 205
   Pro Val Leu Ala Tyr Thr Ser Val Arg Asp Arg Val Gln Phe Gly Trp
   210 215 220
   Thr Leu Pro His Lys Gly Tyr Gln Leu Leu Ala Gln Gln Gly Leu Ala
   225 230 235 240
   His Ile Lys Glu Gln Leu Arg Ala Ala Val Pro Gly Tyr Ala Asp Arg
   245 250 255
   Ile Asp Glu Glu Ile Thr Ser Phe Arg Asp Leu Ser Leu Leu Asp Val
   260 265 270
   Phe Ser Gly Gly Ala Arg Gln Trp Val Arg Asp Gly Leu Leu Leu Ile
   275 280 285
   Gly Asp Ser Ala His Thr His Gly Pro Ile Gly Ala Gln Gly Ile Asn
   290 295 300
   Leu Ala Ile Gln Asp Ala Val Ala Ala His Pro Leu Leu Leu Glu Ser
   305 310 315 320
   Leu Arg Ala Asn Asp Ser Ser Gly Ala Met Leu Gly Arg Phe Val Thr
   325 330 335
   Gly Arg Lys Arg Asp Ile Asp Arg Met Asn Arg Ile Gln Ala Val Gln
   340 345 350
   Gly Lys Ala Met Leu Ser Ala Gly Arg Val Ser Ser Val Val Arg Pro
   355 360 365
   Arg Leu Ala Met Val Val Ala Arg Thr Pro Ile Tyr Arg Ala Met Leu
   370 375 380
   Arg Gln Ile Ala Phe Gly Asn Thr Gly Ile Arg Ile Arg Ala Glu Leu
   385 390 395 400
   Phe Ala Arg Arg
<210> 17
   <211> 96
   <212> PRT
   <213> Amycolatopsis sulphurea
<400> 17
   Met Phe Thr Phe Ile Asn Arg Phe Thr Val Thr Gly Asp Ala Thr Glu
   1 5 10 15
   Phe Arg Arg Leu Leu Gly Gln Ile Thr Ala His Met Thr Ala Gln Pro
   20 25 30
   Gly Phe Arg Ser His Arg Leu Tyr Gln Ser Ala Arg Asp Glu Ala Val
   35 40 45
   Phe Thr Glu Ile Ala Glu Trp Asp Ser Ala Glu Asp His Gln Arg Ala
   50 55 60
   Thr Ala Gly Lys Gly Phe Arg Glu Pro Val Gly Glu Ala Met Lys His
   65 70 75 80
   Ala Thr Ala Glu Pro Ala Pro Phe Val Leu Arg Ala Glu His Gly Ala
   85 90 95
<210> 18
   <211> 550
   <212> PRT
   <213> Amycolatopsis sulphurea
<400> 18
   Met Pro Glu Asp Ser Gly Glu Glu Pro Glu Val Leu Val Ala Gly Ala
   1 5 10 15
   Gly Pro Val Gly Leu Thr Ala Ala His Glu Leu Ala Arg Arg Gly Val
   20 25 30
   Arg Val Arg Leu Val Asp Arg Ser Ala Gly Pro Ala Thr Thr Ser Arg
   35 40 45
   Ala Leu Ala Thr His Ala Arg Thr Leu Glu Ile Trp His Gln Met Gly
   50 55 60
   Leu Leu Gly Glu Leu Leu Pro Arg Gly Arg Arg Val Glu His Phe Thr
   65 70 75 80
   Leu His Leu Lys Gly Lys Thr Leu Met Cys Phe Asp Thr Asn Tyr Asp
   85 90 95
   Thr Met Pro Thr Arg Phe Pro Phe Ser Leu Met Val Asp Gln Val Val
   100 105 110
   Thr Glu Glu Val Leu Arg Arg Gln Val Arg Ala Leu Gly Val Thr Val
   115 120 125
   Glu Trp Gly Val Glu Leu Thr Trp Phe Asp Gln Glu Pro Asp Gly Val
   130 135 140
   Leu Ala Glu Leu Arg His Ala Asp Gly Thr Val Glu Gln Val Thr Ala
   145                 150                 155                 160
   Ala Trp Leu Val Gly Ala Asp Gly Ala Arg Ser Thr Val Arg Lys Arg
                   165                 170                 175
   Leu Asp Leu Arg Leu Gln Gly Asp Ser Thr Gln Thr Trp Leu Asn Ala
               180                 185                 190
   Asp Val Val Leu Asp Thr Asp Leu Ala Gly Asp Ser Asn His Leu Leu
           195                 200                 205
   His Thr Gly Arg Gly Thr Leu Leu Leu Val Pro Phe Pro Glu Pro Gly
       210                 215                 220
   Lys Trp Arg Val Val Asp Thr Glu Asp Thr Asp His Ala Asp Asp Ala
   225                 230                 235                 240
   Arg Ile Val Arg Ala Arg Leu Ala Asp Lys Leu Thr Arg Ala Leu Gly
                   245                 250                 255
   Arg Pro Ile Glu Val Pro Glu Pro Ser Trp Ile Ser Val Phe Thr Val
               260                 265                 270
   Gln Gln Arg Met Ile Asp Arg Met Arg Ala Gly Arg Cys Phe Val Ala
           275                 280                 285
   Gly Asp Ala Ala His Val His Ser Pro Ala Ser Gly Gln Gly Met Asn
       290                 295                 300
   Thr Gly Ile Gln Asp Ala Tyr Asn Leu Ala Trp Lys Leu Ala Asp Val
   305                 310                 315                 320
   Val Arg Gly His Ala Lys Glu Ser Leu Leu Asp Ser Tyr Gly Ala Glu
                   325                 330                 335
   Arg Val Pro Ile Gly Glu Thr Leu Leu Arg Thr Thr Arg Thr Ala Thr
               340                 345                 350
   Ala Leu Val Ser Leu Arg Asn Thr Val Ala Pro Leu Val Met Pro Ala
           355                 360                 365
   Gly Thr Arg Leu Leu Gly Ala Leu Lys Pro Leu Lys Arg Arg Ile Glu
       370                 375                 380
   Arg Thr Met Ile Arg Gly Phe Cys Gly Leu Thr Leu Asn Tyr Thr His
   385                 390                 395                 400
   Ser Pro Leu Ser Leu Ala Cys Ala Thr Pro Asp Gly Ile Gln Pro Gly
                   405                 410                 415
   His Arg Val Gly Cys Ser Val Asp Arg Ala Arg Thr Ser Pro Gly Trp
               420                 425                 430
   Gln Gly Leu Val Thr Glu Leu Thr Asp Pro Arg Trp Thr Leu Leu Ala
           435                 440                 445
   Phe Ala Asp Ser Gln Glu Gln Arg Gln Ile Ala Ala Gln Val Glu Arg
       450                 455                 460
   Arg Tyr Gly Lys Ala Val Ser Val Arg Val Val Ala Glu Ala Ala Thr
   465                 470                 475                 480
   Ser Glu Arg Val Leu Ala Asp Pro Gly Asp Asp Leu Ala Arg Asp Phe
                   485                 490                 495
   Ala Met Arg Ala Gly Tyr Phe Val Leu Ile Arg Pro Asp Gly His Leu
               500                 505                 510
   Ala Ala Lys Gly Arg Leu Ser Asp Asp Leu Asp Gly Ala Phe Gly Ala
           515                 520                 525
   Leu Gly Leu Val Pro Ala Asp Ala Gly Gly Asp Pro Ala His His Leu
       530                 535                 540
   Asp Ser Glu Gly Ser Arg
   545                 550
<210> 19
   <211> 256
   <212> PRT
   <213> Amycolatopsis sulphurea
<400> 19
   Met Arg Ile Ile Asp Leu Ser Ala Thr Met Asp Ala Ala Asp Arg Trp
   1               5                   10                  15
   Glu Ala Asn Pro Val Thr His Glu Val Leu Thr Ala Ala Glu Gly Ala
               20                  25                  30
   Gln His Met Ala Ala Glu Met Lys Glu His Phe Gly Ile Asp Phe Asp
           35                  40                  45
   Pro Ser Val Leu Pro Gly Gly Glu Leu Leu Thr Leu Asp Thr Leu Thr
       50                  55                  60
   Leu Thr Thr His Thr Gly Thr His Val Asp Ala Pro Ser His Tyr Gly
   65 70 75 80
   Thr Pro Arg Asp Gly Val Ala Arg His Ile Asp Gln Met Pro Leu Glu
   85 90 95
   Trp Phe Leu Arg Pro Gly Val Val Leu Asp Leu Thr Gly Glu Pro Val
   100 105 110
   Gly Ala Ala Gly Ala Asp Arg Leu Arg Glu Glu Phe Glu Arg Ile Gly
   115 120 125
   Tyr Thr Pro Lys Pro Leu Asp Ile Val Leu Leu Asn Thr Gly Ala Asp
   130 135 140
   Ala Leu Ala Gly Ser Pro Lys Tyr Phe Thr Asp Phe Thr Gly Leu Asp
   145 150 155 160
   Gly Lys Ala Thr Glu Leu Leu Leu Asp Leu Gly Val Arg Val Ile Gly
   165 170 175
   Thr Asp Ala Phe Ser Leu Asp Ala Pro Phe Gly His Met Ile Ala Glu
   180 185 190
   Tyr Arg Arg Thr Gly Asp Arg Ser Val Leu Trp Pro Ala His Phe Ala
   195 200 205
   Gly Arg Asp Arg Glu Tyr Cys Gln Ile Glu Gly Leu Thr Asn Leu Ala
   210 215 220
   Ala Leu Pro Ser Pro Thr Gly Phe Ser Val Ser Cys Leu Pro Val Lys
   225 230 235 240
   Ile Ala Gly Ala Gly Ala Gly Trp Thr Arg Ala Val Ala Leu Leu Asp
   245 250 255
<210> 20
   <211> 258
   <212> PRT
   <213> Amycolatopsis sulphurea
<400> 20
   Met Lys Phe Asn Leu Leu Gly Pro Met Glu Val Leu Cys Ala Asp Gly
   1 5 10 15
   Thr Val Thr Pro Ser Ala Ala Lys Met Arg Trp Ile Leu Ala Leu Leu
   20 25 30
   Leu Leu His Gly Asn Arg Val Val Asp Gln Ala Ser Met Ile Asp Glu
           35                  40                  45
   Leu Trp Gly Asp His Pro Pro Arg Ser Ala Val Thr Thr Thr Gln Thr
       50                  55                  60
   Tyr Val Tyr Gln Leu Arg Lys Lys Tyr Asp Tyr Tyr Ala Gln Arg Glu
   65                  70                  75                  80
   Gly Arg Lys Ser Phe Ile Val Thr Arg Ala Pro Gly Tyr Leu Leu Gln
                   85                  90                  95
   Leu Asp Asp Asp Gln Leu Asp Val Arg Arg Phe Gln Arg Leu Ser Ala
               100                 105                 110
   Glu Gly Ser Ala Leu Phe Ser Ala Gly His Ala Glu Arg Ala Asp Glu
           115                 120                 125
   Val Leu Arg Gln Ala Leu Arg Leu Trp Arg Gly Pro Ala Leu Ala Gly
       130                 135                 140
   Ile Ala Pro Gly Arg Met Leu Gln Ala His Val Ala Tyr Leu Glu Glu
   145                 150                 155                 160
   Ala Arg Leu Arg Thr Val Gln Val Arg Ile Leu Ala Asp Ala Ala Leu
                   165                 170                 175
   Gly Arg His Arg Asp Leu Ile Pro Glu Leu Arg Ser Leu Val Ile Glu
               180                 185                 190
   His Pro Leu Asp Glu Trp Phe His Gln Gln Leu Ile Thr Ala Leu Ala
           195                 200                 205
   Glu Ala Gly Arg Arg Gly Asp Ala Leu His Ala Cys Arg Val Leu His
       210                 215                 220
   Arg Thr Leu Ala Asp Glu Leu Gly Val Ala Pro Ser Glu Pro Leu Arg
   225                 230                 235                 240
   Lys Leu Gln Gln Asp Leu Leu Thr Gly His Val Arg Arg Ala Pro Ala
                   245                 250                 255
   His Val
<210> 21
   <211> 212
   <212> PRT
   <213> Amycolatopsis sulphurea
<400> 21
   Met Ile Thr Asp Arg Val Arg Ile Leu Ile Val Glu Glu Ser Ser Val
   1               5                   10                  15
   Phe Arg Val Gly Leu Leu Ser Leu Ile Gln Gly Ala Asp Asp Leu Ala
               20                  25                  30
   Ala Ile Asp Ala Val Ala Thr Ile Gly Glu Ala Leu Asp Ser Val Ser
           35                  40                  45
   Arg Gly Ile Val Asp Val Ile Leu Tyr Gly Val Asp Gly Trp Gly Pro
       50                  55                  60
   Glu Val Glu Ser Gly Leu Leu Glu Leu Ser Ala Ala Ala Pro Gly Lys
   65                  70                  75                  80
   Pro Val Val Ile Leu Ser Gln Glu Asn Arg Phe Gly Phe Ile Gln Glu
                   85                  90                  95
   Phe Leu Gly Ile Gly Val Arg Gly Tyr Leu Pro Lys Asn Val Ser Asp
               100                 105                 110
   Met Tyr Leu Leu Ser Val Val Arg Glu Val Ser Arg Asp Glu Arg Cys
           115                 120                 125
   Val Phe Leu Ser Val Pro Ser Val Asp Val Arg Ser Leu Ser Ser Asn
       130                 135                 140
   Leu Arg Thr Pro Leu Ser Arg Arg Glu His Glu Ile Met Ser Leu Val
   145                 150                 155                 160
   Ala Arg Gly Met Thr Asn Ala Gln Ile Gly Asn Cys Leu Ala Ile Thr
                   165                 170                 175
   Gln Gly Thr Val Lys Arg His Leu Arg Asn Ile Phe Val Lys Leu Asn
               180                 185                 190
   Ala Val Ser Arg Leu Asp Ala Val Asn Lys Ala Gln Thr Ala Ala Pro
           195                 200                 205
   Leu Val Pro Ala
       210
<210> 22
   <211> 806
   <212> PRT
   <213> Amycolatopsis sulphurea
<400> 22
   Met Pro Glu Asp Ser Gly Glu Glu Pro Glu Val Leu Val Ala Gly Ala
   1               5                   10                  15
   Gly Pro Val Gly Leu Thr Ala Ala His Glu Leu Ala Arg Arg Gly Val
               20                  25                  30
   Arg Val Arg Leu Val Asp Arg Ser Ala Gly Pro Ala Thr Thr Ser Arg
           35                  40                  45
   Ala Leu Ala Thr His Ala Arg Thr Leu Glu Ile Trp His Gln Met Gly
       50                  55                  60
   Leu Leu Gly Glu Leu Leu Pro Arg Gly Arg Arg Val Glu His Phe Thr
   65                  70                  75                  80
   Leu His Leu Lys Gly Lys Thr Leu Met Cys Phe Asp Thr Asn Tyr Asp
                   85                  90                  95
   Thr Met Pro Thr Arg Phe Pro Phe Ser Leu Met Val Asp Gln Val Val
               100                 105                 110
   Thr Glu Glu Val Leu Arg Arg Gln Val Arg Ala Leu Gly Val Thr Val
           115                 120                 125
   Glu Trp Gly Val Glu Leu Thr Trp Phe Asp Gln Glu Pro Asp Gly Val
       130                 135                 140
   Leu Ala Glu Leu Arg His Ala Asp Gly Thr Val Glu Gln Val Thr Ala
   145                 150                 155                 160
   Ala Trp Leu Val Gly Ala Asp Gly Ala Arg Ser Thr Val Arg Lys Arg
                   165                 170                 175
   Leu Asp Leu Arg Leu Gln Gly Asp Ser Thr Gln Thr Trp Leu Asn Ala
               180                 185                 190
   Asp Val Val Leu Asp Thr Asp Leu Ala Gly Asp Ser Asn His Leu Leu
           195                 200                 205
   His Thr Gly Arg Gly Thr Leu Leu Leu Val Pro Phe Pro Glu Pro Gly
       210                 215                 220
   Lys Trp Arg Val Val Asp Thr Glu Asp Thr Asp His Ala Asp Asp Ala
   225                 230                 235                 240
   Arg Ile Val Arg Ala Arg Leu Ala Asp Lys Leu Thr Arg Ala Leu Gly
                   245                 250                 255
   Arg Pro Ile Glu Val Pro Glu Pro Ser Trp Ile Ser Val Phe Thr Val
               260                 265                 270
   Gln Gln Arg Met Ile Asp Arg Met Arg Ala Gly Arg Cys Phe Val Ala
           275                 280                 285
   Gly Asp Ala Ala His Val His Ser Pro Ala Ser Gly Gln Gly Met Asn
       290                 295                 300
   Thr Gly Ile Gln Asp Ala Tyr Asn Leu Ala Trp Lys Leu Ala Asp Val
   305                 310                 315                 320
   Val Arg Gly His Ala Lys Glu Ser Leu Leu Asp Ser Tyr Gly Ala Glu
                   325                 330                 335
   Arg Val Pro Ile Gly Glu Thr Leu Leu Arg Thr Thr Arg Thr Ala Thr
               340                 345                 350
   Ala Leu Val Ser Leu Arg Asn Thr Val Ala Pro Leu Val Met Pro Ala
           355                 360                 365
   Gly Thr Arg Leu Leu Gly Ala Leu Lys Pro Leu Lys Arg Arg Ile Glu
       370                 375                 380
   Arg Thr Met Ile Arg Gly Phe Cys Gly Leu Thr Leu Asn Tyr Thr His
   385                 390                 395                 400
   Ser Pro Leu Ser Leu Ala Cys Ala Thr Pro Asp Gly Ile Gln Pro Gly
                   405                 410                 415
   His Arg Val Gly Cys Ser Val Asp Arg Ala Arg Thr Ser Pro Gly Trp
               420                 425                 430
   Gln Gly Leu Val Thr Glu Leu Thr Asp Pro Arg Trp Thr Leu Leu Ala
           435                 440                 445
   Phe Ala Asp Ser Gln Glu Gln Arg Gln Ile Ala Ala Gln Val Glu Arg
       450                 455                 460
   Arg Tyr Gly Lys Ala Val Ser Val Arg Val Val Ala Glu Ala Ala Thr
   465                 470                 475                 480
   Ser Glu Arg Val Leu Ala Asp Pro Gly Asp Asp Leu Ala Arg Asp Phe
   485 490 495
   Ala Met Arg Ala Gly Tyr Phe Val Leu Ile Arg Pro Asp Gly His Leu
   500 505 510
   Ala Ala Lys Gly Arg Leu Ser Asp Asp Leu Asp Gly Ala Phe Gly Ala
   515 520 525
   Leu Gly Leu Val Pro Ala Asp Ala Gly Gly Asp Pro Ala His His Leu
   530 535 540
   Asp Ser Glu Gly Ser Arg Met Arg Ile Ile Asp Leu Ser Ala Thr Met
   545 550 555 560
   Asp Ala Ala Asp Arg Trp Glu Ala Asn Pro Val Thr His Glu Val Leu
   565 570 575
   Thr Ala Ala Glu Gly Ala Gln His Met Ala Ala Glu Met Lys Glu His
   580 585 590
   Phe Gly Ile Asp Phe Asp Pro Ser Val Leu Pro Gly Gly Glu Leu Leu
   595 600 605
   Thr Leu Asp Thr Leu Thr Leu Thr Thr His Thr Gly Thr His Val Asp
   610 615 620
   Ala Pro Ser His Tyr Gly Thr Pro Arg Asp Gly Val Ala Arg His Ile
   625 630 635 640
   Asp Gln Met Pro Leu Glu Trp Phe Leu Arg Pro Gly Val Val Leu Asp
   645 650 655
   Leu Thr Gly Glu Pro Val Gly Ala Ala Gly Ala Asp Arg Leu Arg Glu
   660 665 670
   Glu Phe Glu Arg Ile Gly Tyr Thr Pro Lys Pro Leu Asp Ile Val Leu
   675 680 685
   Leu Asn Thr Gly Ala Asp Ala Leu Ala Gly Ser Pro Lys Tyr Phe Thr
   690 695 700
   Asp Phe Thr Gly Leu Asp Gly Lys Ala Thr Glu Leu Leu Leu Asp Leu
   705 710 715 720
   Gly Val Arg Val Ile Gly Thr Asp Ala Phe Ser Leu Asp Ala Pro Phe
   725 730 735
   Gly His Met Ile Ala Glu Tyr Arg Arg Thr Gly Asp Arg Ser Val Leu
   740 745 750
   Trp Pro Ala His Phe Ala Gly Arg Asp Arg Glu Tyr Cys Gln Ile Glu
   755 760 765
   Gly Leu Thr Asn Leu Ala Ala Leu Pro Ser Pro Thr Gly Phe Ser Val
   770 775 780
   Ser Cys Leu Pro Val Lys Ile Ala Gly Ala Gly Ala Gly Trp Thr Arg
   785 790 795 800
   Ala Val Ala Leu Leu Asp
   805
<210> 23
   <211> 643
   <212> PRT
   <213> Amycolatopsis sulphurea
<400> 23
   Met Ala Thr Pro Glu Thr Thr Val Ala Gln Asp Ser Thr Val Ser Gly
   1 5 10 15
   Asp Pro Leu Thr Gly Ile Ala Gly Phe Ile Gly Gly Pro Arg Val Asp
   20 25 30
   Asp Leu Leu Arg Ser Ala Ala Glu Arg Ala Pro Glu His Val Ala Ile
   35 40 45
   Arg Ala Ala Ala Cys Glu Leu Ser Tyr Ala Ala Leu Asp Glu Arg Ala
   50 55 60
   Gly Arg Leu Ala Arg Ala Val His Ala Gln Leu Ala Glu Pro Gly Gln
   65 70 75 80
   Val Val Ala Leu Ala Gly Val Leu Asp Pro Ala Phe Ala Val Ser Phe
   85 90 95
   Phe Gly Ile Ala Arg Ser Gly Ala Val Pro Ala Leu Ile Asn Pro Leu
   100 105 110
   Leu Leu Ala Asp Gly Leu Val His Val Leu Gly Thr Ser Gly Ala Val
   115 120 125
   Thr Ala Ile Val Pro Pro Glu Val His Arg Arg Leu Val Pro Val Leu
   130 135 140
   Asp Arg Leu Pro Glu Leu Arg His Leu Val Leu Thr His Arg Asp Asp
   145                 150                 155                 160
   Gln Thr Glu Pro Ser Gly Pro Pro Thr Leu Asp Glu Val Leu Ala Ser
                   165                 170                 175
   Ala Pro Asp Gly Val Leu Pro Ala Thr Thr Cys Asp Glu Asn Ser Val
               180                 185                 190
   Ala Cys Leu Gln Phe Thr Ser Gly Thr Thr Gly Ala Ala Lys Ala Val
           195                 200                 205
   Arg Leu Ser His Arg Asn Ile Thr Val Asn Ala Ala Gln Ser Gly His
       210                 215                 220
   Ala His Gly Ile Thr Pro Ser Ser Val Leu Phe Asn Tyr Leu Pro Thr
   225                 230                 235                 240
   Phe His Leu Met His Leu Thr Met Ala Val Thr Phe Ala Ala Thr Leu
                   245                 250                 255
   Val Leu His Val Gly Asp Asp Val Ala Gln Ala Val Asp Ala Ala Asp
               260                 265                 270
   Asn Glu Lys Ala Thr His Phe Tyr Ser Leu Pro Met Arg Leu Ser Arg
           275                 280                 285
   Leu Ala Val His Pro Arg Leu Ser Thr Leu Ala Ala Asp Ala Leu Gln
       290                 295                 300
   Val Ile Leu Cys Gly Gly Ser Ala Leu Pro Leu Pro Ser Thr Arg Ala
   305                 310                 315                 320
   Leu Thr Gly Cys Phe Gly Val Pro Val Val Gln Gly Tyr Gly Leu Gln
                   325                 330                 335
   Glu Thr Ser Pro Ser Thr His Phe Asp Ser Leu Ser Cys Pro Lys Thr
               340                 345                 350
   Gly Ser Ser Gly Arg Pro Val Ala Gly Thr Gly Cys Arg Ile Val Asp
           355                 360                 365
   Val Asp Ser Arg Ala Val Leu Pro Val Gly Glu Lys Gly Glu Ile Gln
       370                 375                 380
   Val Arg Gly Pro Gln Leu Met Leu Gly Tyr Leu Gly Arg Glu Pro Gly
   385                 390                 395                 400
   Gln Asp Val Asp Pro Asp Gly Trp Phe Ser Thr Gly Asp Val Gly Tyr
   405 410 415
   Val Asp Ala Glu Gly Val Leu Phe Val Val Asp Arg Ile Lys Asp Val
   420 425 430
   Phe Lys Cys Asp Asn Trp Leu Val Ser Pro Thr Glu Ile Glu Arg Val
   435 440 445
   Val Leu Ser His Pro Glu Val Ala Asp Cys Val Val Leu Asp Tyr Pro
   450 455 460
   Asp Asp Phe Ser Gly Ser Val Ala Tyr Gly Leu Val Val Pro Lys Gly
   465 470 475 480
   Ala Gly Leu Asn Pro Ala Gln Leu Ala Glu Phe Val Ala Glu Arg Leu
   485 490 495
   Pro Tyr Tyr Ala His Leu Arg His Val Glu Leu Thr Asp Arg Ile Pro
   500 505 510
   Arg Ser Pro Asn Gly Lys Leu Gln Arg Arg Ala Leu Arg Glu Gln Ile
   515 520 525
   His Ala Arg Asn Ala Asp Gly Ala Ser Glu Ile Ala Arg Gln Asn Arg
   530 535 540
   Ser Lys Thr Val Phe Thr Phe Ile Asn Arg Phe Thr Val Thr Gly Asp
   545 550 555 560
   Ala Thr Glu Phe Arg Arg Leu Leu Gly Gln Ile Thr Ala His Met Thr
   565 570 575
   Ala Gln Pro Gly Phe Arg Ser His Arg Leu Tyr Gln Ser Ala Arg Asp
   580 585 590
   Glu Ala Val Phe Thr Glu Ile Ala Glu Trp Asp Ser Ala Glu Asp His
   595 600 605
   Gln Arg Ala Thr Ala Gly Lys Gly Phe Arg Glu Pro Val Gly Glu Ala
   610 615 620
   Met Lys His Ala Thr Ala Glu Pro Ala Pro Phe Val Leu Arg Ala Glu
   625 630 635 640
   His Gly Ala
<210> 24
   <211> 612
   <212> PRT
   <213> Streptomyces rimosus
<400> 24
   Met Cys Gly Ile Ala Gly Trp Ile Asp Phe Glu Arg Asn Leu Ala Gln
   1               5                   10                  15
   Glu Arg Ala Thr Ala Trp Ala Met Thr Asp Thr Met Ala Cys Arg Gly
               20                  25                  30
   Pro Asp Asp Ala Gly Leu Trp Thr Gly Gly His Ala Ala Leu Gly His
           35                  40                  45
   Arg Arg Leu Ala Val Ile Asp Pro Ala His Gly Arg Gln Pro Met His
       50                  55                  60
   Ser Thr Leu Pro Asp Gly Thr Ser His Val Ile Thr Phe Ser Gly Glu
   65                  70                  75                  80
   Ile Tyr Asn Phe Arg Glu Leu Arg Val Glu Leu Glu Ser Gln Gly His
                   85                  90                  95
   Arg Phe Arg Thr His Cys Asp Thr Glu Val Val Leu His Gly Tyr Thr
               100                 105                 110
   Arg Trp Gly Arg Glu Leu Val Asp Arg Leu Asn Gly Met Tyr Ala Phe
           115                 120                 125
   Ala Val Trp Asp Glu Ala Arg Gln Glu Leu Leu Leu Val Arg Asp Arg
       130                 135                 140
   Met Gly Val Lys Pro Leu Tyr Tyr His Pro Thr Ala Thr Gly Val Leu
   145                 150                 155                 160
   Phe Gly Ser Glu Pro Lys Ala Val Leu Ala His Pro Ser Leu Arg Arg
                   165                 170                 175
   Arg Val Thr Ala Glu Gly Leu Cys Glu Val Leu Asp Met Val Lys Thr
               180                 185                 190
   Pro Gly Arg Thr Val Phe Ser Gly Met Arg Glu Val Leu Pro Gly Glu
           195                 200                 205
   Met Val Thr Val Gly Arg Ser Gly Val Ala Arg Arg Arg Tyr Trp Thr
   210 215 220
   Leu Gln Ala Arg Glu His Thr Asp Asp Leu Glu Thr Thr Ile Ala Thr
   225 230 235 240
   Val Arg Gly Leu Leu Thr Asp Arg Val Arg Arg Gln Leu Val Ser Asp
   245 250 255
   Val Pro Leu Cys Thr Leu Leu Ser Gly Gly Leu Asp Ser Ser Ala Val
   260 265 270
   Thr Ala Leu Ala Ala Arg Ala Gly Asp Gly Pro Val Arg Thr Phe Ser
   275 280 285
   Val Asp Phe Ser Gly Ala Gly Thr Arg Phe Gln Pro Asp Ala Val Arg
   290 295 300
   Gly Asn Thr Asp Ala Pro Tyr Val Gln Glu Met Val Arg His Val Ala
   305 310 315 320
   Ala Asp His Thr Glu Val Val Leu Asp Ser Ala Asp Leu Ala Ala Pro
   325 330 335
   Glu Val Arg Ala Ala Val Leu Gly Ala Thr Asp Leu Pro Pro Ala Phe
   340 345 350
   Trp Gly Asp Met Trp Pro Ser Leu Tyr Leu Phe Phe Arg Gln Val Arg
   355 360 365
   Gln His Cys Thr Val Ala Leu Ser Gly Glu Ala Ala Asp Glu Leu Phe
   370 375 380
   Gly Gly Tyr Arg Trp Phe His Arg Thr Ala Ala Ile Asp Ala Gly Thr
   385 390 395 400
   Phe Pro Trp Leu Thr Ala Gly Ser Ala Arg Tyr Phe Gly Gly Arg Gly
   405 410 415
   Leu Phe Asp Arg Lys Leu Leu Asp Lys Leu Asp Leu Pro Gly Tyr Gln
   420 425 430
   Arg Asp Arg Tyr Ala Glu Ala Arg Lys Glu Val Pro Val Leu Pro Gly
   435 440 445
   Glu Asp Ala Arg Glu Ala Glu Leu Arg Arg Val Thr Tyr Leu Asn Leu
   450 455 460
   Thr Arg Phe Val Gln Thr Leu Leu Asp Arg Lys Asp Arg Met Ser Met
   465 470 475 480
   Ala Thr Gly Leu Glu Val Arg Val Pro Phe Cys Asp His Arg Leu Val
   485 490 495
   Asp Tyr Val Phe Asn Val Pro Trp Ala Met Lys Ser Phe Asp Gly Arg
   500 505 510
   Glu Lys Ser Leu Leu Arg Ala Ala Val Arg Asp Leu Leu Pro Glu Ser
   515 520 525
   Val Val Thr Arg Val Lys Thr Pro Tyr Pro Ala Thr Gln Asp Pro Val
   530 535 540
   Tyr Glu Arg Leu Leu Arg Asp Glu Leu Ala Ala Leu Leu Ala Asp Ser
   545 550 555 560
   Gln Ala Pro Val Arg Glu Leu Leu Asp Leu Gly Arg Ala Arg Asp Leu
   565 570 575
   Leu Arg Arg Pro Val Gly Ala Val Ser Gln Pro Tyr Asp Arg Gly Ser
   580 585 590
   Leu Glu Leu Val Leu Trp Met Asn Thr Trp Leu Ala Glu Tyr Gly Val
   595 600 605
   Ser Leu Glu Leu
   610
<210> 25
   <211> 340
   <212> PRT
   <213> Streptomyces rimosus
<400> 25
   Met Thr Ala Asp Thr Lys Ala Thr Gly Ala Pro Ala Ala Arg Ala Pro
   1 5 10 15
   Arg Pro Val Ala Leu Leu Leu Pro Gly Gln Gly Ser Gln Tyr Arg Arg
   20 25 30
   Met Ala Ala Gly Leu Tyr Ala Ala Glu Pro Val Phe Ala Glu Ala Val
   35 40 45
   Asp Glu Val Leu Gly Ala Met Gly Ala Glu Gly Ala Arg Met His Ala
   50 55 60
   Asp Trp Leu Ala Glu Arg Pro Glu Leu Pro Val Asp His Val Leu Arg
   65                  70                  75                  80
   Ala Gln Pro Leu Leu Phe Ala Val Asp Tyr Ala Leu Gly Arg Leu Val
                   85                  90                  95
   Thr Ser Trp Gly Ile Arg Pro Val Ala Leu Leu Gly His Ser Ile Gly
               100                 105                 110
   Glu Met Ala Ala Ala Thr Leu Ala Gly Val Phe Thr Val Arg Asp Ala
           115                 120                 125
   Ala Arg Val Val Leu Asp Arg Val Thr Arg Leu Thr Ala Ala Pro Pro
       130                 135                 140
   Gly Gly Met Leu Ala Val Ala Glu Ser Ala Ala Ser Leu Glu Pro Phe
   145                 150                 155                 160
   Leu Gly Asp Gly Val Val Val Gly Ala Val Asn Ala Pro Arg Gln Thr
                   165                 170                 175
   Val Leu Gly Gly Pro Glu Asp Ala Leu Arg Ala Val Gly Glu Thr Leu
               180                 185                 190
   Arg Ala Arg Gly Ile Thr Ala Gln Arg Val Pro Ala Leu Ser Pro Phe
           195                 200                 205
   His Ser Pro Ala Ile Ala Pro His Ala Arg Gly Ala Glu Ala Val Leu
       210                 215                 220
   Ala Thr Val Glu Arg Arg Pro Pro Arg Thr Val Val His Ser Cys Tyr
   225                 230                 235                 240
   Thr Ala Ala Pro Leu Thr Ala Gln Gln Val Ala Asp Pro Ala Tyr Trp
                   245                 250                 255
   Ala Ala His Pro Val Asp Gln Val Arg Phe Trp Pro Ala Leu Asp Gly
               260                 265                 270
   Leu Leu Ala Pro Gly Gly Leu Val Val Val Glu Ala Gly Pro Gly Arg
           275                 280                 285
   Thr Leu Ser Ser Leu Ala Leu Arg His Pro Ser Val Arg Arg Gly Asp
       290                 295                 300
   Cys Met Val Val Pro Leu Ser Pro Lys Arg Ala Asp Gly Pro Glu Asp
   305                 310                 315                 320
   Asp Arg Val Ala Leu Gly Glu Ala Val Asp Ala Leu Arg Gly Glu Gly
                   325                 330                 335
   Tyr Arg Ile Pro
               340
<210> 26
   <211> 22747
   <212> DNA
   <213> Amycolatopsis sulphurea
<400> 26
   ccgatgcgcg gtcgccccac ccggcttcca gggcggcttg aggatcgccg ggcaggctct      60
   ctgctggtga ctgggctgac cggcgacaga gggagacgcg gtgacaggac caagcgatgc     120
   ccatcgtgtc gtgattaccg ggattggggt ggtcgccccg ggcgaccgtg gtaccaagca     180
   gttctgggag ctgatcacgg cgggccgtac ggccacccgg ccgatctcgt tgttcgacgc     240
   gagttccttc cggtcgaggg tggccgccga gtgcaacttc gatccgatcg ccgccgggct     300
   gagccagcgg cagatccgga aatgggaccg caccacgcag ttctgcgtgg tggcggcaag     360
   ggaggcggtg gccgacagcg ggatgctcgg ggagcaggat cccctgcgta cgggggtggc     420
   gatcggcacc gcctgcggca tgacccagag cctggaccgc gagtacgcgg tggtcagcga     480
   cgagggcagc agctggctgg tcgacccgga ttacggggtg ccccagctct acgactactt     540
   cctgccgtcc tcgatggcca ccgagatcgc gtggctggtg gaggcggaag gaccggtggg     600
   gctggtttcc accggatgca cgtcgggggt cgacgtgatc gggcacgccg ccgacctgat     660
   ccgcgacggc gaggccgaca tcatggtcgc cggtgcctcc gaggcgccga tctcgccgat     720
   cacggtggcc tgcttcgacg cgatcaaggc caccacggca cgcaaccacg aacccgagtc     780
   ggcttccagg ccgttcgacc agacccgcag cgggttcgtg ctcggcgagg gcgcggccgt     840
   cttcgtgctg gaggagctga ggcacgccaa gcggcggggc gcgcacatct acgccgaaat     900
   cgtcgggtac gcgtcccggt gcaacgccta cagcatgacc gggttgcgtc cggacggccg     960
   cgagatggcc gacgcgatcg acggggcgtt gaaccaggcc aggatcgacc cgtcccggat    1020
   cggctacgtc aacgcccacg ggtcgtcgac caggcagaac gatcggcacg agaccgcggc    1080
   catcaagacc agcctcggag cgcacgccta tcaggtgccg gtcagctcga tcaagtcgat    1140
   ggtcgggcat tcgctcggcg cgatcggctc gctggaggtc gcggcctgcg cgctgaccat    1200
   cgagcattcg gtgatcccgc cgacggcgaa tctgcacgtg cccgacccgg agtgcgacct    1260
   ggactacgtg ccactggtgg cgcgggaaca ggaggtcgac gtggtgctca gcgtcgcgag    1320
   cgggttcggc ggtttccaga gtgccatcct gctgaccggc ccggacggca gaaccgggaa    1380
   gcgggtaacg cagcgatgag cgtggagaca gaaccggcgc cggtgcccgg ccgcagcacg    1440
   gtacggccgg tggtgaccgg gttgggcgtg atcgcgccca acggtatggg caccgaggcc 1500
   tactgggcgg cgacccttcg tggcgacagc ggactgcggc ggatcacccg gttcgacccg 1560
   gacggctacc cggcccggat cgcgggcgag gtcagcttcg acccggccgg gcggctgccc 1620
   gaccggctgt tgccgcagac cgaccacatg acccggctcg cgctgatcgc cgcggaggag 1680
   gcactcgccg acgcgggtgc cgatccgcgg aacctgcccg actacgcgac cggggtcatg 1740
   accgcggcct cgggcggcgg attcgagttc gggcaacggg aactgcagga gctgtggagc 1800
   aaaggcggct cctacgtcag cgcgtaccag tccttcgcct ggttctaccc ggtgaacacc 1860
   gggcagatct cgatccggca cgggatgcgc ggctccagcg gcacgctcgt gtccgaacag 1920
   gccggtgggc tggacgcggt cgccaaggcc cgcaggcacg tgcgcgacgg aacaccgctg 1980
   atggtgaccg gcggcatcga cggatcgctg tgcccgtggt cctggctgtg catgctgcgc 2040
   tccggccggt tgagcacggc gagtgatcct cagcgcgcct atctgccctt cgataccgag 2100
   gcttccggga tggtgccggg tgagggtggc gcgttgctgg tgatcgagga tcccgccgcg 2160
   gcacaacggc gtggcgtgga ccggatctat gggcagatcg ccggctattg cgcgacattc 2220
   gaccccggcc cgggttcacg tcgtccgccc ggcctgcgcc gggcggtcga gcaggcgctg 2280
   gccgaagcac ggctgcatcc gtccgaagtg gacgtggtgt tcgccgacgc cgccggtctg 2340
   cccgacctcg accgggccga gatcgaagtg ctggtccgga tcttcggcgc gcgggccgtc 2400
   ccggtcactg cacccaagac gatgaccggc cggctgctgg ccggtggaag ctcgctggac 2460
   ctggccacgg cgttgctctc cctgcgggac aaggtcattc cgcccaccgt ccacatagga 2520
   aagttcggtt accgggacga gatcgacctg gttcgcgaca gcccccggca ggcgccgctg 2580
   tcgactgcgc tggtgctggc ccgagggtat ggcgggttca actccgcgat ggtgcttcgc 2640
   ggtgccacct gatccccaca cgaaaggaag gcaaccatgg cagagttcac gatcgccgag 2700
   ctggtgcggc tgctgcgcga atgcgccgga gaggaggaag gcgtggacct cgacggcgag 2760
   gtcggggacc tgccgttcga cgaactcggc tacgactccc tcgcgctgtt caacaccatc 2820
   gggcggatcg agcgtgagta cactgtggac ctccccgagg acgtagtgtg gcaggcgacc 2880
   acgccgggag cgctggtcga tctggtcaac agcagtcgca cgtcgcccgc tgccgccgac 2940
   tgaaaaggct gtgaagggcc ccttcacgga ctcagagtcc gtgaaggggc ccttcacgga 3000
   ctcgtctcgc ccgttcagcg ggtttctgcc agccgcttga ccagctgcag ctcggccagg 3060
   ctgtccgcac tcgacgtctt gtgcagccgg tcacgcaatt cagggagctg ttcggctgcc 3120
   gacgggttca ccagcaccgt atgcgcggac gtgacggcga ctcccgcagg cgtctcggtc 3180
   agggtccacc ggccgaggtg caggtccacc ggtgcggtca cggtcagctg cttgtacgcg 3240
   attcccttgt ccggcaagca gatgcggacg aaccggctcg gctcgtccgc atcttgcgtg 3300
   cgcacgtcgt agaactgcac gtcgttctcg tcctcggtga cctcaagcgc ggtgaggcag 3360
   ggcagatgtg caggccagga cccgacgtct cgcagcgcgc ggtagacctg cccgagttcg    3420
   ctctcgatca ccagcgaatc ctcgaacgac caggtcagct ccgcgagccg ggtacggttc    3480
   tcgacccggc tcttgagggt ctccatctgc agcttgccgt gccggttcag ttcggtgacc    3540
   ccggccgcgc gatcctccgg cgtggcggcg tggaaggcgt gccgggactc caccaggcac    3600
   cggtcacccg gcagcggttt gaaggtccac gacccggtca gcccggtgat cgggccgacc    3660
   gagccctgct gctcgatgtc catccgcagc gcgctcgcgt ccaggtaccg gcgggaggtc    3720
   cagatccgga tcctgcccag cgggttgacc gcccagaact gcaccagatc ctcggtcggc    3780
   gctcgctcca cgaactcggt gtgcgcgatc cacgggtaga gcagcggcca gttcgcgacg    3840
   tcgaccagca tggcgtaggt gatctcggcg ggcgcgtcca cgacgatact ggcctgcgtc    3900
   tgctcggcct cggggtgtga catgggttct cctcgcgaat ggggtgtggg aaagagggcg    3960
   ttcaccgggc ggacccggcg aacgccctcg taggaaacga aaacggcggg ttcaggcctt    4020
   gtctgcggtg aataccgtgt gcatggcctt ctccgcggtg aaaatctgcg ggttgacgaa    4080
   accggcgtcg gtgaacatgc gctcgtaggt cttggtcgag tgtgtctcgc cttcgtcggt    4140
   catcaccagc atgatctgcg agaacaggta cggcagcggc ttgtcctccg ggcgctcgcc    4200
   ctcgacgaag gtgtgcccgg tgaccgcgat ccgcccaccc ggcttgagca cgtcgaacag    4260
   cctgcgcaac aggttcgtcg acgtgtccgc cgagaaatgg tgcagcacat tggtgatcat    4320
   cacgacgtcg tagggaccgc cgagcgggac gctgaacatg tcgcccggga ttccctcgaa    4380
   ccggtcgctg atcccgttgc gctcgtagtt cttcgcggtg atcggcagta cgttcggcca    4440
   gtccacgccc cagacccggg ccttcggctc ggccttggcc aggttgacgc cgtagtagcc    4500
   gtggctgcag gccacgtcga gcacgtcgac gctgtcgcgg tccttggccc agggcagcaa    4560
   ctgctccgcc atcagttcgg ccgcaccgtt gttgaaccag gtggtgttct ccgcgaagtc    4620
   ctcccagtag tcgaattccg gggtcagggc gtggctgtcc atcacggcgc cgcccttgcg    4680
   caccgcctcg gtgagccgct tctgcgcgtc ccattcccag tcgctggcgc ccagcttcaa    4740
   cgacggcccg aagaaggtcg ggctggagct gagcaggaac ttgtcgccgt ccaccggcaa    4800
   cgcgtacttc ccgtcgaccg tgcgcagcaa cccgatggct gccaacgagt ccagcgtgat    4860
   gcgcagtcct ctggcgtcca ccccgagcgc acgcgccagg ccgtcggcgt cgacggtacg    4920
   gtcgccgatg ccgtcgaaga tgttcagctc gatcgccgtg cgcagcagtg ccgttttcac    4980
   atagccgcgc atgatttcgt gcagcttctt cgcggtcact gtggtcggtt ccgtctcggt    5040
   catgaccgtc ctctcgtcga tccccttgat cccgcaagcc atagtgctca gggcggctag    5100
   tgcagcaata gagcggacgg ccacggatgg acggctccac gagtccgggt cgagcctgcc    5160
   tcggcaggct ggcgaaacca ccgacccggg acaagaagga gcggacatgc ggctgttgct    5220
   ggtcacttgg aatgcgccgg ctcacctgtt cgcgatggtg cccctcggct gggccgcgca 5280
   agtagccggg cacgaggttc gcgtcgcggc tccgccctcc tgtaccgagg cgatcggccg 5340
   taccggcctg accgccgtac ccgtcggcac gcagcgtccg gccgcgccgt ccgggcctcc 5400
   gccgggagcg cccagcggtc gctggccggt cgactgggcg gtgcatcccg aactgctcga 5460
   cgacagccgg cacgagcttc tgcgttcgct ggccgcgcgc cagttcgccg ccgccgagcc 5520
   gatgctggac gacttgatcg agttcgcacg ctggtggtcg ccggacgtcg tggtgtacga 5580
   cccgaccagc ctggccggcg aagtggcggc caccgtgctc ggcgtgcccg ccttcgcctg 5640
   ttcctggggc cgcgcggcgg ccgtccggat cgagcggggt ctgggctccg agccgctgct 5700
   tggctacgcg cggctgtttg aacggttcgg gtgccaggcc ccgcagggac ccgcctcctg 5760
   gttcgacccg ttccccgccg gtctgtggct ggctgaaccg gatttgccgc ggcaggcgat 5820
   gcgcttcgtg cctggaaccg gcggcgacgc cggttcgcta cccggctggc tccgggaaca 5880
   ctcggctcgg ccgaggatct gcgtcaccag cgcggaaccc ggtggtctgt tgcggccgga 5940
   agccgtacgg gcattctacc ggcacgcgct caccgtactg tccgatgtgg acgctgaggt 6000
   cgtggtgccc gcgggtccgg cggcccgcac gctgctggcc gagattccgc acaccgcgcg 6060
   gatcgtcgac ccggtggccg cgcatctgct cgtcccggcc tgccggctca ccgtgcacca 6120
   gggtgacggg ctgtccactt tggccggtct gaattcgggg gttccccagt acgtcctggc 6180
   gccacgtccg gagcaggagc tggtggggca ccagctgcac cgagccggtg cgggcggcta 6240
   ccggagcctg tccgaacccg tggacgttcc ggccgaacgt gcggtgctgg atgccttgct 6300
   ggcaccggaa agcgggggag cggcggcccg caagctgcag gaggaaacgc tggcactccc 6360
   gctcccgtcc gccgtgctcg gccggatcga gtccgccacc cgctgagcct gcgctcggcc 6420
   tgcgcgccga gcccccgacc aaggctgtga agggcccctt cacggactca gagtccaatg 6480
   ccaggaactt aaggtctggt gggtggtgtt gatcacggtt tgggtgtgat cggcgtggtg 6540
   gtgtggatag gcaacgaggc tccggctaga gctggtgatt gtcgagatca tcagcaagcc 6600
   aggagcctcg ttgtccgttc atggtgtcat gccctcgggc tggtcctcgc cggtgggtcg 6660
   gttgttgtca caggccgggc tggggttgct gtcgtgggtc gtcccgcccg cgctggttga 6720
   tgaggcgctg gccgtcgccg gccgggacga acggcggttc cgggcactgc cgtcgcggtt 6780
   gggcgtgtac ttcgtgctcg cgttgtgcct gctgcgcacc aagtccggca acgcgacgat 6840
   cagggcgatg ttctcgcagg agagtctgcc ccggttgtcc gtgctgggct ggtggccgcc 6900
   ggccagcacg gcgctgacca agctgcggga ccggatcggc gtggtgccct tccagttgtt 6960
   gttcggcgcg ctggcgcggg cggcgcctac ccggaacagg ccgtggtcgc acgcgttcgg 7020
   gctggaggtg tgtgcctggg acggcaccga ggtcgagccg gccgacaccg cggccaaccg 7080
   tgaacacttc ccgccccatc accgcacagg cgtggcccgc gggccgtcca agatccgggt 7140
   gctggtgctg ctgtcgtgtg gcagccgccg tctgctcggc gcggtcaccg gcccgctgag 7200
   ccagggcgag cccaccctgg cctatcagct gctgccccgg ctgcacgacc ggatgctgct 7260
   gctggccgac cgctgcttcc tcggctatcc actgtggacc gcggcgcggg aacggggcgc 7320
   gcatctgctg tggcgggcca agcagaacac cccgaagctg cccgtgcagc acgcgttgcc 7380
   ggacgagtcc tggctgtcca ccctccacgc cccggccgac gcccgccgct gggcgcgcaa 7440
   cgtgcgccgc aacaagcaac gcgggcaccg cccgcccacg ccccgcccga tcaacggcat 7500
   cgtcgtccgg gtggtcgaag cactgatcac cgtcaccgtc gacggcgtca cccgcaccga 7560
   gaaataccgg ctggtcacca gcctgctcga ccccgcacac gcacccgccg gccagctcgt 7620
   cgccctctac gcccgccgct ggaccgccga aaccggcatc aaagagatca aaaccacgct 7680
   gctggccaag cggcccctgc gcggccacac cccgatccgc gcccagcagg aactctgggc 7740
   caccctgatc gtctaccagg ccatccggct gctgatcagc cacgcagcgc tcacccagaa 7800
   cctcgacccg tcccggatct ccttcacctc cgcccgcgac gccgccgaac acgcgatcac 7860
   taccacaccc gccgacacat cccgacacct ccaatgggtg gcccaggact tatgccgaca 7920
   gctgatcacc gtccacaccc accaccgcgt ctacccccga gcactcaaac gcaccaccac 7980
   gcgctacccc caccgcagca aaaccccgca accgaccagc accaaagcca gctaccaggt 8040
   ccacatcctg cccacagcag aaacaacacc acccaccaca accaaaccaa caccacacca 8100
   acccagaacc gacctaagtt cctggcattg gactcagagt ccgtgaaggg gcccttcaca 8160
   gcccttcacg gttggtcgag gcgcgccacg gcctgcccgg aggccacgaa caggccgccg 8220
   ccaggagtgg gggttcccct ggcggcggcc cggacgagat cccccatgcc gggggacctc 8280
   cgcatcaggc ggccgcggcc tgcctgcgac ggctttccac ctgtacgagg agcatcgacg 8340
   ggatgagcac gatcgcggtg cacgcgacga tccaccagtg cgtgtagccg atcgcgtcgg 8400
   ccgcgccctc gtccgtctgc ggacctgagg ccagctggaa ggccaccagc accgtcgtca 8460
   gcgcggtccc gaacgagccg ccgacccgtt gcagcacatt gaagatcggg gtggcgtgcg 8520
   gcacgtcctc cttgccgatc atggccagcc cggcggccat cgccggcgtg gtggtcatcc 8580
   cgatcccgat gccgcgcacg atctggacca gcgacagcca cacgtaactg tcgtgcgggg 8640
   tgaccagcgc gagcgggatg gtgccgatga tggtgacgac caggccggcg aagatgaccc 8700
   gggcgccgcc gatcttgtcg gtcatcttgc ccgccagcgg cagcgccagc acggtgccga 8760
   tcgccgacgg cgcggtcagc agaccggcgg tgaccagatc ctcgtgccgc acctggagga 8820
   agtagatcgg cagcacgatc atcgcgccga acagcgcgat ccccatgcag aagatcacga 8880
   ccgccgccga ggagaaggcc cggtccttga acagccgcag gttcagcagc ggattcctcg 8940
   agcgcaccgc gtgcagggtg aacagcgcca gcagcagcgc cccggccacg gcggcgatca 9000
   ccgcgatggt ctcggtgcgg ccgccgttct ccccgatccc ggccacgccg taggtcagca 9060
   gcggcaggcc ggccaccagg atgatcaggc cgcgcacatc cagcgcgggc gcacccttcg 9120
   gcggcgcctt cggcagccag cgcaggccgg cgaagaagcc gagcgcgccg agcggcacgg 9180
   tgatccagaa cagccacggc cagccgaggc cctgggtcag cacgctgccc agcgtggtgc 9240
   ccaccgcggg cgccaggtag atcggcacgc tgaccgcggt cagcatccgg ccgatccgct 9300
   tcggcccggc cgcggtgacc agcaggatct gcccggccgg caccagcagg ccgcccgcga 9360
   tcccctgcag gacccggaag gtgatcagca tttccaccga ggtcgacagc gcgcacagcg 9420
   ccgagaacag ggtgaacagt gccacgctgc ccagccacag ccgggtcgcc ccgtagcgct 9480
   tggacgccca cccgctgacc gggaccgcgg tggccagcgc catcaggtac ccggtcgccg 9540
   tccactgcac cgtgcccagc ggggcgctca gcttctggct gagggtgtcc agggcgacgt 9600
   tgacgatggt cgaggtgaac atggacgtga acccgccgag gatcaggatc gacgccagtc 9660
   cccacagcgc cgggctgatc cggttctcgt cggcgccgac cgtgtccggt ctcgcgagat 9720
   tgtccttcat cgagcttcct tgcgtgagtc gtcgtggtgg tgcgatccgt gtccgcccgg 9780
   tgaccgagcg tcgacttagc accgctaagt cttagcggtg ctaatatggc ccccgagaca 9840
   ccgatacgtc aagtcggtgc acgcaacccg aggggatgcc gtgcggctga gtcccgaaac 9900
   cttcgcgcgc gccgcgctga agctgctcaa caaatccggc ctggaggggg tgagcctgcg 9960
   caagctcggc gacgagctgg gcgtgcaggg gcccgcgctg tacgcgcact tcaagaacaa 10020
   gcaggagctg ctcgacctga tggccgagat catgctcgac gaggcgctcg ccccgctgga 10080
   cgcgatgacc gaggtggccg actggcactg gtggctggcc gaacgggcca ggaccatccg 10140
   gcgcaccctg ctgtcctatc gggacggtgc gctgctgcac gcgggatccc ggccgaccgc 10200
   cgacggcgcc gaagcgatcc cggccctgct gcggccgttg cgagaggcgg gcttcagcga 10260
   caaggaagca ctcaccgtga tcatcaccat cggccgctac acgctggggt gtgtgatcga 10320
   cgagcagcgg ccgggcgagc ccgccccgca gcccggcccg ggtgccgacg acaccttcga 10380
   gttcgggctc caggcgttgc tggccggact gcgggcccgg ctgccggaac gggtacccga 10440
   cagcgccgga taactgccgg gagggttcag cggtcggtgc gcggggtcgc cttgacgaac 10500
   tcggccagcc tgcgcacccc ctcgcggatc tcctcggcgg tcagcgggct gaagccgagc 10560
   cggacgatgt gctcgccgcc gtcaccgacg tagaacatgc tcatcggcgc ccagcccacc 10620
   ccgtggtcgc gggccgagcg gtccagtgct tcgaggccgg cgacgaaggg cacctcgacg 10680
   gagaggaaga acccgccgcc cggcacgttc cagcgcacgc cgtgctcggc gaaccgctcc 10740
   ggcgggaagt gttcggcgag gctgctcagc gtcacctcca ggtgccgcac gtacaggtcg 10800
   gccagctccc ggttcgcggt gcgcaggctg aaatccgcgt ccaggagcac cccgccgacc 10860
   agggcttgcg agatgcccga ggtgccgacg gtgaacatgc tcttgacctt gccgagttcc 10920
   tcggcgagcg gcttggccgc gccggggccg ccggtcactt cctggtctgc gaccaggtag 10980
   ccgacgcgtg cgccggggaa gacggtcttg gcgaaggagc cgaggtagat caccctgcgc 11040
   tgggtgtcga gtgccttgag cgtcggcacc tgcgcaccgt cccggccgaa cagaccgtac 11100
   ggattgtcct ccagcagcag caaatcctgc tcggcggcga cttcgagcag ccggtgccgg 11160
   gtggcgatcg gcagcgagtg tcccgagggg ttggagaagt ccggggtcac gtagcaggcc 11220
   accgggcgca gaccggacgc gcgtacctcg cgggccaccg ccgcgacctg ctcgggatcg 11280
   ataccctcgg cgccctccgg aaccgcggcc atcgggatgt cgagcatctt cgcggctccc 11340
   gcgatgccga cgtagcaagg cgaaacggtc agcaggacgt cgctcggcga ggcgaacagc 11400
   ccgcgcagcg cgatcatcat cgcttcctgg cagccgtggg tcaccatgac cgcctccggc 11460
   gcgacgtgga tgtcctcgtc cacctccagc gtgcgcgcga tcatgtcccg gatcagcccg 11520
   ttgatcggcc cgtactggaa cagcgccttc ttgatccgtt cctcgggcat gccccgctca 11580
   cgcagatgct cgacgaagcg gtccaggtag tggtgcagct tggcgatgtc gaagaaacct 11640
   tcatacggcc ggccggaggc gaacgagacc gcctgcggat accggaaagc cgtctcgctg 11700
   agcaaccgca tcgagtccat ccgcggatcg gacacccccg tgtgcaggcc ggccagttcg 11760
   agcgtcacgg ccgagccggt ggtcaggtct tcccgagcct tcgtcatggt catccagcct 11820
   tctgatgggg gagcggcgac ggtggccact gtggactgct cgcctcaaga ggtggtctcg 11880
   cccggttgga acggcgagtg ccgggatccg accgacaatc gagccgtgcc ggttacggtc 11940
   ccggcatgac ggatatccgg actgacttct gcgtcgtcgg tggcggaccg gccgggctga 12000
   ccctggcgct gctgctggcc cgatccggcg tacgggtcgt cgtggtcgaa cggtcccgtt 12060
   ccttcgatcg ggaatatcgc ggcgagatcc tgcaaccggg tggccaggcc ttgctcgccg 12120
   agctgggcgt gctgactccg gcccgggagc acggagcgca cgagcatcac cgatttctct 12180
   tggaggagca cggaaaagtc ctgatcaacg gcgactaccg gcggctgccc ggcccgttca 12240
   actgcttgct cagcatcccg caacggcacc tgctgaggga actgctggcg cagtgtcacg 12300
   agcacgcggg cttccagtac ttgtccggca cgaaggtcac cggtctggtc gaggacggtg 12360
   gccgggttcg gggcgtggtc tgtggcgacg accaggtggt gctcgcgcac tgcgtgatcg 12420
   gggccgacgg acggtattcg aaggtgcggc agctcgccgg gattcccgcc gaccgggtcg 12480
   agggcttccg tcaggacgtg ttgtggttca agctgtccgc cgacggcgag ctgccgagtg 12540
   aggtgcgggt gttccgggcg ggcggcaacc cggtgctggc ctacacgtcg gtacgtgacc 12600
   gcgtgcagtt cggctggacg ttgccgcaca agggttatca gctgctggcg cagcagggcc 12660
   tcgcgcacat caaggagcag ctgcgggccg ccgtgcccgg ctacgcggac cggatcgacg 12720
   aggagatcac cagcttccgg gacctttccc tgctggacgt cttctccggg ggcgcccggc 12780
   agtgggtgcg cgacggtctg ctgctgatcg gcgacagtgc gcacacccac ggcccgatcg 12840
   gcgcgcaggg gatcaacctg gccatccagg atgctgtggc cgcacatccc ctgctgctgg 12900
   agtcgttgcg tgccaacgac tccagcgggg cgatgctcgg ccggttcgtg accgggcgca 12960
   agcgggacat cgaccggatg aaccggatcc aggccgtcca gggcaaggcg atgctgtccg 13020
   cgggccgggt ctcgtcggtg gtccggccga ggctggcgat ggtggtcgcc cgcaccccga 13080
   tctaccgcgc gatgttgcgg cagatcgcgt tcggcaacac cgggatccgg atccgcgccg 13140
   agttgttcgc ccgccgctga ccggtgatca gcccgggatg gtcgtctcga ccatgtgcag 13200
   gtaggagttc accgcgctga cccggtccac ctgcagcccg gcctgtccga tcagcccgag 13260
   caggccgtcc ttggtgtgct tcttgccgcc cacgttgagc agcagcaaca ggtccatcgc 13320
   ggtcgcgaac ttcatctccg gagtgccgtc gaccaggttc tcgatgatca ccacgcgggc 13380
   gccgggccga ccggctgcca ccgcgttgcg cagcgcgagg acggtgctct cgtcgtccca 13440
   ctccaggatg ttcttgaaca ggtagacgtc ggcctgcacc gggatctccc ggcggcagtc 13500
   gcccgccacc agctcggccc ggtccgccag ggctccgccc acgcgcagcc gctcgtcggc 13560
   gcccgccacc accgcgggca ggtcgaacag caccccgttc agcttttcgt tgcgggacag 13620
   cagggtggcc aggacgttgc cctgtccgcc cgcgatgtcg gccagccgct cggccccggt 13680
   caggtcgagc cggtcggcga tggccagggc ggagagcttg ctggactggg tcatggcctt 13740
   gtcgaagacc tcggccgact ccggccactg ctcgtgcagg tactcgaaga agtccttgcc 13800
   gtgcaagtcg tcgaagacgt tcttgccggt gcggacggcc tggtcgagat ggggccacag 13860
   ctcccaggtc cacggctcgg tgccccacag caggatgtgc ttgaggctgc gtggcgcgtc 13920
   ctcgcgcagc agccgggagg cgtcggtgtg cacgaacttg tccggctccg gttcggcgaa 13980
   cacctcgaaa caggtcagcg accgcatcag gcggcgcagg gcgcccgggt ccgcgtgcac 14040
   ggccttggcc agctcgtcca ccgtcgcggg ctggtcgtcg agaacgtcgg cgaggccgag 14100
   tttggcggca gcgcggaccg aggcggcgca cgccgcgctg agcgccagct cgcggactcg 14160
   catcaccgcg gcgggaagaa cggtacctgt catgactgct tacctttctg ctgggtgggg 14220
   gctgtggagg tctgtgaagg ggcccttcac ggacttcgtg gcggtcggag ctaggcgccg 14280
   tgttcggcgc gcagcacgaa cggagcgggc tccgcggtcg cgtgcttcat ggcctcgccg 14340
   accggctcgc ggaaaccctt gcccgcggtc gcccgctggt ggtcctcggc gctgtcccat 14400
   tcggcgatct cggtgaacac cgcttcatcc cgggccgact ggtagagccg gtgcgaccgg 14460
   aagccgggct gcgcggtcat gtgcgcggtg atctggccga gcagccgccg gaactcggtg 14520
   gcgtcgccgg tgacggtgaa ccggttgatg aaggtgaaca cggtcttgct cctgttctgt 14580
   cgggcgattt ccgatgcgcc atcggcattt ctcgcgtgga tctgttcgcg cagcgcacgg 14640
   cgttgcagct tcccgttggg ggagcgggga atccggtcgg tcagttcgac gtggcgcagg 14700
   tgcgcgtagt acggcagccg ttcggcaacg aactcggcga gctgcgccgg attgagcccg 14760
   gcgcccttgg gcaccaccag cccgtacgcg accgatccgc tgaaatcgtc ggggtagtcc 14820
   agtaccacgc agtcggccac ctcggggtga ctcagcacga cccgctcgat ctccgtcggc 14880
   gacacgagcc agttgtcgca cttgaagacg tccttgatcc ggtcgaccac gaacagcacg 14940
   ccctcggcgt cgacgtagcc gacgtccccg gtgctgaacc agccgtcggg atcgacgtcc 15000
   tggcccggct cgcggccgag gtagccgagc atcagctgcg ggccacggac ctggatctcg 15060
   cccttttctc ccaccggcag taccgcgcgg gagtccacgt cgacgatcct gcagccggtg 15120
   cccgccaccg gccgtcccga cgagcccgtt ttggggcagg acaaggaatc gaagtgcgtc 15180
   gacggcgacg tctcctgaag tccgtaccct tggacgaccg gcaccccgaa acagccggtc 15240
   agcgcgcggg tcgacggcag cggcagcgcc gagccgccgc acaggatgac ctgcagtgcg 15300
   tccgccgcca gcgtggacag gcgcgggtgc accgccagtc gggacagtcg catgggcagg 15360
   ctgtagaagt gcgtggcctt ctcattgtcg gcggcgtcca cggcctgagc cacgtcgtcg 15420
   ccgacgtgca gcaccagcgt ggcggcgaag gtgaccgcca tcgtcaggtg catcaggtgg 15480
   aaggtcggca gatagttgaa cagcaccgac gacggggtga tcccgtgcgc gtgtccggac 15540
   tgggccgcgt tgacggtgat gttgcggtgg ctgagccgga ccgccttggc tgctcccgtg 15600
   gtgccgctcg tgaactgcag gcaggccacg gaattctcgt cgcaagtggt ggcgggcagc 15660
   acgccgtccg gtgcggacgc gaggacctcg tccagggtcg gcggaccgga cggttcggtc 15720
   tggtcgtcgc gatgggtcag cacgaggtgc cgcagctcgg gcagccggtc cagcaccggc 15780
   accagcctgc gatgcacctc cggcggcacg atcgccgtga ccgcgcccga cgtgcccagc 15840
   acgtgcacca gtccgtccgc cagcagcagc gggttgatca gcgcgggcac cgcccccgac 15900
   ctggcgatac cgaagaacga cacggcgaac gccgggtcga gtacccccgc cagcgccacc 15960
   acttgccccg gttcggccag ctgcgcgtgg acggcccgcg ccagtctgcc ggctcgctca 16020
   tcgagcgccg cgtagctcag ttcgcaggct gccgcacgaa tcgccacgtg ctcgggcgcg 16080
   cgttcggcgg ccgaacgcag caggtcgtcg accctggggc cgccgatgaa ccccgcgatc 16140
   ccggtcaacg ggtcgccgga caccgtgctg tcctgggcga cggtcgtctc gggcgtagcc 16200
   atgtatccct cctccgctgg ttgggtttca gttcctcggc aggtcgtcgc ggtacacctg 16260
   gcgctcgcgc accaggagcg cgccgccctc gcgcaccagc acgtccacgc agtcacagct 16320
   gaggtcgacc gcagccttgc cacccagcgg cgtaccgatg atcaccgcgt agctcttggc 16380
   cacgatgctg ccgtcgggct gcggatcgac ctgcaccatg cccagccagt gccgccgctg 16440
   gatgccctgt tccgccaggc gggtggccgc ttcgagcgcg ccggcctcga tggccgtccg 16500
   gccgcgggcg ggttcggggt gcgcgttggc ggcgaacatc ccgtcctcgg tgaaggtctt 16560
   ggcccactcc tgcacgcggc cctcgtccag gtagcgcatc tggcgcccgt agaactgctg 16620
   gacctccacg tacagcgcga tgtcctcggt gctgccgact gcctgtaccg cctgtgacat 16680
   gactgctccc tcgcttgctc gtggtcggca aacgatggca ccggggcatc ggacgccact 16740
   caactgagga cgaccggcac cggctcgaac cgggcgctga tcggacttga gccgccgggt 16800
   acagcctggt cccgttcaca acggacgggc tggagggaag gctgatgagg agcaccgagg 16860
   agccgcgggt cgccttggtc acgggagcga ccagcgggat cggtctcgcc gtcaccaagg 16920
   cgctggccgc caggggactg cgggtgttca tctgcgcgcg caaccgggag aacgtcgttt 16980
   ccacggtgaa ggagttgcgg gcgcagggtt tggacgtgga cggccaggcc ggtgacgtgc 17040
   ggtcggtggc cgccgttcgc gaggtggtcg agtccgcggt caaccggttc ggcaccatca 17100
   gcgtgctggt caacaacgcc ggccgcagcg ggggcgggat caccgcgaag atcaccgacg 17160
   agctgtggca ggacgtgatc gacaccaatc tcaacagcgt cttcacggtc acccgcgagg 17220
   tgctgaccac cggcgggctc gacggggccg acggcggccg gatcatcaac atcgcctcga 17280
   ccgggggcaa gcagggcgtc ccgctcggcg cgccctactc ggcggccaag agcggggtca 17340
   tcggcttcac caaggcgctg ggcaaggaac tggccaagac cggggtcacg gtgaacgccg 17400
   tctgccccgg ctacgtcgaa acgccgatgg ccgtccgcgt ccggcaggcg tatgccagca 17460
   cttgggacac caccgacgaa aacgtgctgg cgcggttcaa cgacaagatc ccgctcggcc 17520
   gctactgcac tccggaagag gtggcgggca tggtcgacta cctggtcgcc gacacggccg 17580
   cttcggtgac ggcgcaggcg atcaacgtct gcggcggcct cggcaactac tgaccccgca 17640
   cgctcacgga aaggcacagc catgcccgcc gcagcacagc agcacaccga acaccggatc 17700
   gacatcgacg ctcccgccgg cctggtgtac cgcatcatcg cggacgcgac cgagtggccg 17760
   cggcacttca ccccgaccgt ccacgttgat cagtccgaac tggacggaca caccgaacgc 17820
   ctgcacatct gggccaacgc caacgggcag ctgaagagct ggacctcgct ccgggaactc 17880
   gacgagcggg ccggccggat ccggttccgg caggaggtgt ccgcgccccc ggtcgcctcg 17940
   atgagcggcg agtggatcgt gtccgagcgg gtcgcggagc gcaccacgct ggtgctgacg 18000
   cacgacttcg ccgcggtgga cgacgatccc gccggagtgg aatggatcac caaggccacc 18060
   aacgggaaca gtgacaccga actggcgaac atcaaggcac tggccgagcg ctgggagcgg 18120
   atggaccggc tggccttcga cttcgaggac tccgtgctgg tacgggcgcc caaggaacgg 18180
   gcgtaccact tcctggaccg cgtggacctg tggccggacc ggctgcccca cgtcgccagg 18240
   ctggagctgc gcgaggacgt gcccggagtt cagcacatgt ccatggacac caaggcgaag 18300
   gacggatcga cgcacaccac ggtgtcggtg cgggtgtgct tccccgaagc ccggatcgtc 18360
   tacaagcaac tcgtgccgcc cgcgttgctg accacccaca ccggagtgtg gacgttcgag 18420
   gacacagcgg acggtgtttt ggtgacttcc gcgcacacgg tcgtgctcaa cgaggccaac 18480
   atcggcacgg tgcccggccc ggccgcgact gtcgagtcga cgcgcgactt cgtccgcaac     18540
   gcgatcagcg gcaacagcca ggccacgctg cgtcatgcca aggcgttcgc cgaggcgacc     18600
   gatgcctgag gactccggcg aagagcccga agtcctcgtg gccggggcgg ggccggtcgg     18660
   gctgaccgcc gcgcacgagc tggcccgccg aggggtgcgg gtacggctgg tggaccgtag     18720
   cgccggcccg gccacgacga gtcgtgcgct cgcgacgcac gcccgcacgt tggagatctg     18780
   gcaccagatg gggctgctcg gcgagctgct gccgcggggc cggcgggtcg agcacttcac     18840
   actgcatctg aagggtaaaa ccctgatgtg cttcgacacc aactatgaca ccatgccgac     18900
   ccggttcccg ttcagcctca tggtggacca ggtcgtgacc gaggaagtgc tgcgccggca     18960
   ggtgcgggca ctcggggtca cggtcgagtg gggcgtcgag ctgacctggt tcgaccagga     19020
   gccggacggg gtcctggcgg agctgcggca tgcggacggg acagtcgagc aggtcaccgc     19080
   ggcctggctg gtcggcgcgg acggtgcccg cagcaccgtc cgcaagcggt tggacctgcg     19140
   cctgcagggg gactccacac agacctggct caacgccgac gtggtgctcg acacggatct     19200
   ggccggcgac agcaaccacc tgctgcacac cggacgcggc accttgctgc tggttccctt     19260
   tccagaaccg ggaaagtggc gggtggtcga caccgaggac accgaccacg ccgacgatgc     19320
   ccggatcgtc cgggccaggc tggccgacaa gctcaccagg gcgctcggcc gccccatcga     19380
   ggtgcccgag ccgagctgga tctcggtttt caccgtgcag cagcggatga tcgaccggat     19440
   gcgggccggc cgttgtttcg tcgccggtga cgccgcccat gtgcacagcc cggcgtccgg     19500
   gcagggcatg aacaccggga tccaggacgc ctacaacctg gcgtggaagc tggcggacgt     19560
   ggtgcgcggc cacgccaagg agagcctgct cgacagctat ggggccgagc gggtgccgat     19620
   cggcgagacg ttgttgcgta ccaccaggac cgccactgcg cttgtgagcc tgcgcaacac     19680
   cgtcgcgccg ttggtcatgc cggccggaac ccggctgctg ggggcgctca agccactcaa     19740
   acggcggatc gagcgcacca tgatccgggg cttctgcggg ctgaccctga actacaccca     19800
   cagcccgctg agcctggcct gcgcaacccc ggacggcatc cagccgggac atcgggtggg     19860
   ctgctcggtg gatcgtgccc ggacctcacc gggatggcaa gggctggtca cggaactgac     19920
   cgatccccgg tggactttgc tcgccttcgc cgattcccag gaacagcggc agatcgccgc     19980
   gcaagtcgag cggcgctacg gcaaggccgt ctcggtgcgg gtggtggccg aggccgccac     20040
   gagcgagcgg gttcttgccg acccgggcga tgacctagca cgagatttcg ccatgcgggc     20100
   agggtatttc gtgctgatcc ggccggacgg ccacctggcg gcgaaagggc ggctgagcga     20160
   tgacctggat ggcgcattcg gcgctctggg actcgtgccg gccgatgccg ggggagaccc     20220
   ggcccaccac ctcgattccg aaggatcacg catgcgcatc atcgacctgt ccgcgaccat     20280
   ggacgctgcc gatcgctggg aggccaaccc ggtcacccac gaggtgctca cggccgccga     20340
   aggcgcgcag cacatggcgg cggagatgaa agagcacttc gggatcgact tcgacccctc 20400
   ggtgctgccc ggcggggagt tgctcacgct ggacacgttg acgctgacca cgcacaccgg 20460
   cacgcacgtc gacgccccgt cgcactacgg gactccccgc gacggcgtgg ccaggcacat 20520
   cgaccagatg ccgctggagt ggttcctgcg gccgggggtg gtcctcgacc tcacgggcga 20580
   gccggtcggc gcggccggtg cggaccggct gcgcgaggag ttcgagcgaa tcggctatac 20640
   cccgaagccg ctggacatcg tgctgctcaa caccggcgcc gacgcgctgg ccggaagccc 20700
   gaagtacttc accgacttca ccgggctcga cgggaaggcc accgagctgc tgctcgattt 20760
   gggggtacgg gtcatcggta ccgacgcgtt cagcctggac gcgccgttcg gtcacatgat 20820
   cgccgaatac cggcgtacgg gggatcgctc cgtgctgtgg cccgcgcatt tcgcgggccg 20880
   ggaccgggag tactgtcaga tcgaggggtt gaccaacctg gccgcgctgc cgtcgccgac 20940
   cggtttttcg gtatcctgcc tgccggtcaa gatcgccggc gcgggcgccg gctggacccg 21000
   ggccgttgcc ctgctggact aggaaggaag gtccgtgaag gggcccttca cggactcaga 21060
   gtccgtgaag ggccccttca cagccgcggt gaccgatgta tcgggagata catgccggcg 21120
   tcgggcgatg cccttccttg gatgtactcc tcttgagcgg ccctcggctg ggggagtacc 21180
   aataccgtat gaaattcaac ctgcttggtc cgatggaagt tctgtgcgcc gatggcacgg 21240
   tgacgcccag cgccgcgaaa atgcgctgga ttctggcatt gctcctgttg cacggaaatc 21300
   gagtggtcga tcaggcttcg atgatcgacg aattgtgggg agatcacccg ccgcgcagtg 21360
   cggtgaccac cacgcagacc tacgtttacc aattgcgcaa gaagtacgac tactacgcac 21420
   aacgagaggg gagaaagagt ttcatcgtca cccgggcgcc cggttatctg ctgcagctgg 21480
   acgacgatca gctcgatgtc cgaagattcc agagactcag cgccgagggc agcgccctgt 21540
   tctcggcggg ccacgccgaa cgggcggacg aggtgctgcg gcaggcactg cggctgtggc 21600
   ggggccccgc gctggccggc atcgcacccg gccgcatgct gcaggcgcac gtcgcctacc 21660
   tggaagaggc ccggctgcgc accgtgcagg tgcgcatcct cgccgacgcg gcactcgggc 21720
   ggcaccggga tctgattccg gagctgcggt cactggtgat cgagcatccg ctcgacgaat 21780
   ggtttcacca gcagctgatc acggcactgg ccgaggcagg gcgcagggga gacgccctgc 21840
   acgcctgccg ggtcctgcac cgcaccctcg ccgacgaact cggcgtggcg ccctccgagc 21900
   cgctgcgcaa gctccagcag gacctgctga ccggccacgt ccggcgggca cccgcgcatg 21960
   tgtgacctgc ggtggagccg gagacggcgg ccgccgccgt ctccggcctc acgcggggac 22020
   cagcggcgcc gcggtctgcg ccttgttcac cgcgtccagc cgggaaaccg cgttcagctt 22080
   cacgaagatg ttgcgcaggt ggcgtttcac cgtcccctgg gtgatggcca ggcaattccc 22140
   gatctgtgcg ttggtcatcc cgcgggcgac cagcgacatg atctcgtgct cgcgcctgga 22200
   cagcggagta cgcaggttcg aggacagcga ccggacgtcg acactcggca ccgagaggaa 22260
   gacgcagcgc tcgtcgcggc tcacctcgcg caccaccgag agcaggtaca tgtcggagac 22320
   attcttgggc aggtaacccc ggacgccgat gcccaggaat tcctgaatga aaccgaatct 22380
   gttctcctgg ctcaggatga cgacgggctt ccccggagcc gccgccgaca attcgagcag 22440
   gccggattcg acttccggac cccagccgtc gactccatag agaatgacgt ccacaattcc 22500
   cctggagaca ctgtcgagtg cttcgccgat ggtcgccacc gcgtcaatgg cggccagatc 22560
   gtcggcgcct tgaatcagcg aaagcagccc gacgcgaaaa acggaactct cttcgacgat 22620
   cagaattctt acacggtcag ttatcatggt gtgataacac tcccccggta tggtcaaccg 22680
   tgccagcccg cgcggctggc ggttccttcg tatagtcccc cgactgcgtg gcataaaaat 22740
   tggcgga 22747
<210> 27
   <211> 20
   <212> PRT
   <213> Amycolatopsis sulphurea
<400> 27
   Gly Pro Val Gly Leu Val Ser Thr Gly Cys Thr Ser Gly Val Asp Val
   1               5                   10                  15
   Ile Gly His Ala
               20
<210> 28
   <211> 24
   <212> PRT
   <213> Amycolatopsis sulphurea
<400> 28
   Val Pro Val Ser Ser Ile Lys Ser Met Val Gly His Ser Leu Gly Ala
   1               5                   10                  15
   Ile Gly Ser Leu Glu Val Ala Ala
               20
<210> 29
   <211> 9
   <212> PRT
   <213> Amycolatopsis sulphurea
<400> 29
   Val Ser Glu Gln Ala Gly Gly Leu Asp
   1               5
<210> 30
   <211> 6
   <212> PRT
   <213> Amycolatopsis sulphurea
<400> 30
   Leu Gly Tyr Asp Ser Leu
   1               5
<210> 31
   <211> 17
   <212> PRT
   <213> Artificial Sequence
<220>
   <223> Conserved sequence to be found in ChdOI and ChdOII
<220>
   <221> SITE
   <222> (2)..(2)
   <223> Xaa can be any naturally occurring amino acid
<220>
   <221> SITE
   <222> (4)..(5)
   <223> Xaa can be any naturally occurring amino acid
<220>
   <221> SITE
   <222> (7)..(9)
   <223> Xaa can be any naturally occurring amino acid
<220>
   <221> SITE
   <222> (11)..(16)
   <223> Xaa can be any naturally occurring amino acid
<400> 31
   Gly Xaa Gly Xaa Xaa Gly Xaa Xaa Xaa Ala Xaa Xaa Xaa Xaa Xaa Xaa
   1               5                   10                  15
   Gly
<210> 32
   <211> 11
   <212> PRT
   <213> Artificial Sequence
<220>
   <223> Conserved motif to be found in ChdY
<220>
   <221> SITE
   <222> (2)..(2)
   <223> Xaa can be any naturally occurring amino acid
<220>
   <221> SITE
   <222> (6)..(6)
   <223> Xaa can be any naturally occurring amino acid
<220>
   <221> SITE
   <222> (8)..(8)
   <223> Xaa can be any naturally occurring amino acid
<220>
   <221> SITE
   <222> (10)..(10)
   <223> Xaa can be any naturally occurring amino acid
<400> 32
   His Xaa Gly Thr His Xaa Asp Xaa Pro Xaa His
   1               5                   10
<210> 33
   <211> 22
   <212> DNA
   <213> Artificial Sequence
<220>
   <223> Primer
<400> 33
   tcctcactgc aggtcgagta cc 22
<210> 34
   <211> 18
   <212> DNA
   <213> Artificial Sequence
<220>
   <223> Primer
<400> 34
   cgggaagtcg cggtatgc 18
<210> 35
   <211> 20
   <212> DNA
   <213> Artificial Sequence
<220>
   <223> Primer
<400> 35
   cgcgctggtc aaagtctacg 20
<210> 36
   <211> 18
   <212> DNA
   <213> Artificial Sequence
<220>
   <223> Primer
<400> 36
   ctggacgcct cgccgtac 18
<210> 37
   <211> 30
   <212> DNA
   <213> Artificial Sequence
<220>
   <223> Primer
<400> 37
   tatatacata tgaaggacaa tctcgcgaga 30
<210> 38
   <211> 29
   <212> DNA
   <213> Artificial Sequence
<220>
   <223> Primer
<400> 38
   tatatatcta gaggacctcc gcatcaggc 29
<210> 39
   <211> 40
   <212> DNA
   <213> Artificial Sequence
<220>
   <223> Primer
<400> 39
   agtcgaattc gcaccatatg agaccaagcg cgtccgggtg 40
<210> 40
   <211> 43
   <212> DNA
   <213> Artificial Sequence
<220>
   <223> Primer
<400> 40
   cgactctaga ggatcactag ttaccagccc gacccgagca cgc 43
<210> 41
   <211> 24
   <212> DNA
   <213> Artificial Sequence
<220>
   <223> Primer
<400> 41
   tatatagcat gcgcatcatc gacc 24
<210> 42
   <211> 19
   <212> DNA
   <213> Artificial Sequence
<220>
   <223> Primer
<400> 42
   gcgtcggtgc tgatgaccc 19
<210> 43
   <211> 19
   <212> DNA
   <213> Artificial Sequence
<220>
   <223> Primer
<400> 43
   ggtcatcagc accgacgcg 19
<210> 44
   <211> 33
   <212> DNA
   <213> Artificial Sequence
<220>
   <223> Primer
<400> 44
   tatataacta gtcgtccagc tgcagcagat aac 33
<210> 45
   <211> 28
   <212> DNA
   <213> Artificial Sequence
<220>
   <223> Primer
<400> 45
   atatacatat gcgcatcatc gacctgtc 28
<210> 46
   <211> 32
   <212> DNA
   <213> Artificial Sequence
<220>
   <223> Primer
<400> 46
   tatatatcta gactagtcca gcagggcaac gg 32
<210> 47
   <211> 26
   <212> DNA
   <213> Artificial Sequence
<220>
   <223> Primer
<400> 47
   atatacatat gcctgaggac tccggc 26
<210> 48
   <211> 31
   <212> DNA
   <213> Artificial Sequence
<220>
   <223> Primer
<400> 48
   tatatacata tgcgcatcat cgatctgtcg a 31
<210> 49
   <211> 30
   <212> DNA
   <213> Artificial Sequence
<220>
   <223> Primer
<400> 49
   atatatctag actactcctc caccaccgcc 30